# EUROPEAN PATENT APPLICATION

(11) **EP 4 029 865 A1**
(43) Date of publication of application: **20.07.2022**
(21) Application number: 20862539.2
(22) Date of filing: 09.09.2020
(51) Int. Cl.: C07D 471/04, C07D 405/14, C07D 413/14, C07D 519/00, A61K 31/4375, A61K 31/497, A61K 31/4709, A61K 31/4025, A61P 35/00

(54) **NOVEL TRICYCLIC AROMATIC HETEROCYCLIC COMPOUND, PREPARATION METHOD THEREFOR, PHARMACEUTICAL COMPOSITION AND APPLICATION THEREOF**

(30) Priority: 09.09.2019 CN 201910848926
(71) Applicant: Shanghai Longwood Biopharmaceuticals Co., Ltd., Shanghai 201108 (CN)
(72) Inventor: WANG, Zhe, Shanghai 201108 (CN); BAI, Haiyun, Shanghai 201108 (CN); LI, Deliang, Shanghai 201108 (CN); QIAN, Anran, Shanghai 201108 (CN)
(74) Representative: Lavoix
(86) International application number: PCT/CN2020/114253
(87) International publication number: WO 2021/047547

(57) **Abstract**

The present invention provides a novel tricyclic aromatic heterocyclic compound, a preparation method therefor, a pharmaceutical composition and an application thereof. Specifically, the present invention provides a compound as represented by the following formula I, or optical isomers, hydrates, and solvates thereof, or pharmaceutically-acceptable salts thereof. Definitions of groups are as stated in the description. The compound as represented by formula I can be used for treating diseases related to a PD-1/PD-L 1 signal pathway.

## Description

### TECHNICAL FIELD

The present invention relates to the field of small molecule drugs, and specifically, the present invention provides a small molecule compound that can be used to treat diseases related to PD-1/PD-L1 signaling pathway.

### BACKGROUND OF THE INVENTION

The immune system plays a vital role in controlling and curing many diseases, such as various cancers, diseases caused by viruses, etc. But the cancer cells can evade or inhibit the immune system in some way to proliferate rapidly. One way is to alter the activator molecules and inhibitory molecules expressed on immune cells. Blocking inhibitory immune checkpoints, such as PD-1, has been proven to be a very effective approach to suppressing cancer cells.

PD-1 is programmed cell death protein-1, also known as CD279. It is mainly expressed in activated T cells and B cells, and its function is to inhibit the activation of cells, which is a normal homeostatic mechanism of the immune system, because excessive T/B cell activation may cause autoimmune diseases, PD-1 is a protective wall of our body. PD-1 is a type I transmembrane glycoprotein composed of 268 amino acids, and its structure mainly includes the outer immunoglobulin variable domain, the hydrophobic transmembrane domain and the intracellular domain. The intracellular domain contains two phosphorylation sites, and they are located in the immunoreceptor tyrosine inhibitory motif and the immunoreceptor tyrosine switching motif, respectively, which also proves that PD-1 can negatively regulate T cell receptor-mediated signal. PD-1 has two ligands, PD-L1 and PD-L2, which differ in expression way. PD-L1 is up-regulated in a variety of tumor cells, and it binds to PD-1 on T cells, inhibits T cell proliferation and activation, makes T cells in a state of inactivation, and ultimately induces immune escape.

PD-1/PD-L1 plays an inverse immunomodulatory role. When PD-1 binds to PD-L1, it can cause tyrosine polyphosphorylation in the immunoreceptor tyrosine switch motif domain of T cells, and the phosphorylated tyrosine can bind to the phosphatase protein tyrosinase 2 and protein tyrosinase 1, which can impede the activation of extracellular signal-regulated kinase and also block the activation of phosphatidylinositol 3-kinase (PI3K) and serine-threonine protein kinase (Akt), thereby inhibiting T lymphocyte proliferation and the secretion of related cytokines. The PD-1/PD-L1 signal inhibits the activation and proliferation of T cells, and at the same time, it can also induce the secretion of cytokines interleukin 2, interferon γ and IL-10. In addition, PD-1/PD-L1 signaling also has a similar immune function on B cells. After PD-1 binds to B cell antigen receptors, the PD-1 cytoplasmic domain interacts with tyrosinase containing a protein tyrosinase 2 binding site, thereby hindering B cell activation.

The immunotherapy based on PD-1/PD-L1 is a new generation of immunotherapy that has attracted much attention. In recent years, a series of surprising findings have confirmed that PD-1/PD-L1 inhibitors have strong antitumor activity against a variety of tumors. Currently available PD-1/PD-L1 antibody inhibitors include BMS's Ninolumab, Merck's Lambrolizumab and Roche's Atezolizumab. In addition, there are many PD-1/PD-L1 antibody modulators in development, including CureTech's Pidilizumab, GSK's AMP-224 and AstraZeneca's MEDI-4736.

Although tumor immunotherapy is considered to be a new generation of revolution in cancer treatment after targeted therapy, the PD-1 monoclonal antibodies currently on the market and under development have their own shortcomings. They can only be administered by injection and cannot be taken orally. They are unstable in the body, are easily decomposed by proteases, and are prone to immune cross-reaction. Moreover, it is difficult to purify and the production cost is high. Therefore, small molecule modulator of PD-1/PD-L1 interaction is a better option for tumor immunotherapy.

In summary, there is an urgent need in the art to develop novel small-molecule modulator of PD-1/PD-L1 interaction.

### SUMMARY OF THE INVENTION

The object of the present invention is to provide a novel small-molecule modulator of PD-1/PD-L1 interaction.

The first aspect of the present invention, a compound represented by the following formula I, or an optical isomer, a hydrate, a solvate thereof, or a pharmaceutically acceptable salt thereof: 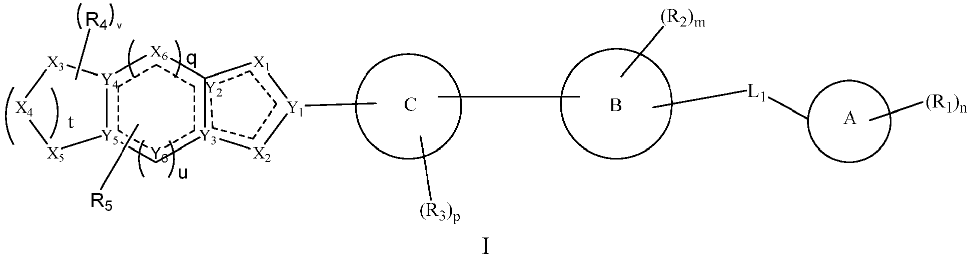 wherein, n is 0, 1, 2, 3, 4, or 5;
m, p, q, t, v, and u are each independently selected from 0, 1, 2, 3 or 4;
X₁, X₂, X₃, X₄, X₅ and X₆ are each independently selected from the group consisting of N, O, S, SO, SO₂, C(R)₂, CHR, and NR;
Y₁, Y2, Y3, Y4, Y₅ and Y₆ are each independently selected from the group consisting of N, CH, and C;
wherein, a hydrogen (if present) on a carbon atom of X₃, X₄, X₅, X₇, X₈, X₉ may be each independently substituted by deuterium; 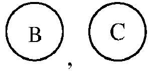 are each independently selected from the group consisting of substituted or unsubstituted C6-C10 arylene, or substituted or unsubstituted 5-12 membered (preferably 5-7 membered) heteroarylene having 1-3 heteroatoms, substituted or unsubstituted 5-12 membered heterocyclylene, and substituted or unsubstituted 5-12 membered C3-C12 (preferably C5-C12) cycloalkylene; 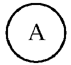 is selected from the group consisting of substituted or unsubstituted 5-12 membered heteroaryl, substituted or unsubstituted C6-C10 aryl, substituted or unsubstituted 5-12 membered heterocyclyl, substituted or unsubstituted 5-12 membered C3-C12 (preferably C5-C12) cyclic group, wherein the heterocyclyl has 1-3 heteroatoms;
L₁ is selected from the group consisting of chemical bond, substituted or unsubstituted C1-C4 alkylene, substituted or unsubstituted C2-C4 alkenylene, substituted or unsubstituted C2-C4 alkynylene, -S-, -O-, substituted or unsubstituted -NH-, -S(O)-, -S(O)₂-, substituted or unsubstituted -NHC(O)NH-, 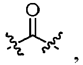
substituted or unsubstituted 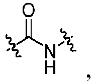 substituted or unsubstituted 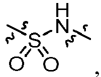 substituted or unsubstituted 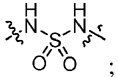 and 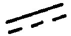 represents single bond or double bond;
R, R₁, R₂, R₃, R₄, and R₅ are each independently selected from the group consisting of H, -CN, trifluoromethyl, -CHF2, -OCF3, -OCHF2, sulfonamido, nitro, hydroxyl, halogen, -S-R₈, - S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl (the two substituents here are recommended to be reserved), NR_{d}Rₑ (eg amino), substituted or unsubstituted C1-C6 amino, substituted or unsubstituted -(C1-C6 alkylene)-NH-(C1-C6 alkylene), carboxy, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, wherein a hydrogen on a carbon atom of substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e. =O), =NRf, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms may be each independently substituted by deuterium; substituted or unsubstituted 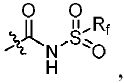 substituted or unsubstituted 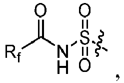 substituted or unsubstituted 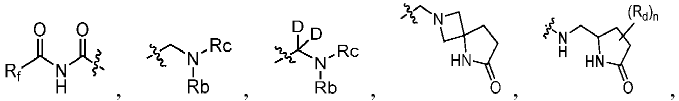 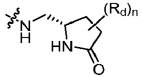 (please modify the structure here modified, 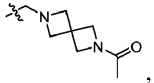 wherein, Rb and Rc are are each independently selected from the group consisting of H, substituted or substituted C₁-C₈ alkyl; or Rb and Rc together with adjacent N atom form substituted or unsubstituted 3-10 membered heterocyclyl having 1-3 heteroatoms selected from N, S and O, or Rb and Rc together with adjacent N atom form substituted or unsubstituted 4-10 membered ring, the substituents include but are not limited to hydroxyl, carboxyl, sulfhydryl, amino, F, C, wherein, a hydrogen on a carbon atom of Rb, Rc and Rd may be each independently substituted by deuterium; or -(L₁ₐ)ᵣ-(L₂ₐ)ₛ-(L₃ₐ)ₛ-; -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉;
R₈ and R₉ are each independently selected from the group consisting of H, hydroxy, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NR_{d}Rₑ (eg amino), substituted or unsubstituted C1-C6 amino, substituted or unsubstituted-(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12-membered heterocyclyl with 1-4 heteroatoms, substituted or unsubstituted 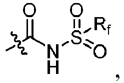 substituted or unsubstituted 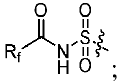 substituted or unsubstituted 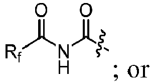 -(L₁ₐ)ᵣ-(L₂ₐ)ₛ-(L₃ₐ)ₛ-;
each L₁ₐ is each independently the group selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, and -S(O)₂-;
L2a is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, and substituted or unsubstituted 3-10 membered heterocyclylene with 1-3 heteroatoms;
L3a is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g}, - OR_{g}, -N(R_{g})₂, -CO₂R_{g}, -CON(R_{g})₂, -CONHCOR_{g}, NR_{g}-CO-N(R_{g})₂, and -NRg-SO₂-N(Rg)₂;
r is 1, 2, 3, 4, 5, 6;
s is 0, 1, 2 respectively;
R_{d}, Rₑ and R_{g} are each independently selected from the group consisting of H, substituted or unsubstituted C₁-C₆ alkyl, substituted or unsubstituted C₃-C₁₀ cycloalkyl, substituted or unsubstituted C₆-C₁₀ aryl; or Rd and Re together form substituted or unsubstituted 4-10 (preferably 5-10) membered heterocyclyl having 1-3 heteroatoms selected from N, S and O;
R_{f} is selected from the group consisting of H, substituted or unsubstituted C₁-C₆ alkyl, substituted or unsubstituted C₆-C₁₀ aryl, substituted or unsubstituted 5-10 membered heteroaryl, cyano, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, and -C(=O)-substituted or unsubstituted C₂-C₆ alkynyl;
unless otherwise specified, the "substituted" refers to substitution with one or more (eg 2, 3, 4, etc.) substituents selected from the group consisting of halogen (including -F, -Cl, -Br, -I), -CH₂Cl, -CHCl₂, -CCl₃, -CH2F, -CHF2, -CF3, oxo (=O), -CN, hydroxyl, amino, C1-C6 alkylamino, carboxyl, -NHAc, 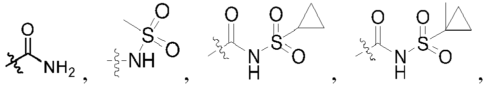 substituted or unsubstituted group selected from the group consisting of C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, 5-10 membered heterocyclyl with 1-3 heteroatoms selected from N, S and O; the substituent is selected from the group consisting of halogen, hydroxyl, carboxyl, oxo, cyano, C1-C6 alkoxy, and C1-C6 alkylamino;
in the above formulas, any one of the heteroatom is selected from the group consisting of B, P, N, S and O.

In another preferred embodiment, when both A and L1 are absent, B is a group selected from the group consisting of substituted or unsubstituted C6-C10 aryl, substituted or unsubstituted 5-12 membered (preferably 5-7 membered) heteroaryl with 1-3 heteroatoms, substituted or unsubstituted 5-12 membered heterocyclyl, substituted or unsubstituted 5-12 membered C3-C12 (preferably C5-C12) cycloalkyl.

In another preferred embodiment, the compound of formula I has a structure shown in the following formula:

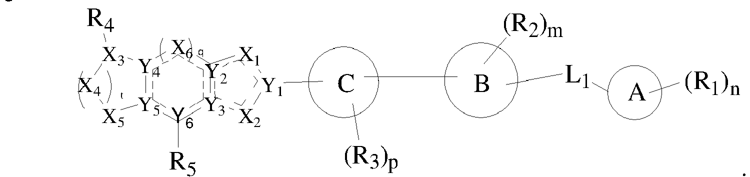

In another preferred embodiment, the present invention provides a compound represented by the following formula I, or an optical isomer, a hydrate, a solvate thereof, or a pharmaceutically acceptable salt thereof: 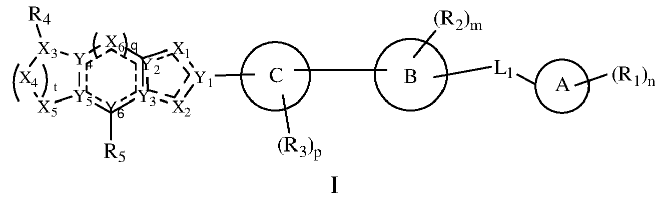 wherein, n is 0, 1, 2, or 3;
m, p, q, and t are each independently selected from 0, 1, 2, 3 or 4;
X₁, X₂, X₃, X₄, X₅ and X₆ are each independently selected from the group consisting of N, O, S, SO, SO₂, C(R)₂, and NR;
Y₁, Y2, Y3, Y4, Y₅ and Y₆ are each independently selected from the group consisting of N, CH, and C; 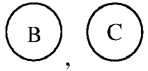 are each independently selected from the group consisting of substituted or unsubstituted C6-C10 arylene, or substituted or unsubstituted 5-12 membered (preferably 5-7 membered) heteroarylene having 1-3 heteroatoms, substituted or unsubstituted 5-12 membered heterocyclylene, and substituted or unsubstituted 5-12 membered C5-C12 cycloalkylene; 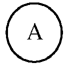 is selected from the group consisting of substituted or unsubstituted 5-12 membered heteroaryl, substituted or unsubstituted C6-C10 aryl, substituted or unsubstituted 5-12 membered heterocyclyl, substituted or unsubstituted 5-12 membered C5-C12 cyclic group, wherein the heterocyclyl has 1-3 heteroatoms;
L₁ is selected from the group consisting of chemical bond, substituted or unsubstituted C1-C4 alkylene, substituted or unsubstituted C2-C4 alkenylene, substituted or unsubstituted C2-C4 alkynylene, -S-, -O-, substituted or unsubstituted -NH-, -S(O)-, -S(O)₂-, substituted or unsubstituted -NHC(O)NH-, 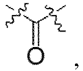 substituted or unsubstituted 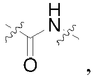 substituted or unsubstituted 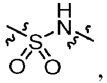 substituted or unsubstituted 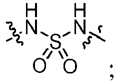
R, R₁, R₂, R₃, R₄, and R₅ are each independently selected from the group consisting of H, -CN, trifluoromethyl, sulfonamido, nitro, hydroxyl, halogen, -S-R₈, -S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted -(C1-C6 alkylene)-NH-(C1-C6 alkyl), carboxy, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, substituted or unsubstituted 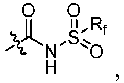 substituted or unsubstituted 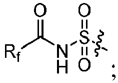 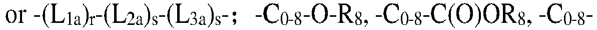 OC(O)OR₈, -C₀₋₈-NR₈R₉, -C₀₋₈-N(R₈)C(O)R₉, -C₀₋₈-C(O)NR₈R₉;
R₈ and R₉ are each independently selected from the group consisting of H, hydroxy, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted-(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12-membered heterocyclyl with 1-4 heteroatoms, substituted or unsubstituted 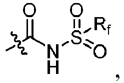 substituted or unsubstituted 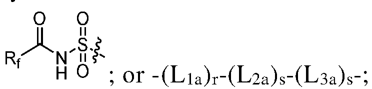
each L₁ₐ is each independently the group selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, and -S(O)₂-;
L2a is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, and substituted or unsubstituted with 5-10 membered heterocyclylene with 1-3 heteroatoms;
L3a is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, and -SO₂-NH-COR_{g};
r is 1, 2, 3, 4, 5, 6;
s is 0, 1, 2 respectively;
Rd, Re and Rg are each independently selected from the group consisting of H, substituted or unsubstituted C₁-C₆ alkyl, substituted or unsubstituted C₃-C₁₀ cycloalkyl, substituted or unsubstituted C₆-C₁₀ aryl; or Rd and Re together form substituted or unsubstituted 5-10 membered heterocyclyl having 1-3 heteroatoms selected from N, S and O;
Rf is selected from the group consisting of H, substituted or unsubstituted C₁-C₆ alkyl, substituted or unsubstituted C₆-C₁₀ aryl, substituted or unsubstituted C₆-C₁₀ heteroaryl, cyano, -C(=O)-NRdRe, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C2-C6 alkenyl, and -C(=O)-substituted or unsubstituted C2-C6 alkynyl;
unless otherwise specified, the "substituted" refers to substitution with one or more (eg 2, 3, 4, etc.) substituents selected from the group consisting of halogen including but are not limited to -F, Cl, Br, -CH₂Cl, -CHCl₂, -CCl₃, -CH2F, -CHF2, -CF3, oxo, -CN, hydroxyl, amino, C1-C6 alkylamino, carboxyl, -NHAc, a group selected from the group consisting of C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, 5-10 membered heterocyclyl with 1-3 heteroatoms selected from N, S and O, which is unsubstituted or substituted by one or more substituents selected from the following group; the substituent is selected from the group consisting of halogen, hydroxyl, carboxyl, cyano, C1-C6 alkoxy, and C1-C6 alkylamino;
in the above formulas, any one of the heteroatoms is selected from the group consisting of B, P, N, S and O.

In another preferred embodiment, 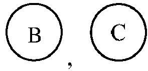 are each independently a divalent group formed by a ring selected from the following group: 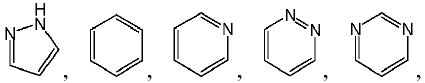 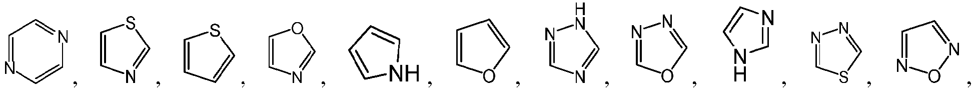 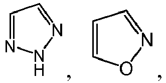 wherein the bonding position of the ring can be N or C (substituents on the ring are not shown).

In another preferred embodiment, R₂ and R₃ are each independently selected from the group consisting of methyl, -F, Cl, Br, -CH₂Cl, -CHCl₂, -CCl₃, -CH2F, -CHF2, -CF3, - CN, hydroxyl, amino.

In another preferred embodiment, the bonding positions of 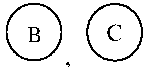 can be *o-, p-,* or *m-*position.

In another preferred embodiment, at a bonding position of 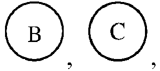 an H connected to N or C is replaced by a bond.

In another preferred example, the 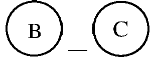 is a structur shown below:

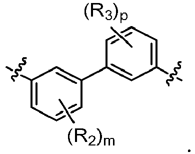

In another preferred embodiment, 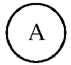 has a structure shown in the following formula: 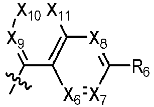 wherein,
X₆, X₇, X₈, X₉, X₁₀ and X₁₁ are each independently selected from the group consisting of N, CR;
R6 is selected from the group consisting of H, halogen, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NRdRe (eg amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 5-12 membered heterocyclyl with 1-4 heteroatoms, substituted or unsubstituted 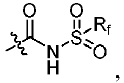 substituted or unsubstituted 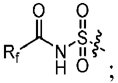 substituted or unsubstituted 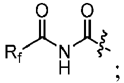 or -(L₁ₐ)ᵣ-(L₂ₐ)ₛ-(L₃ₐ)ₛ-, -C₀₋₈-O-R₈, -C₀₋₈-C(O)OR₈, -C₀₋₈-OC(O)OR₈, - C₀₋₈-NR₈R₉, -C₀₋₈-N(R₈)C(O)R₉, -C₀₋₈-C(O)NR₈R₉.

In another preferred embodiment, 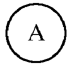 has a structure selected from the group consisting of 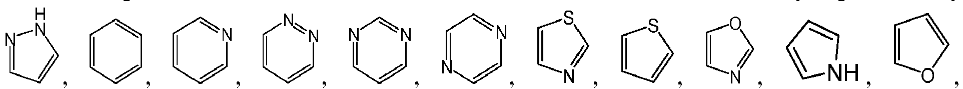 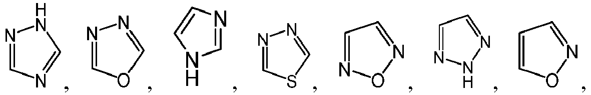 wherein the bonding position of the ring can be N or C (substituents on the ring are not shown).

In another preferred embodiment, R₆ is R₁; or is 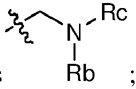 wherein, Rb and Rc are each independently selected from the group consisting of H, substituted or unsubstituted C₁-C₈ alkyl; or Rb and Rc together with the adjacent N atom form a substituted or unsubstituted 3-10 membered heterocyclyl having 1-3 heteroatoms selected from N, S and O.

In another preferred embodiment, IV: 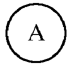 has a substitutent as shown in the following formula 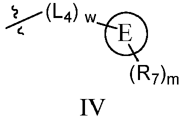 wherein, w is 0, 1, 2, 3, 4, 5, 6;
each L₄ is independently selected from the group consisting of substituted or unsubstituted C1-C4 alkylene, -S-, -O-, NR_{f}, -S(O)-, -S(O)₂-; preferably substituted or unsubstituted C1-C4 alkylene, wherein, a hydrogen on a carbon atom of the substituted or unsubstituted C1-C4 alkylene may be each independently substituted by deuterium, provided that a structure formed by each L4 is chemically stable;
Ⓔ is selected from the group consisting of substituted or unsubstituted C3-C10 cycloalkyl, substituted or unsubstituted 3-10 membered heterocyclyl having 1-3 heteroatoms selected from B, P, N, S and O; preferably, Ⓔ is substituted or unsubstituted 3-8 membered nitrogen-containing heterocyclyl, or substituted or unsubstituted 4-10 membered cyclic amido, wherein a hydrogen on the ring-forming carbon atom of 3-8 membered nitrogen-containing heterocyclyl, substituted or unsubstituted 4-10 membered cyclic amido may be each independently substituted by deuterium;
each R₇ is independently selected from the group consisting of substituted or unsubstituted C1-C6 alkyl, -CN, hydroxy, amino, carboxyl, -OR_{g}, -N(R_{g})₂, -CO-NH-SO₂-R_{g}, -NH-SO₂-R_{g}, -SO₂-NH-CO-R_{g}, -CO₂R_{g}, -CON(R_{g})₂, -CONHCOR_{g}, NR_{g}-CO-N(R_{g})₂, -NR_{g}-SO₂-N(R_{g})₂; R_{f} and R_{g} are defiend as above, wherein a hydrogen on a carbon atom of R_{f} and R_{g} may be independently substituted by deuterium; wherein, the substituent is selected from the group consisting of halogen, hydroxyl, carboxyl, cyano, and C1- C6 alkoxy.

In another preferred embodiment, at least one of R₄ is selected from the following group: NRdRe; Rd and Re are each independently selected from the following group: H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 3-10 membered cycloalkyl, or substituted or unsubstituted 3-10 membered heterocyclyl having 1-3 heteroatoms selected from N, S and O. In another preferred embodiment, the substituent described here is carboxyl, hydroxyl, 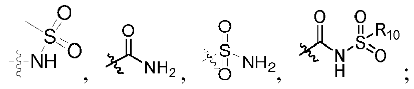 R₁₀ is substituted or unsubstituted C₁-C₆ alkyl, substituted or unsubstituted C₃-C₁₀ cycloalkyl, preferably, R₁₀ is methyl, isopropyl, cyclopropyl and 1-methylcyclopropyl, wherein a hydrogen on a carbon atom of Rd, Re and R₁₀ may be each independently substituted by deuterium.

In another preferred embodiment, 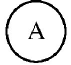 ring has a substituent shown in the following formula IV: 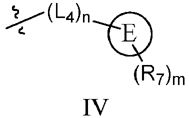 wherein, each L4 is independently selected from the following group: substituted or unsubstituted C1-C4 alkylene, -S-, -O-, -NRa-, -NR_{f}, -S(O)-, -S(O)₂-; preferably substituted or unsubstituted C1-C4 alkylene, provided that a structure formed by each L4 is chemically stable; 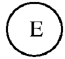 is selected from the group consisting of substituted or unsubstituted C5-C10 cycloalkyl, substituted or unsubstituted 3-10 membered heterocyclyl having 1-3 heteroatoms selected from B, P, N, S and O; preferably, 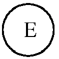 is 3-8 membered nitrogen-containing heterocyclyl; each R₇ is independently selected from the group consisting of substituted or unsubstituted C1-C6 alkyl, -CN, hydroxy, amino, carboxyl, -OR_{g}, -N(R_{g})₂, -CO-NH-SO₂-R_{g}, -NH-SO₂-R_{g}, -SO₂-NH-CO-R_{g}, -CO₂R_{g}, -CON(R_{g})₂, -CONHCOR_{g}, NR_{g}-CO-N(R_{g})₂, -NR_{g}-SO₂-N(R_{g})₂; wherein, the substituent is selected from the group consisting of halogen, hydroxyl, carboxyl, cyano, and C1-C6 alkoxy.

In another preferred embodiment, at least one of R₄ is selected from the following group: NRdRe; Rd and Re are each independently selected from the following group: H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 3-10 membered cycloalkyl, or substituted or unsubstituted 3-10 membered heterocyclyl having 1-3 heteroatoms selected from N, S and O. In another preferred embodiment, the substituent described here is carboxyl, hydroxyl, 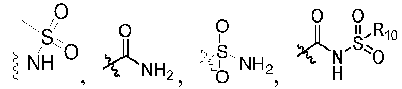 ; R₁₀ is substituted or unsubstituted C₁-C₆ alkyl, substituted or unsubstituted C₃-C₁₀ cycloalkyl, preferably, R₁₀ is methyl, isopropyl, cyclopropyl and 1-methylcyclopropyl, wherein a hydrogen on a carbon atom of Rd, Re and R₁₀ may be each independently substituted by deuterium.

In another preferred embodiment, the compound of formula I has a structure shown in the following formula:

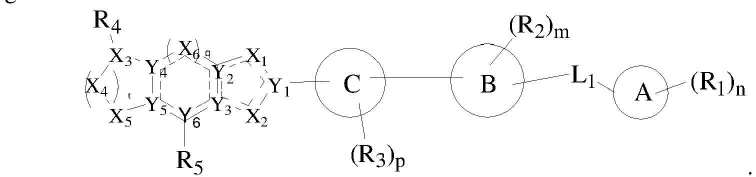

In another preferred embodiment, at least one of R4 is selected from the following group: NRdRe; Rd and Re are each independently selected from the following group: H, substituted or unsubstituted C₁-C₆ alkyl, substituted or unsubstituted C₃-C₁₀ cycloalkyl, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉, substituted or unsubstituted C₆-C₁₀ aryl; or Rd and Re together form substituted or unsubstituted 5-10 membered heterocyclyl having 1-3 heteroatoms selected from N, S and O; preferably, the substituent is carboxyl.

In another preferred embodiment, following group: 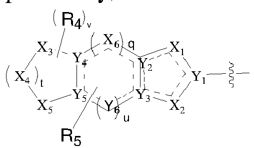 is a structure selected from the 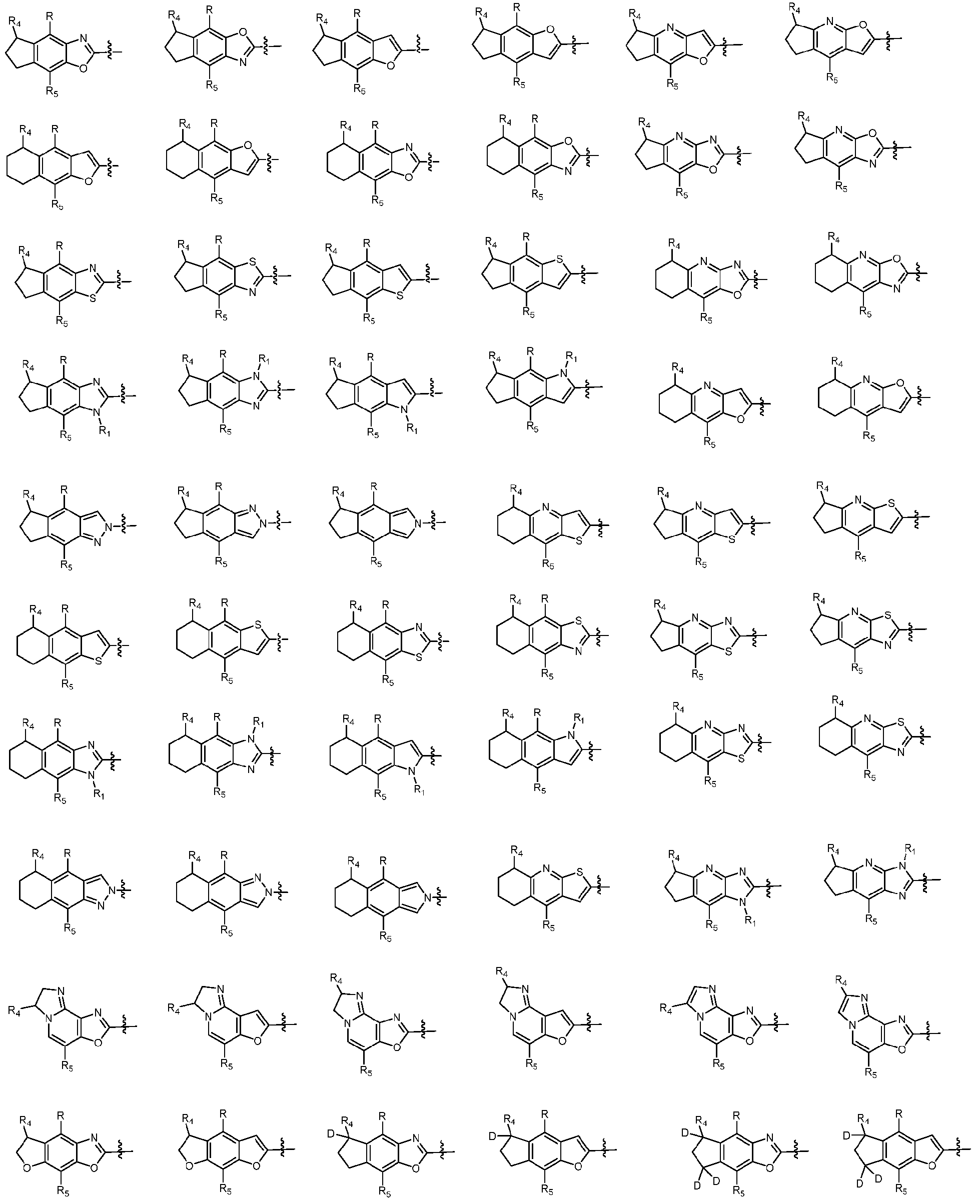 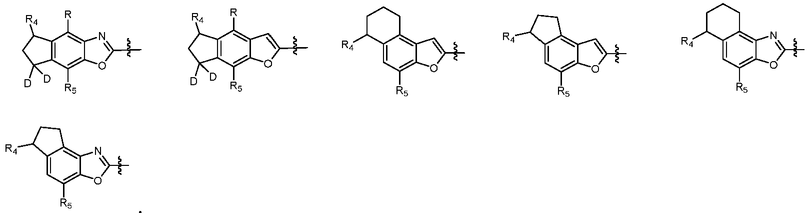 and

In another preferred embodiment, the compound is selected from the following table:

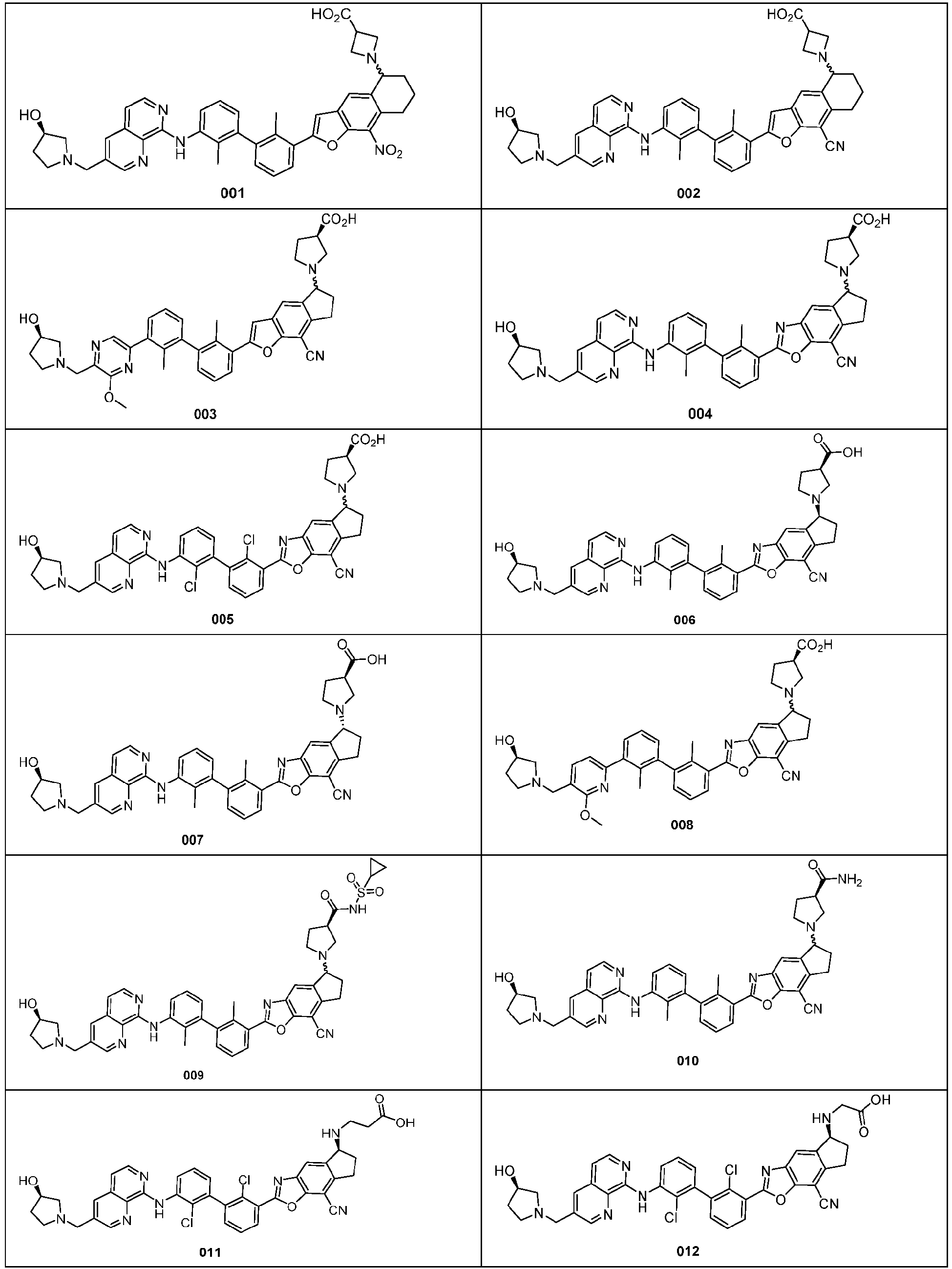

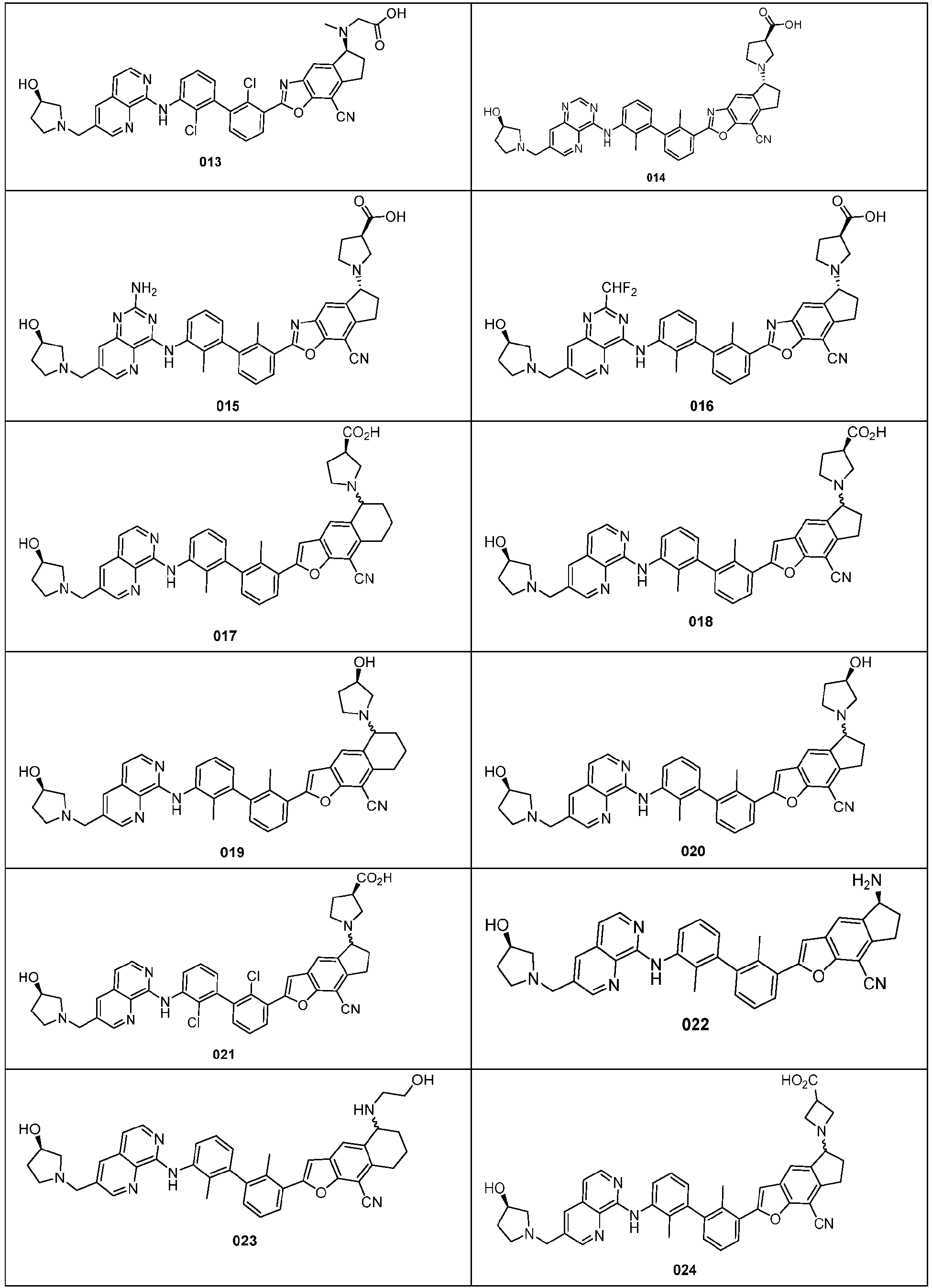

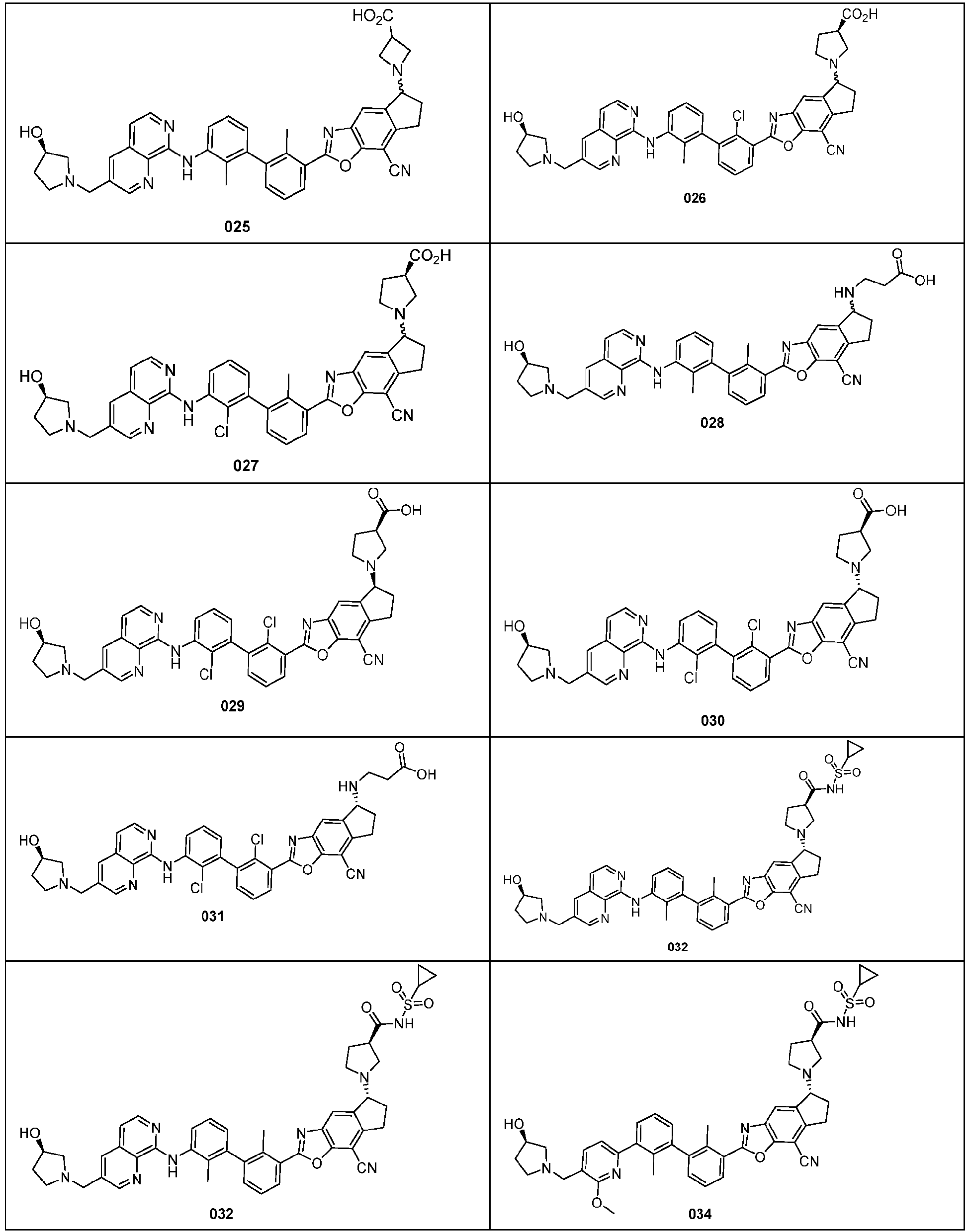

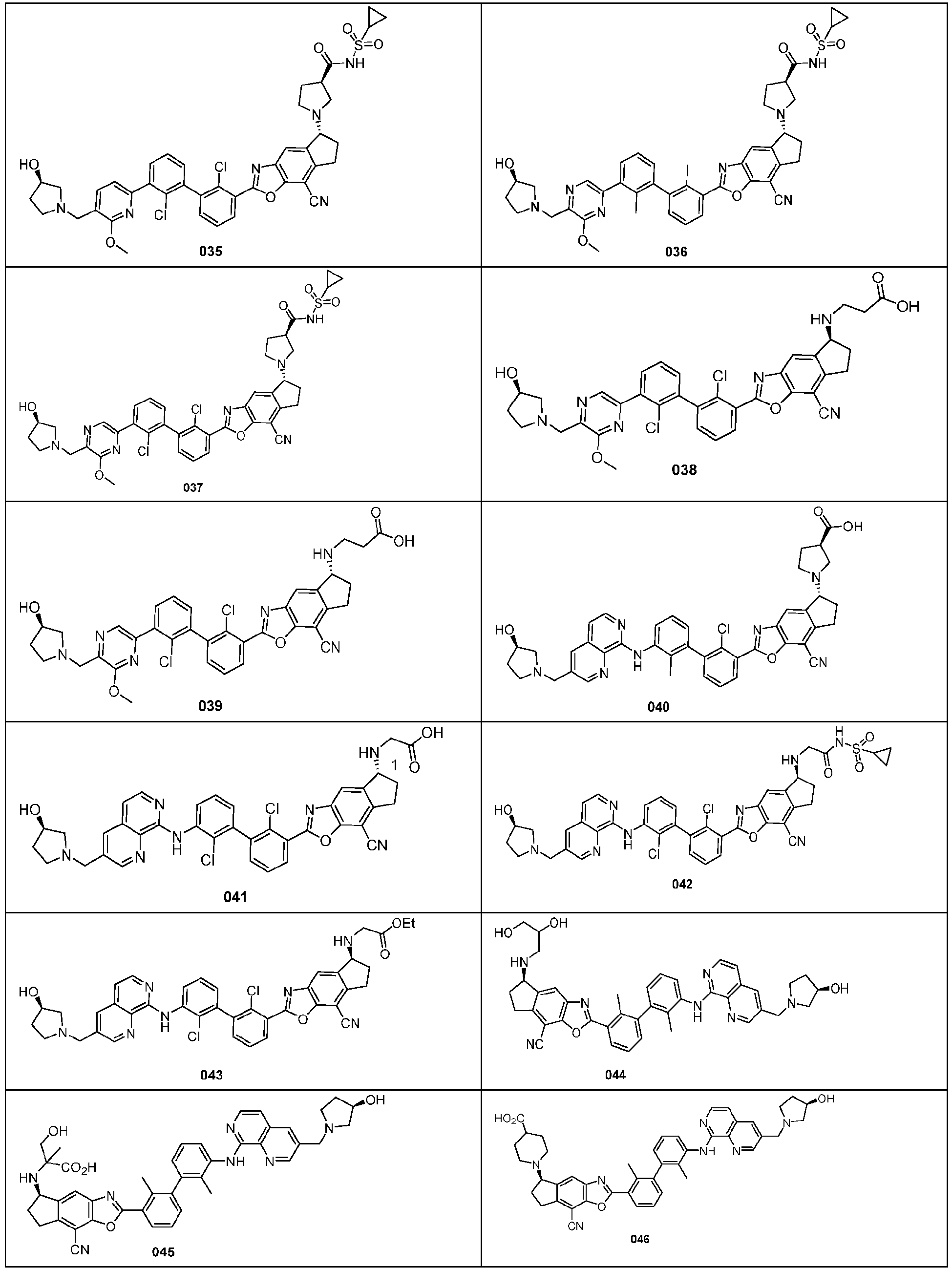

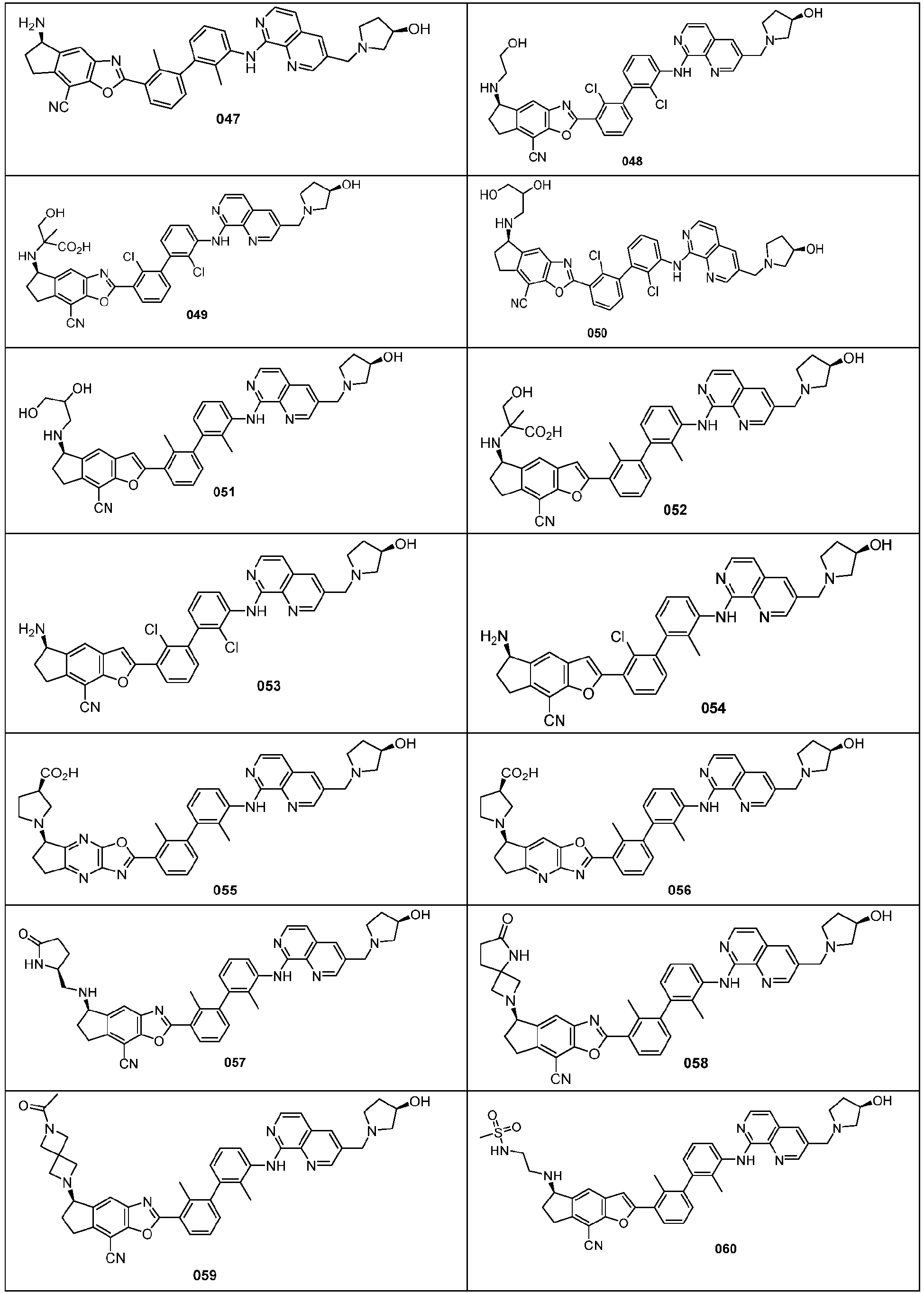

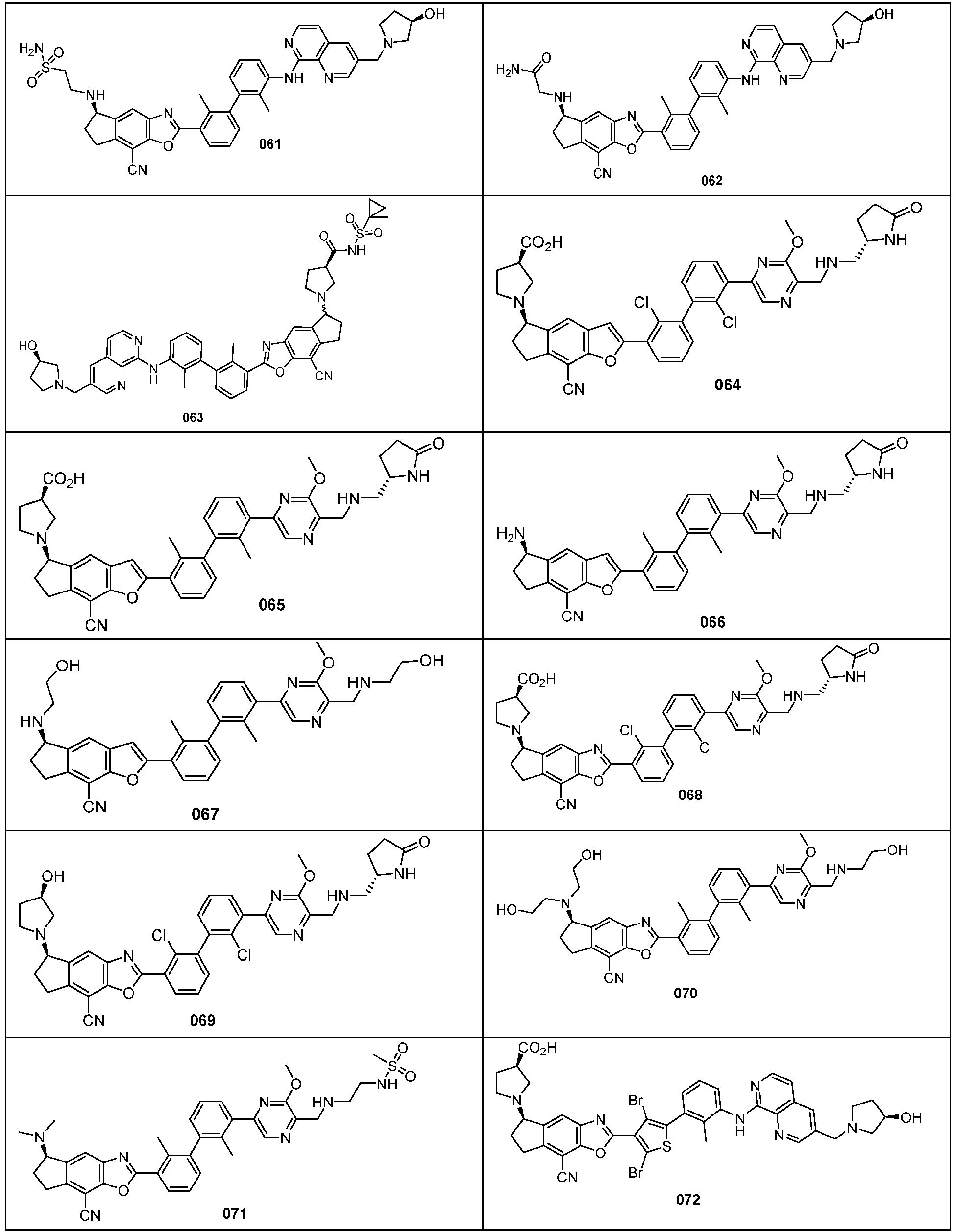

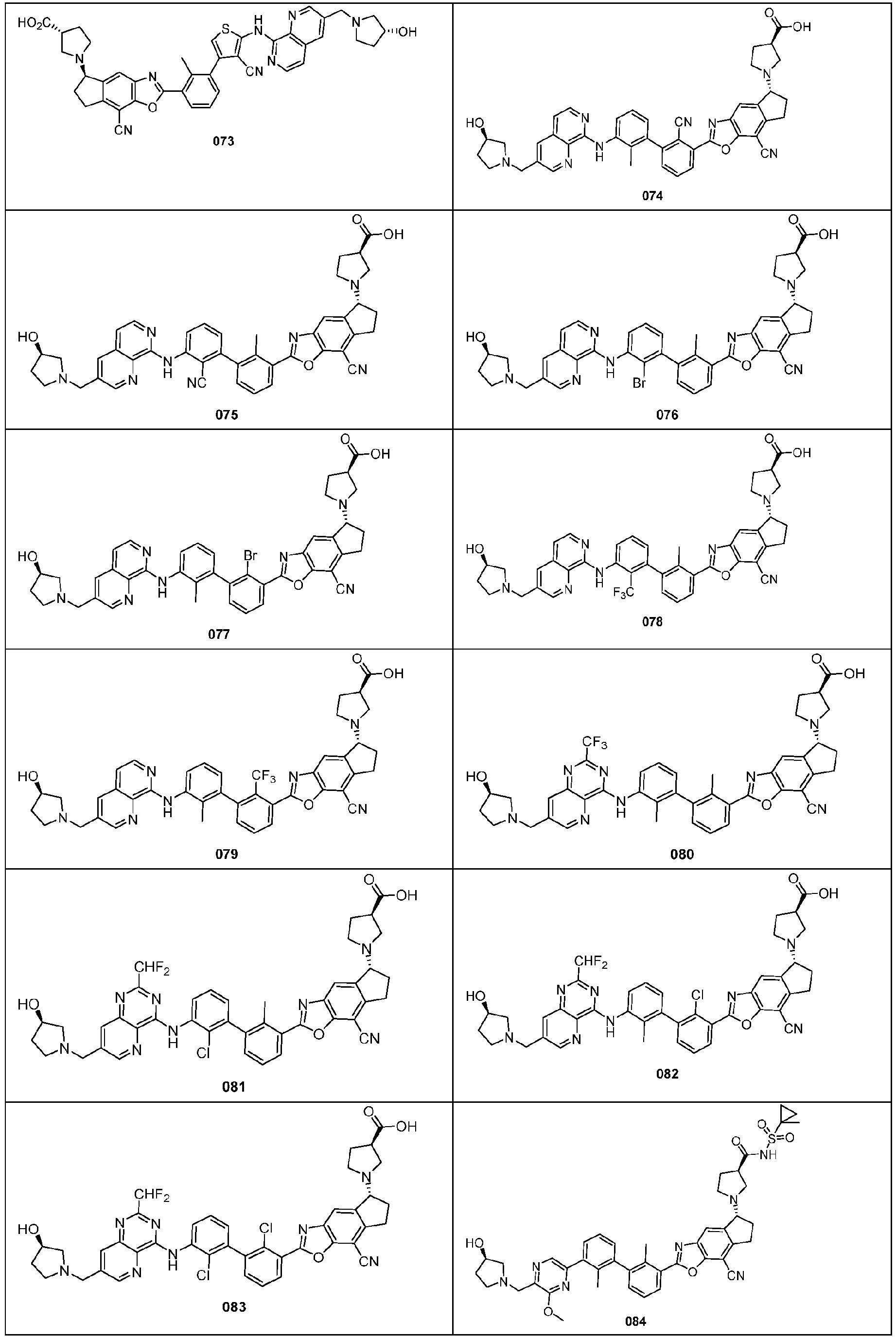

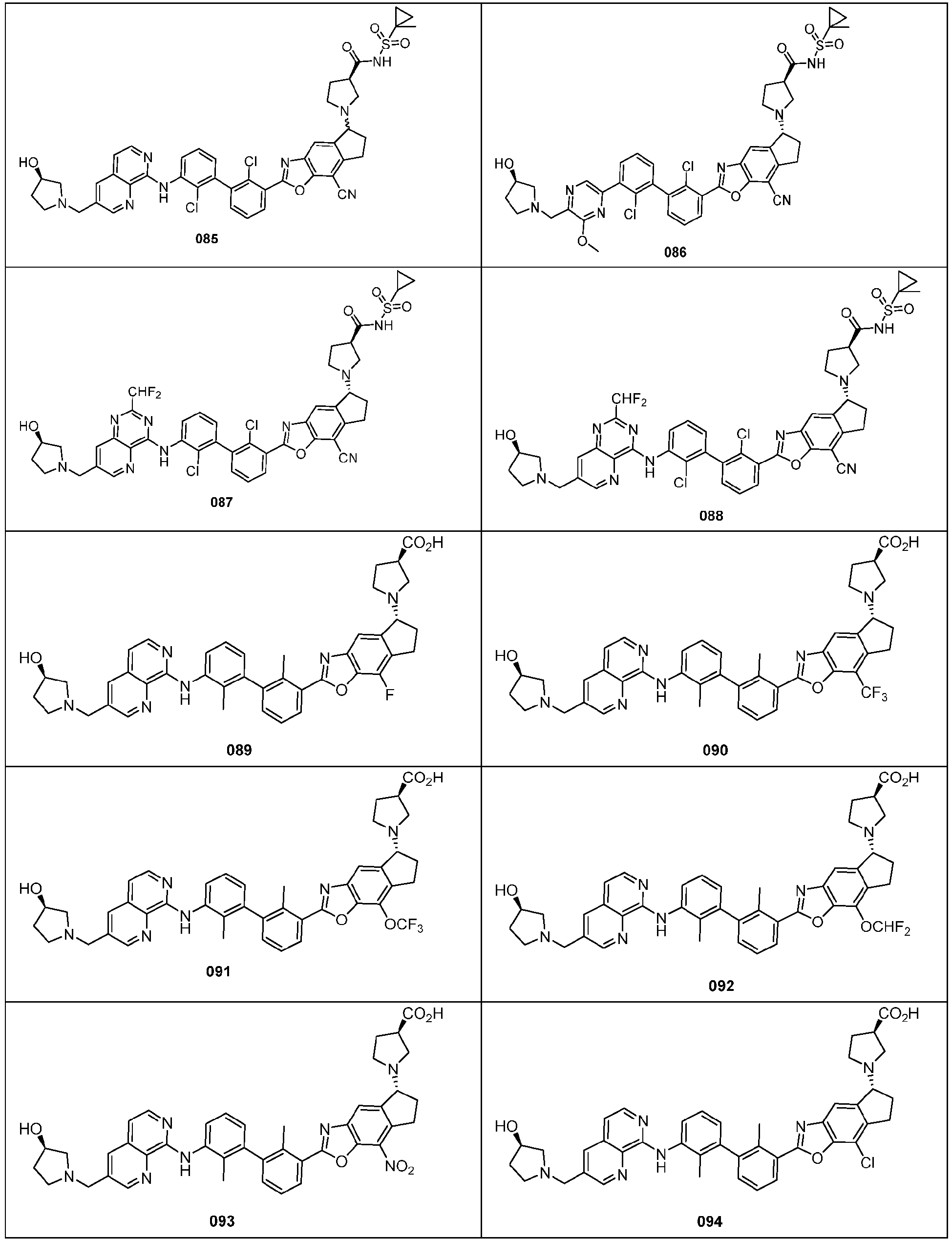

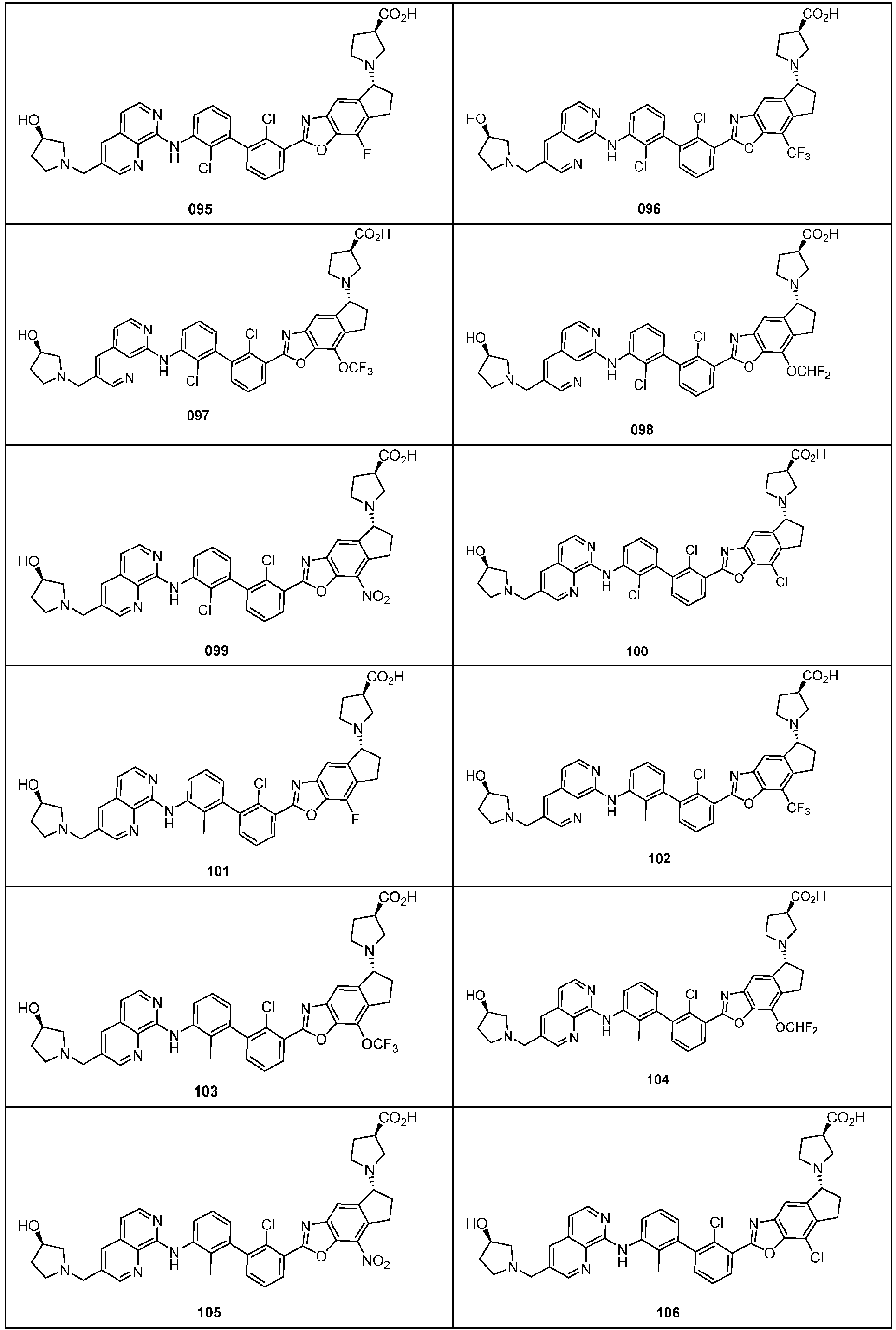

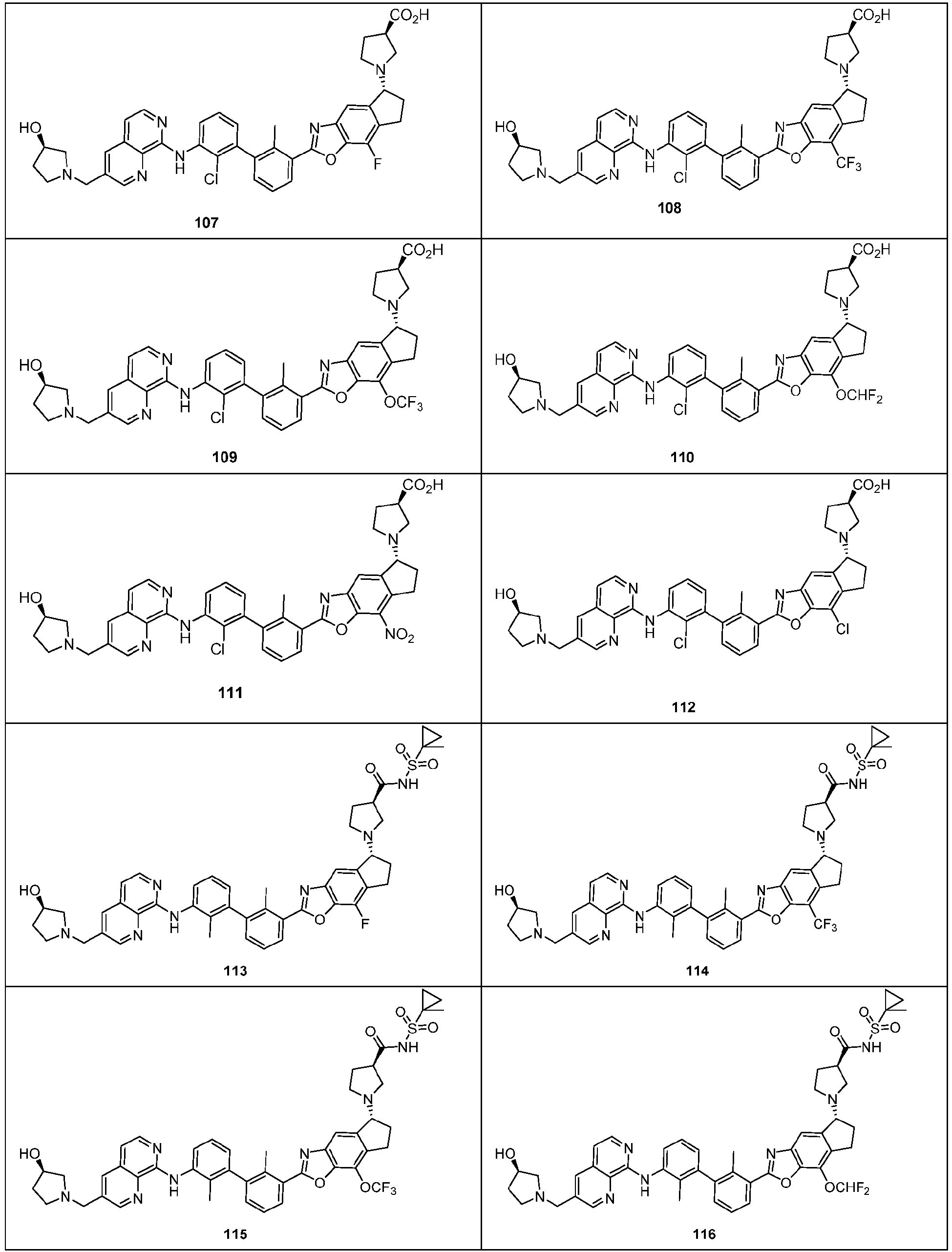

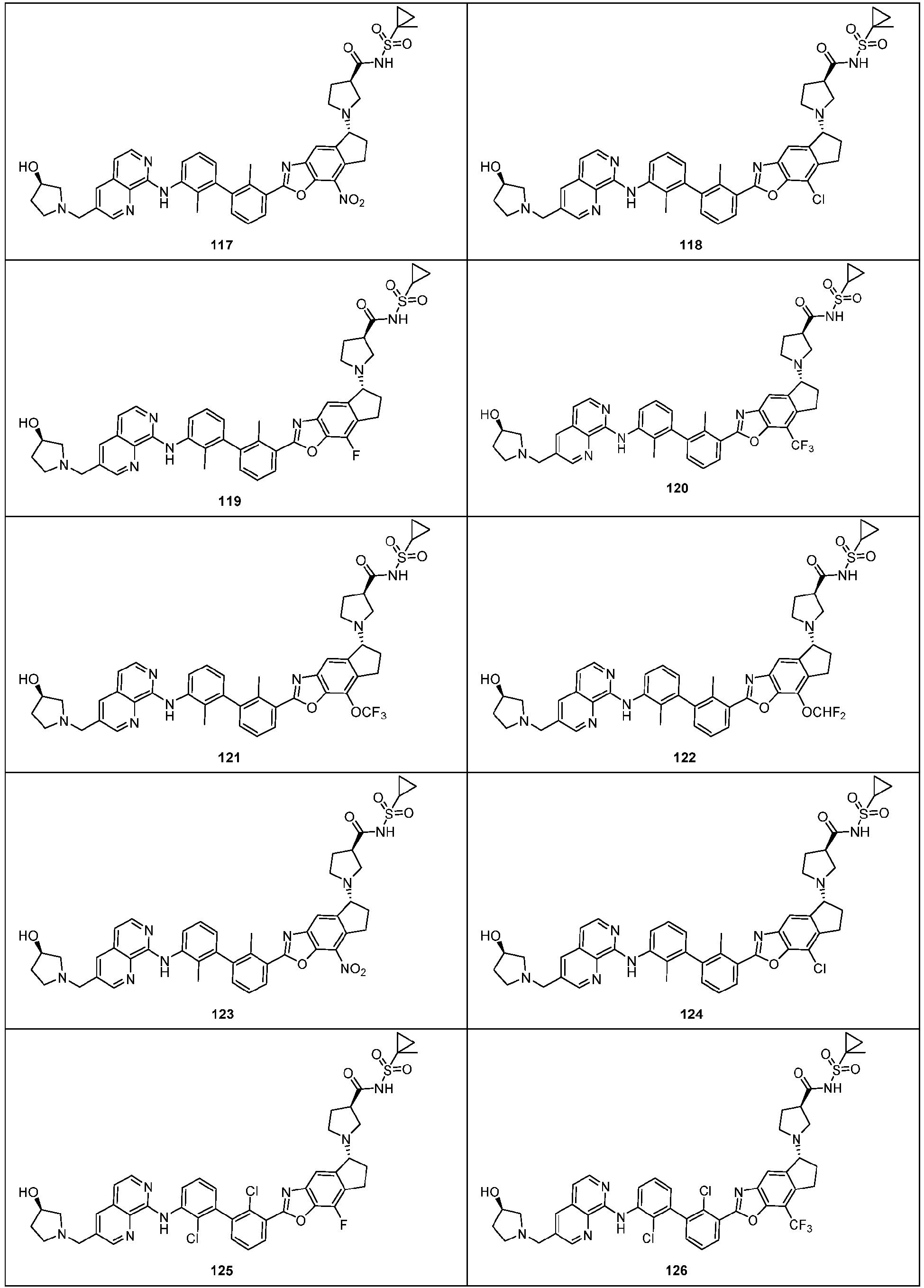

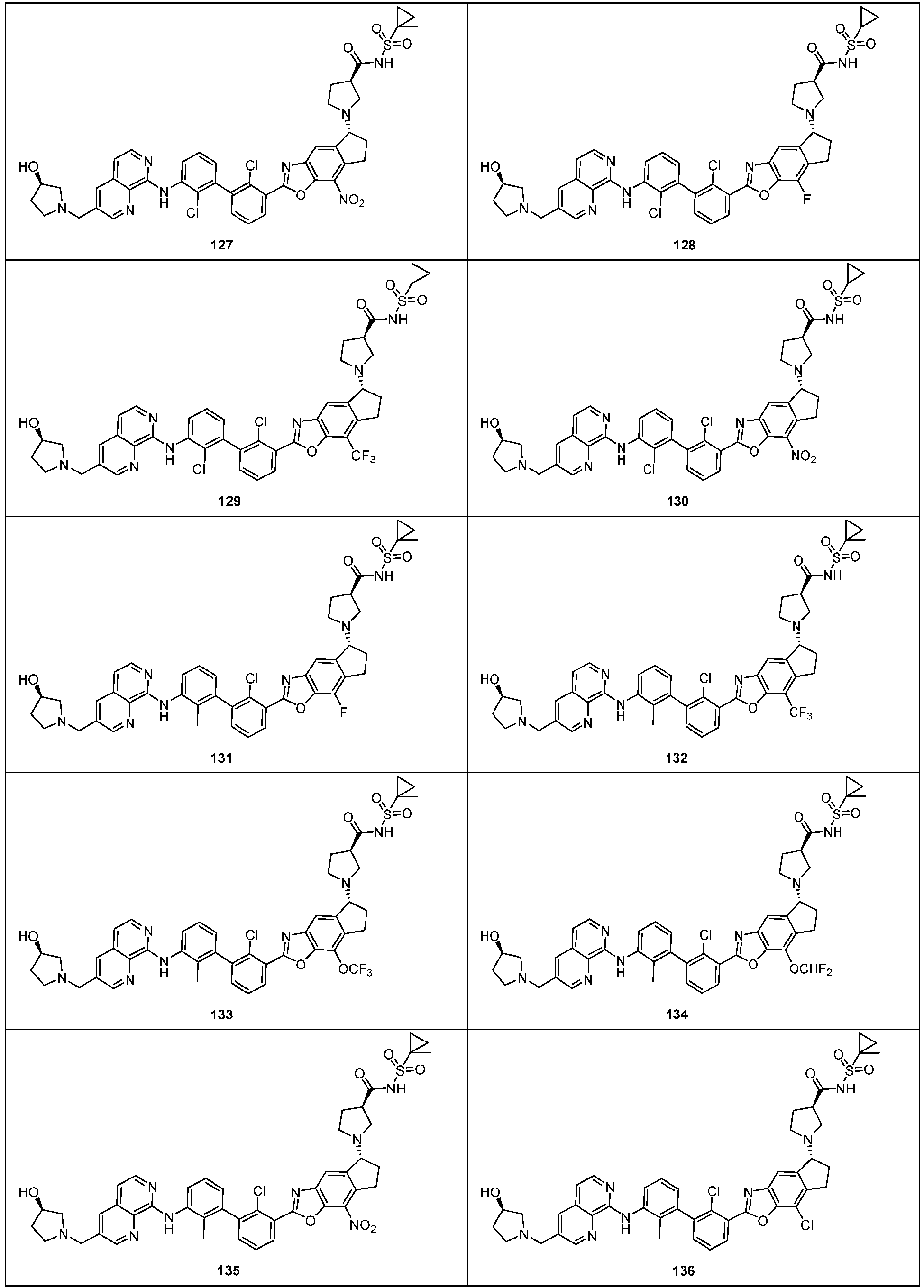

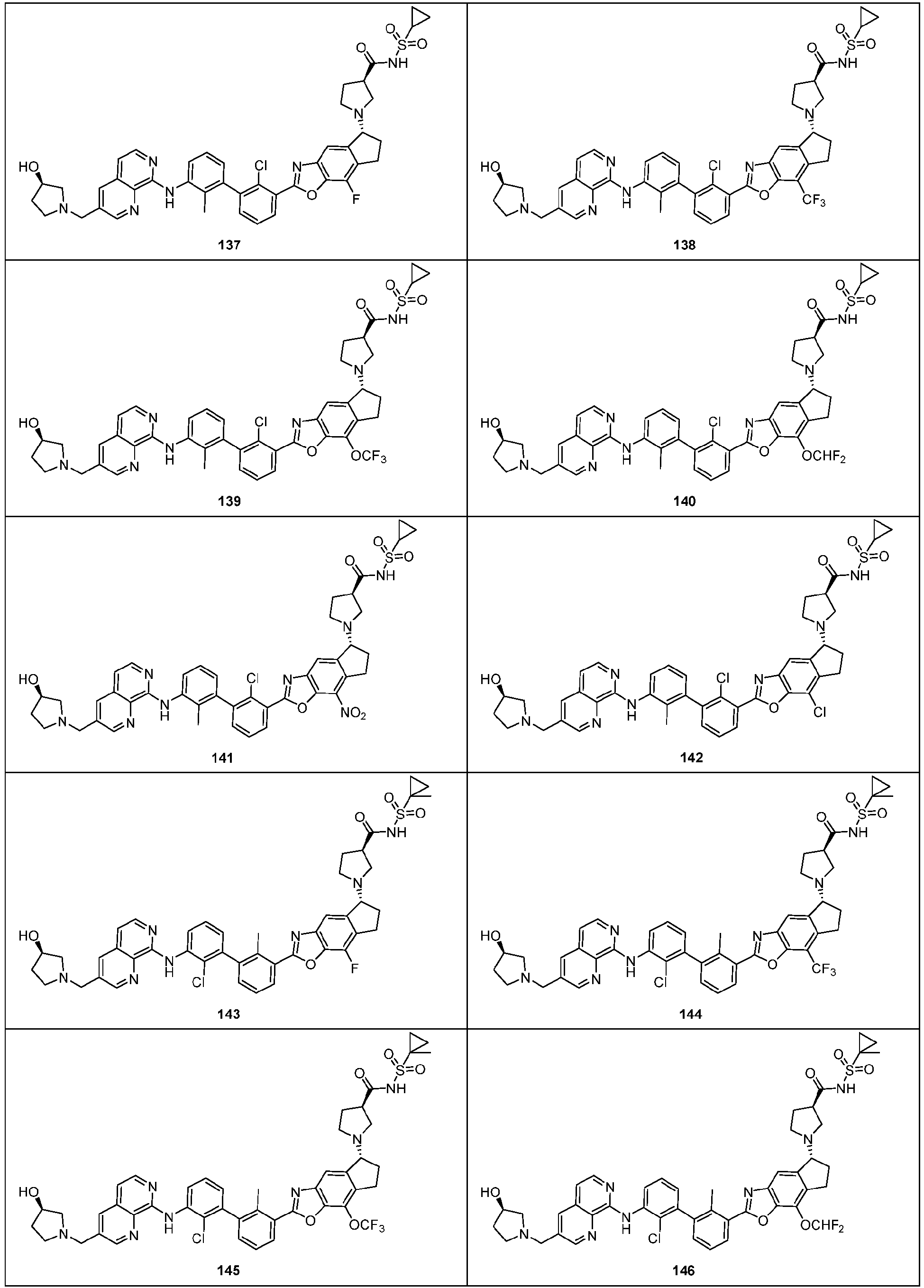

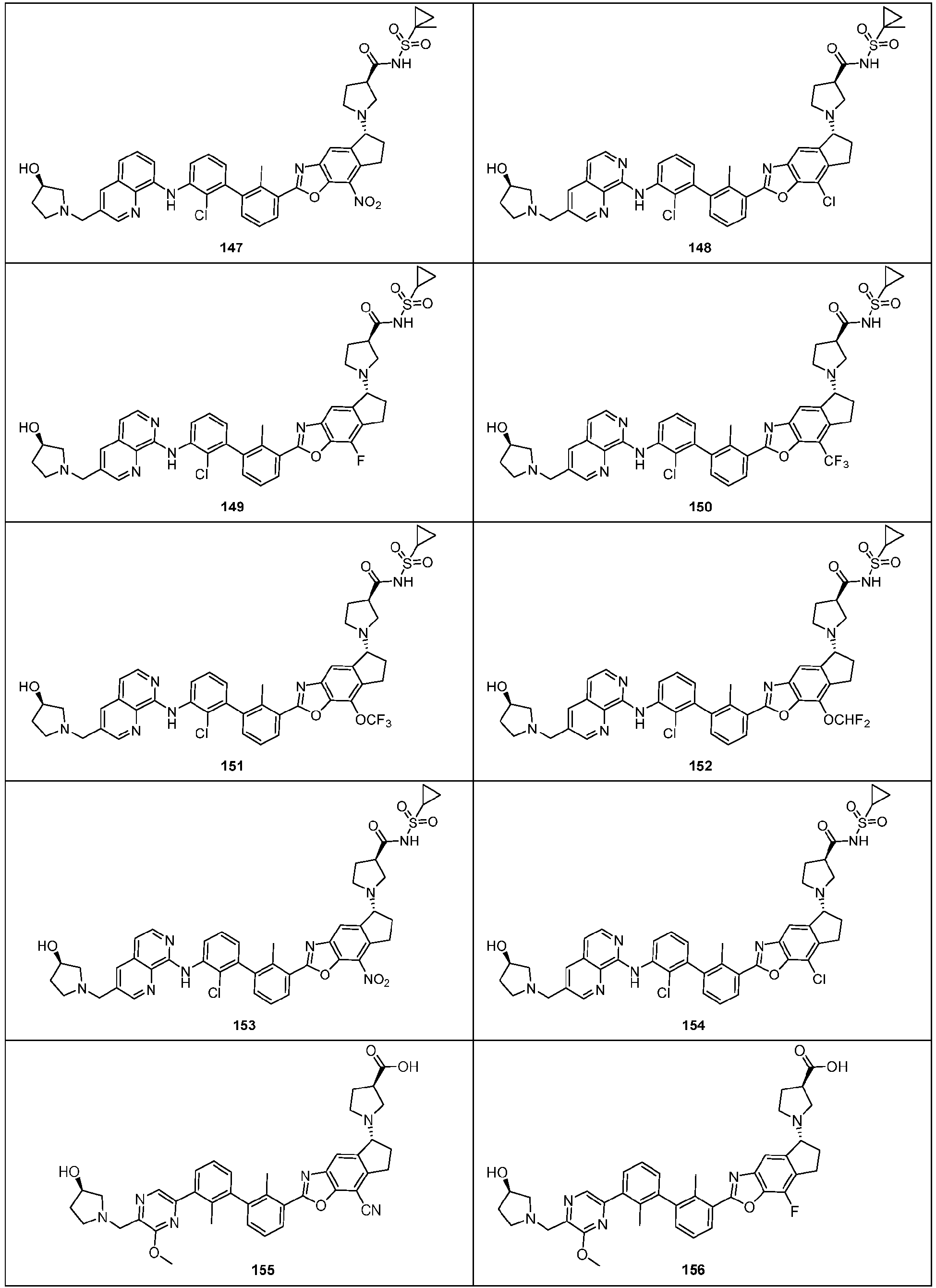

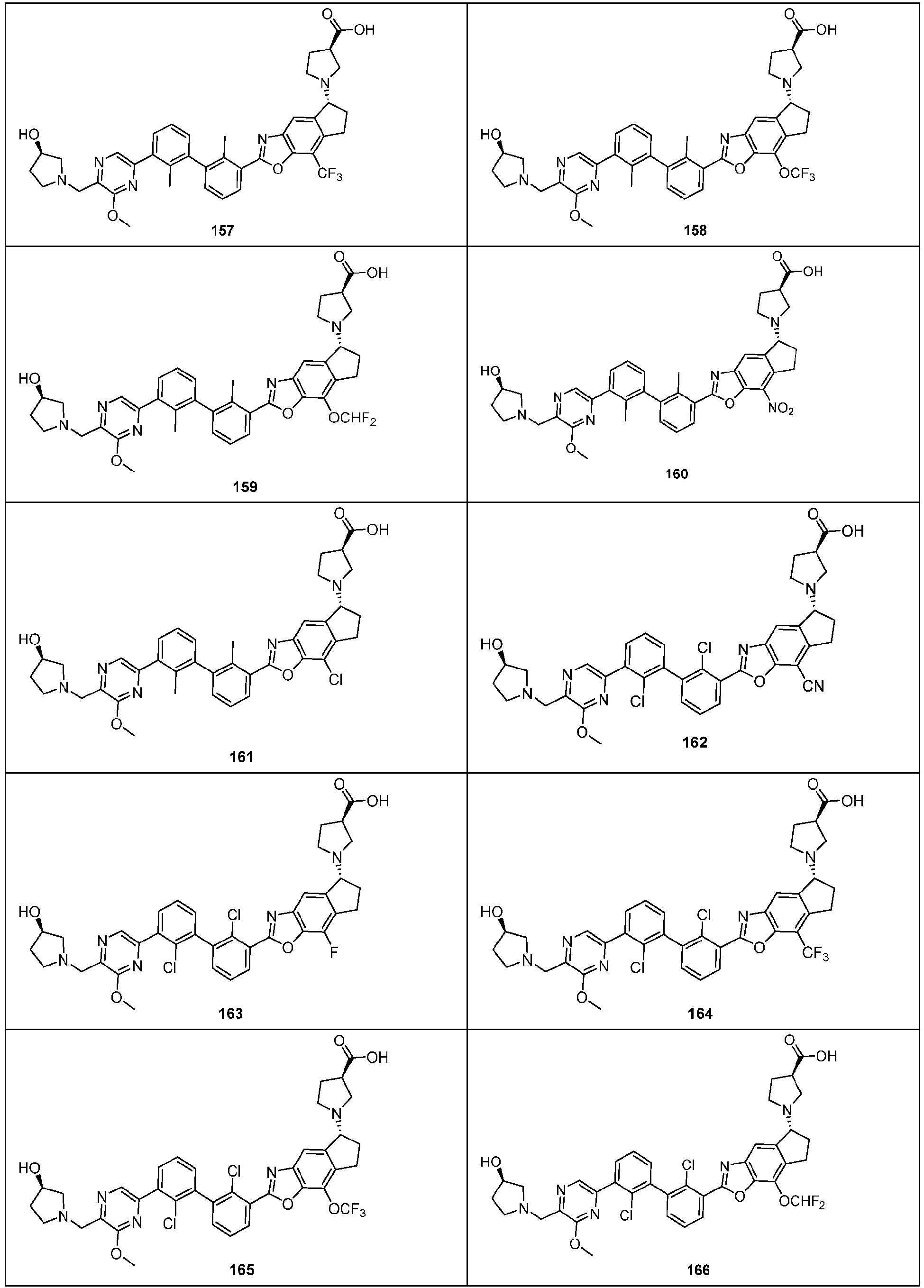

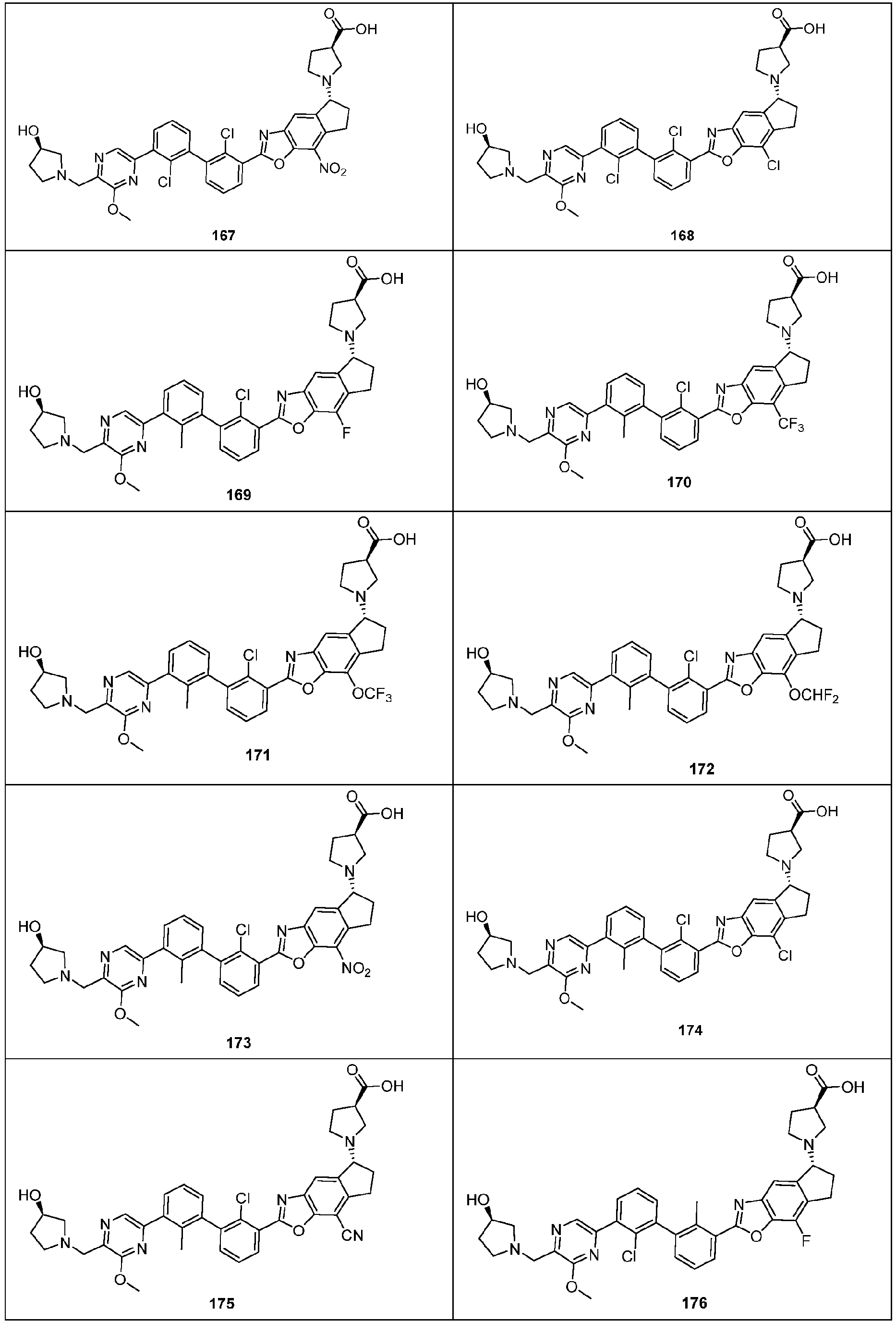

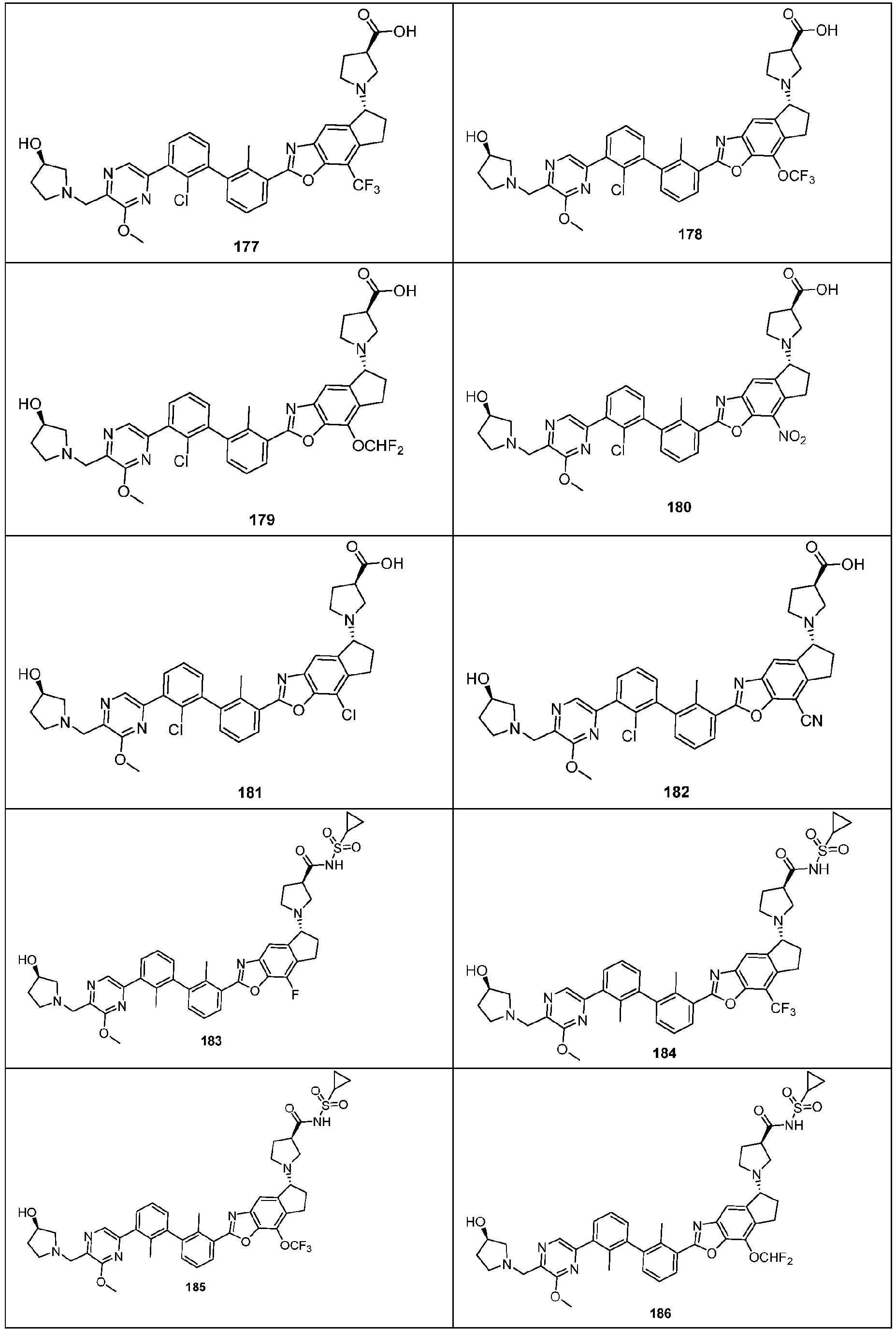

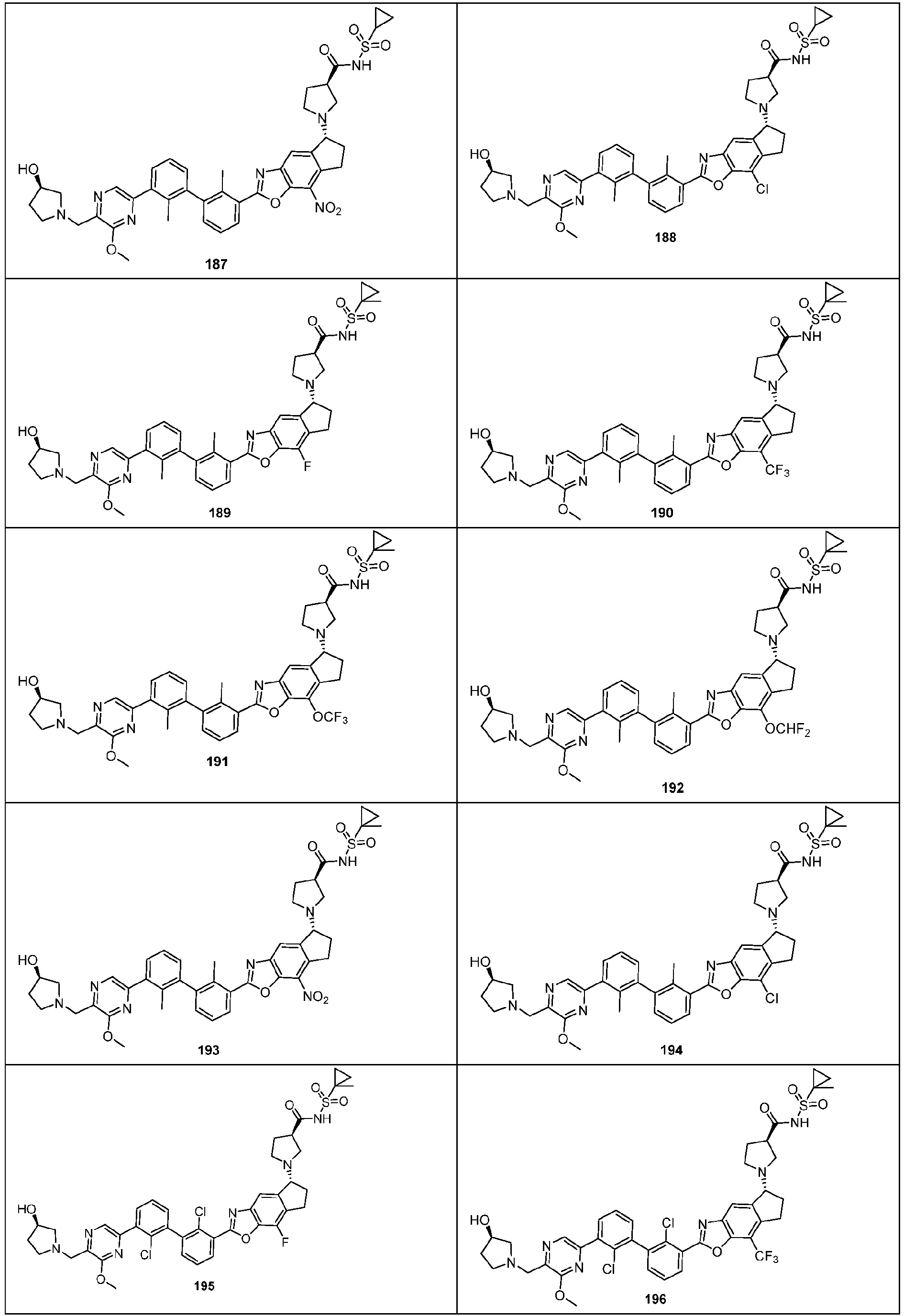

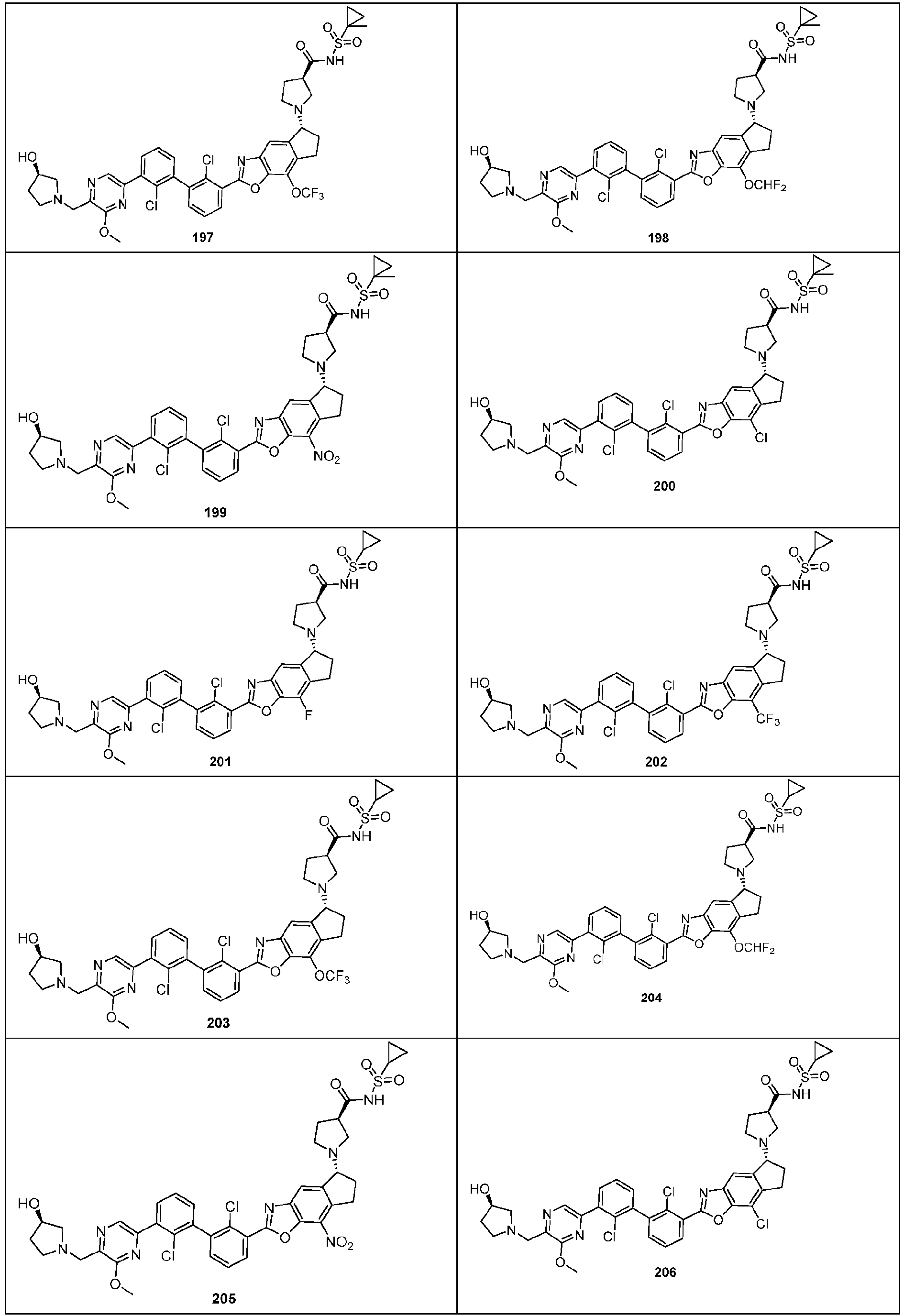

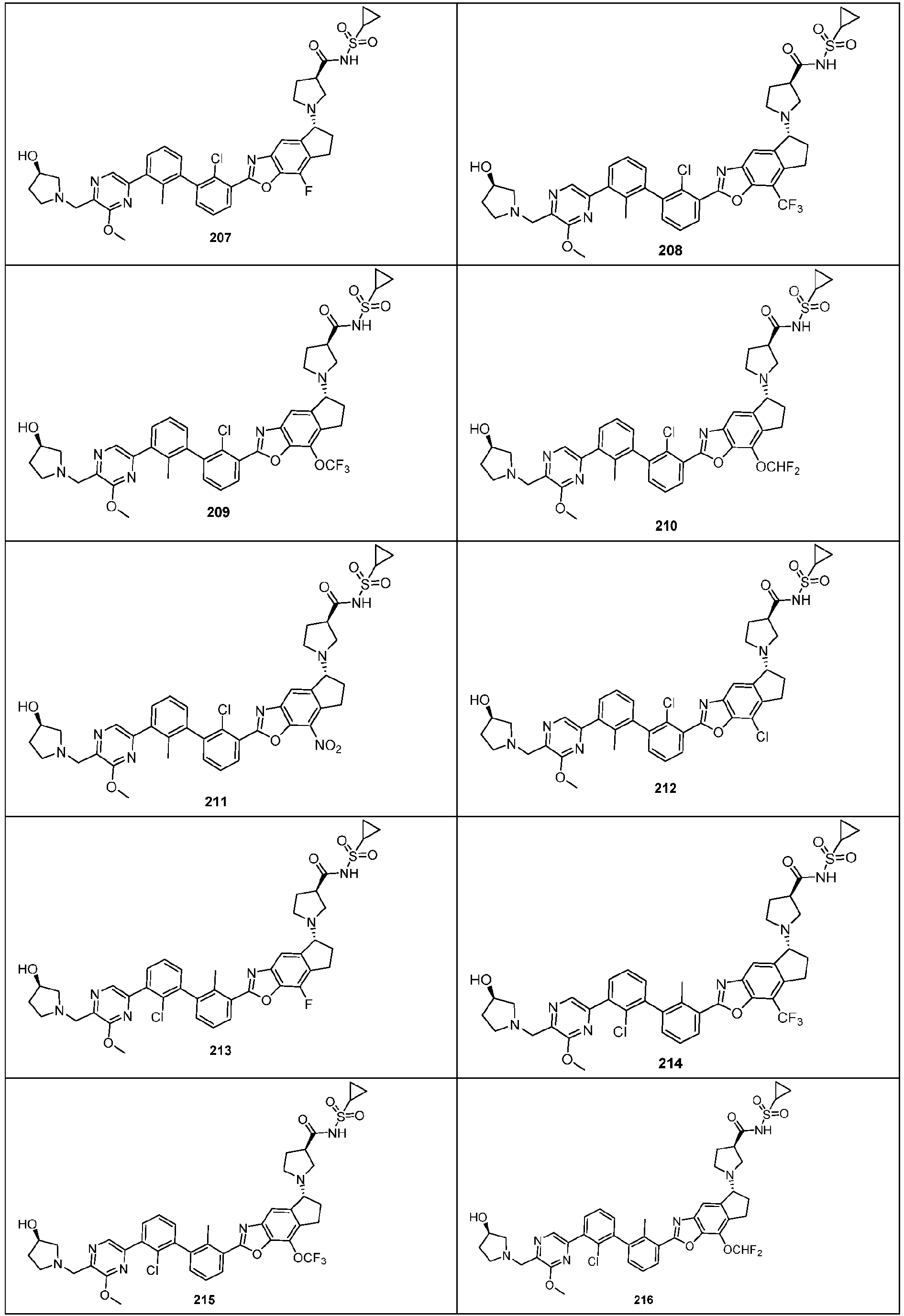

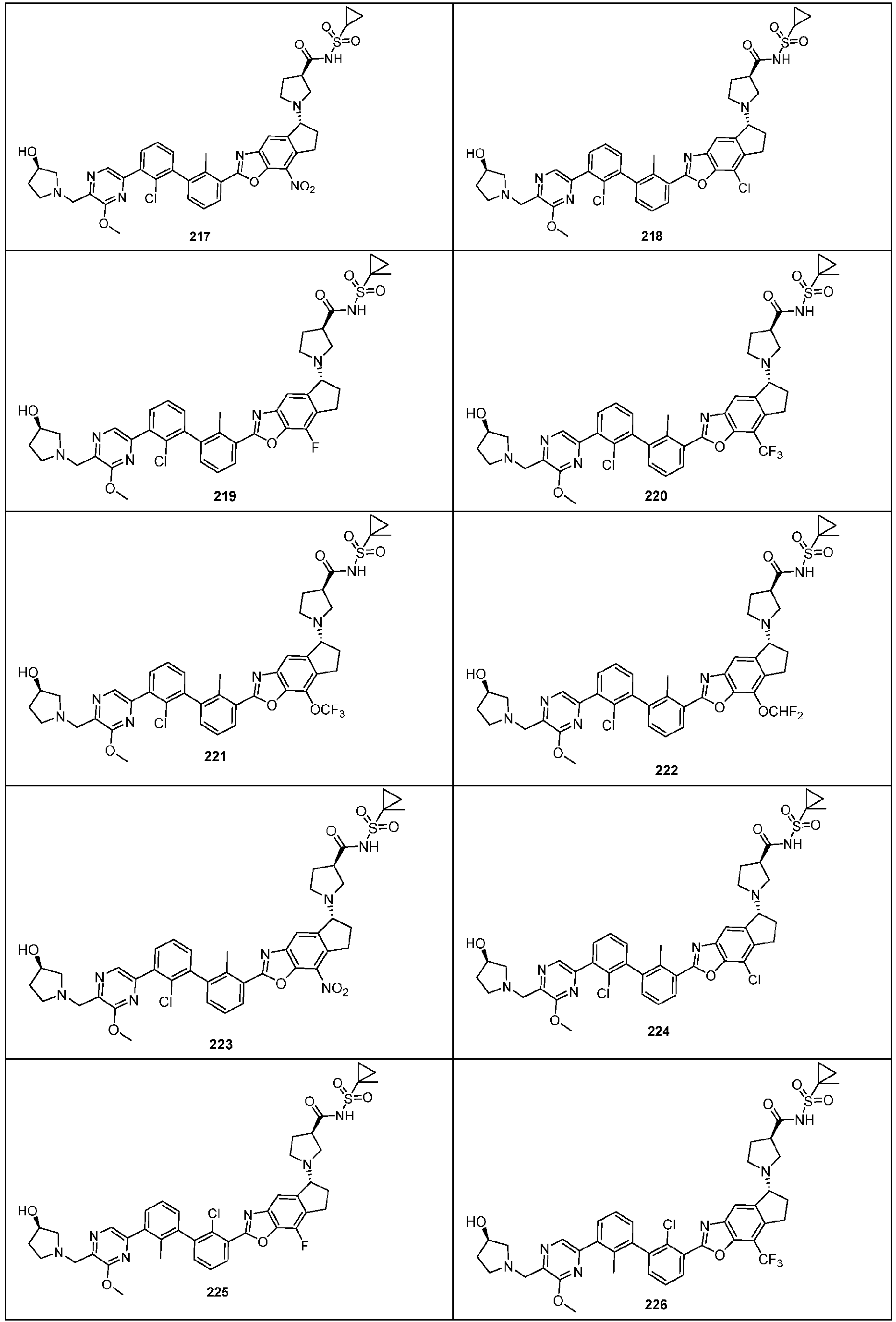

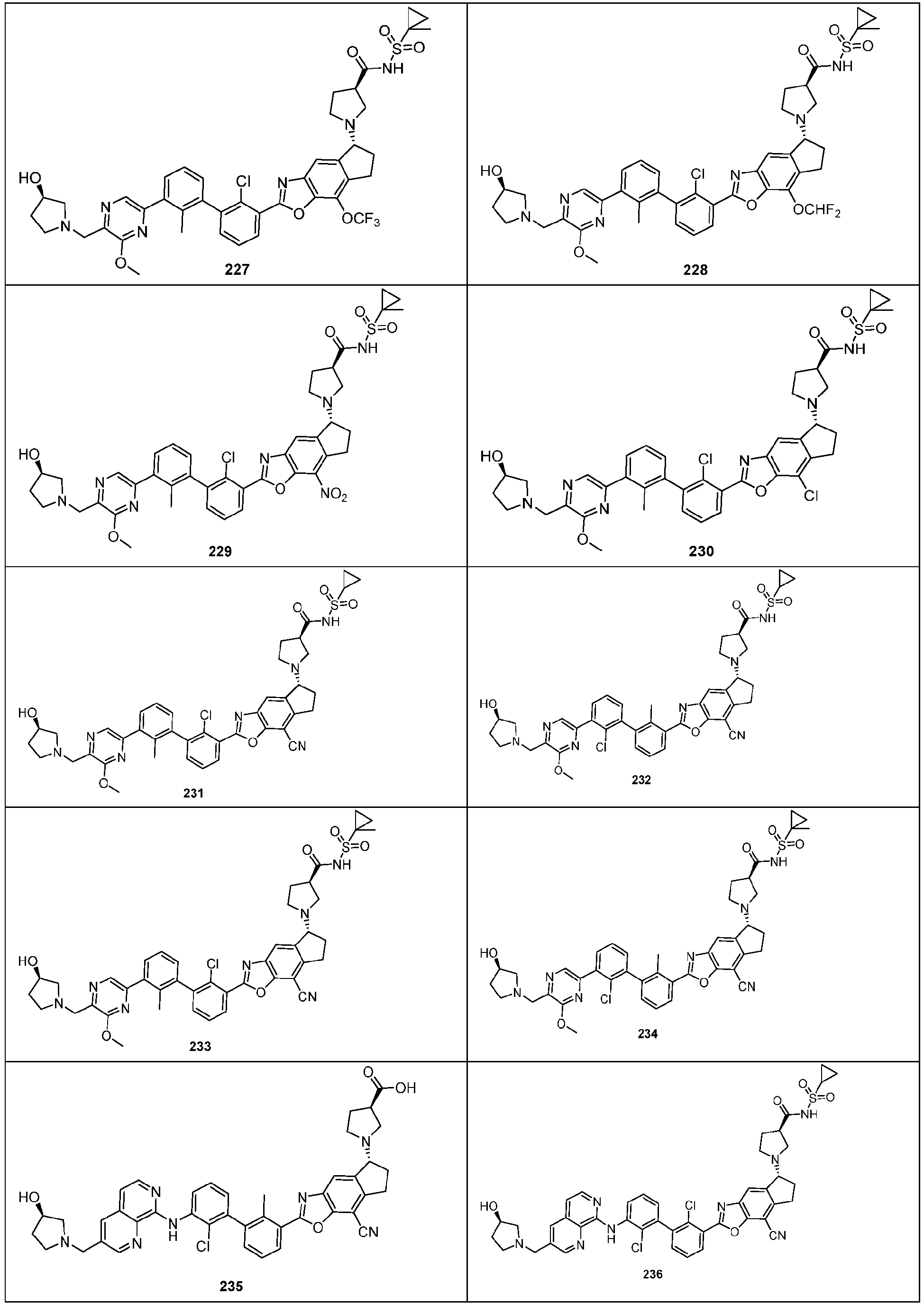

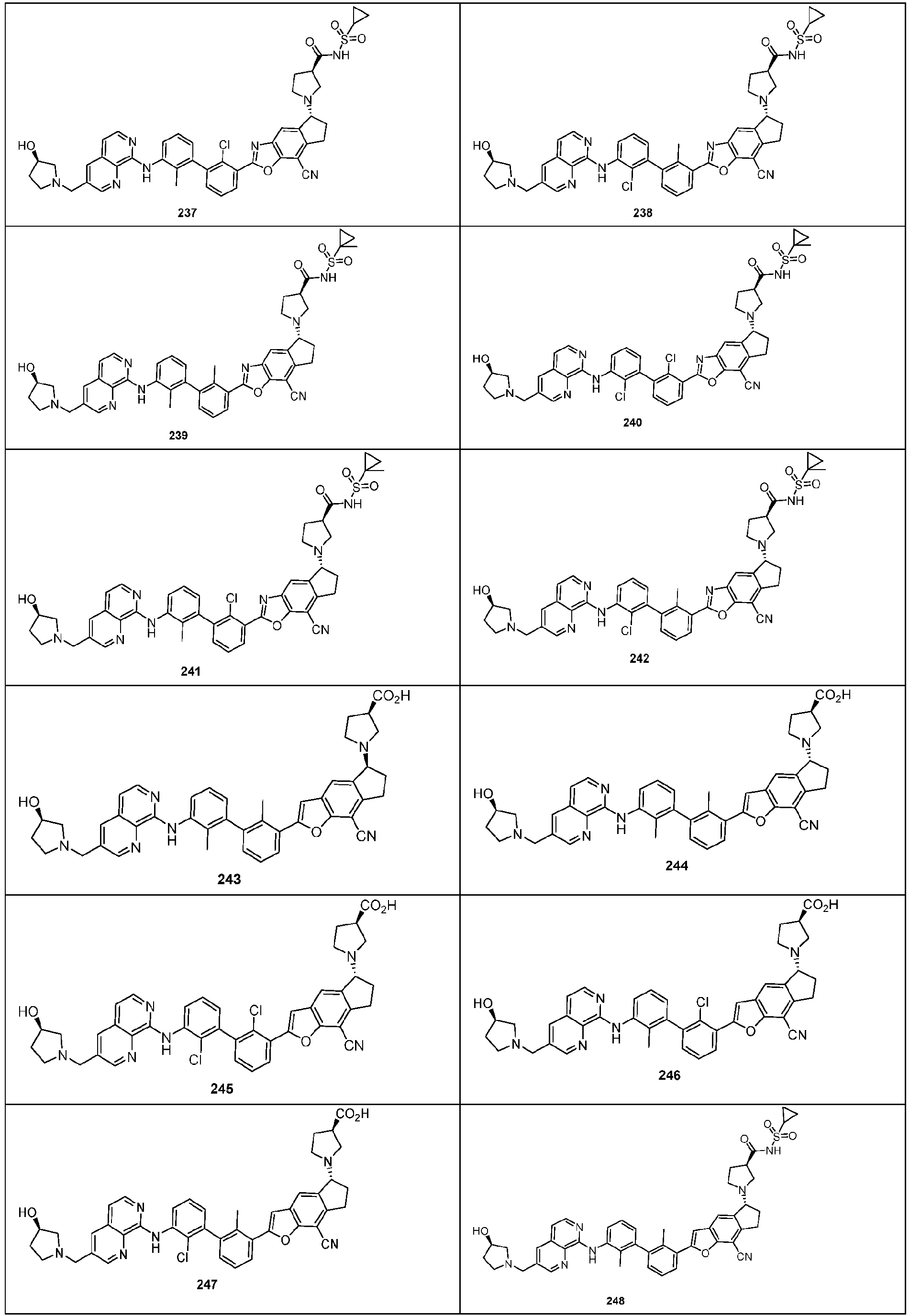

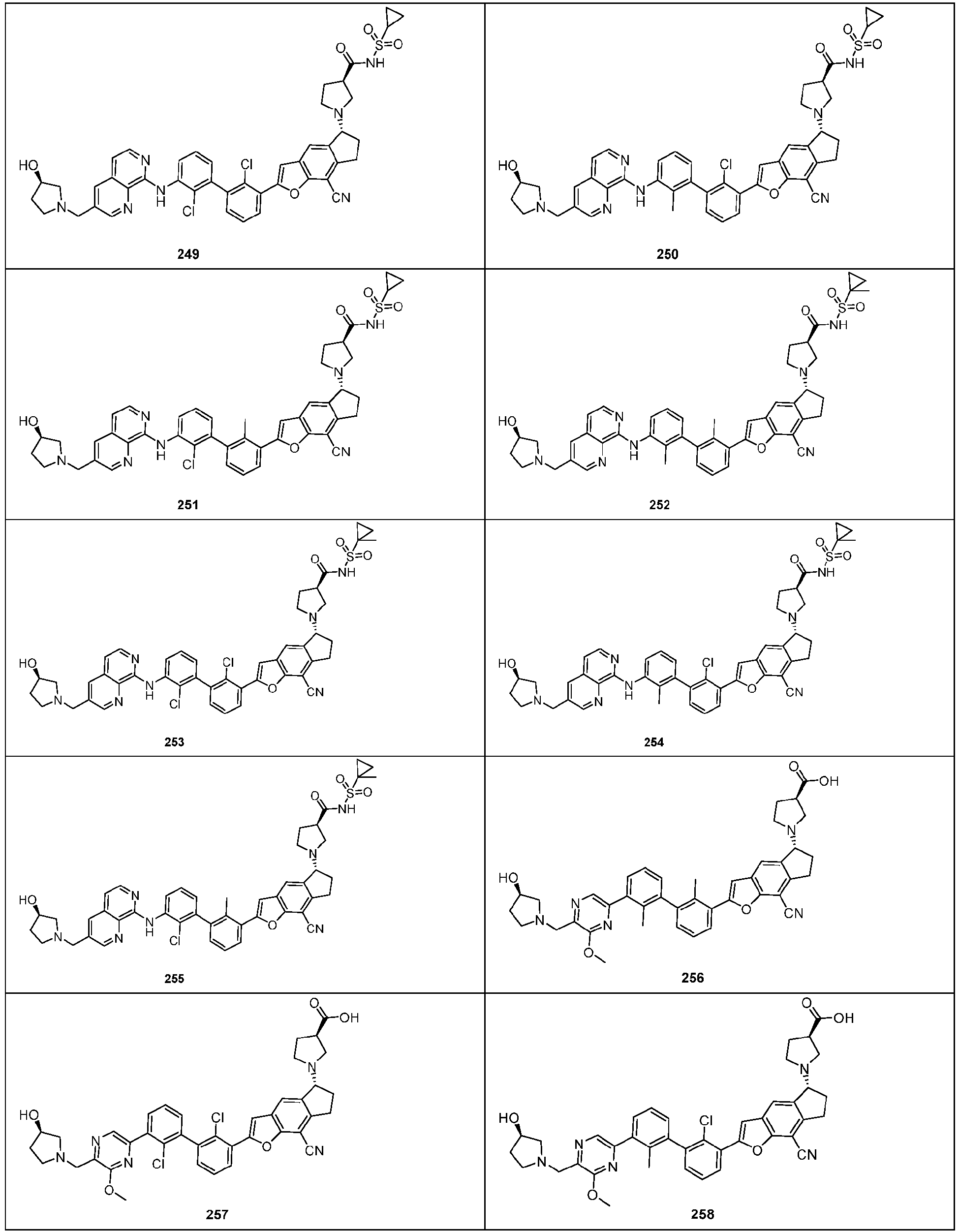

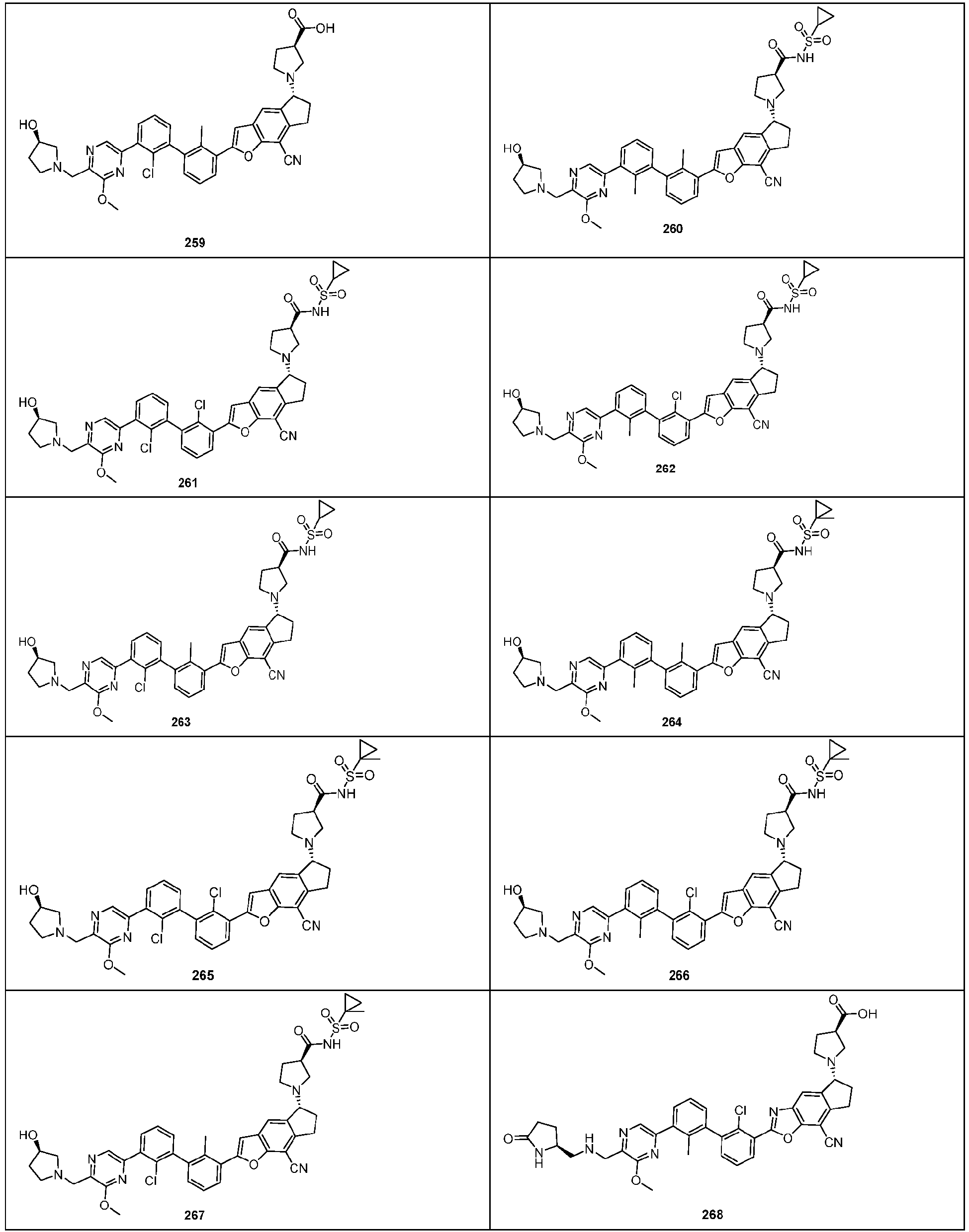

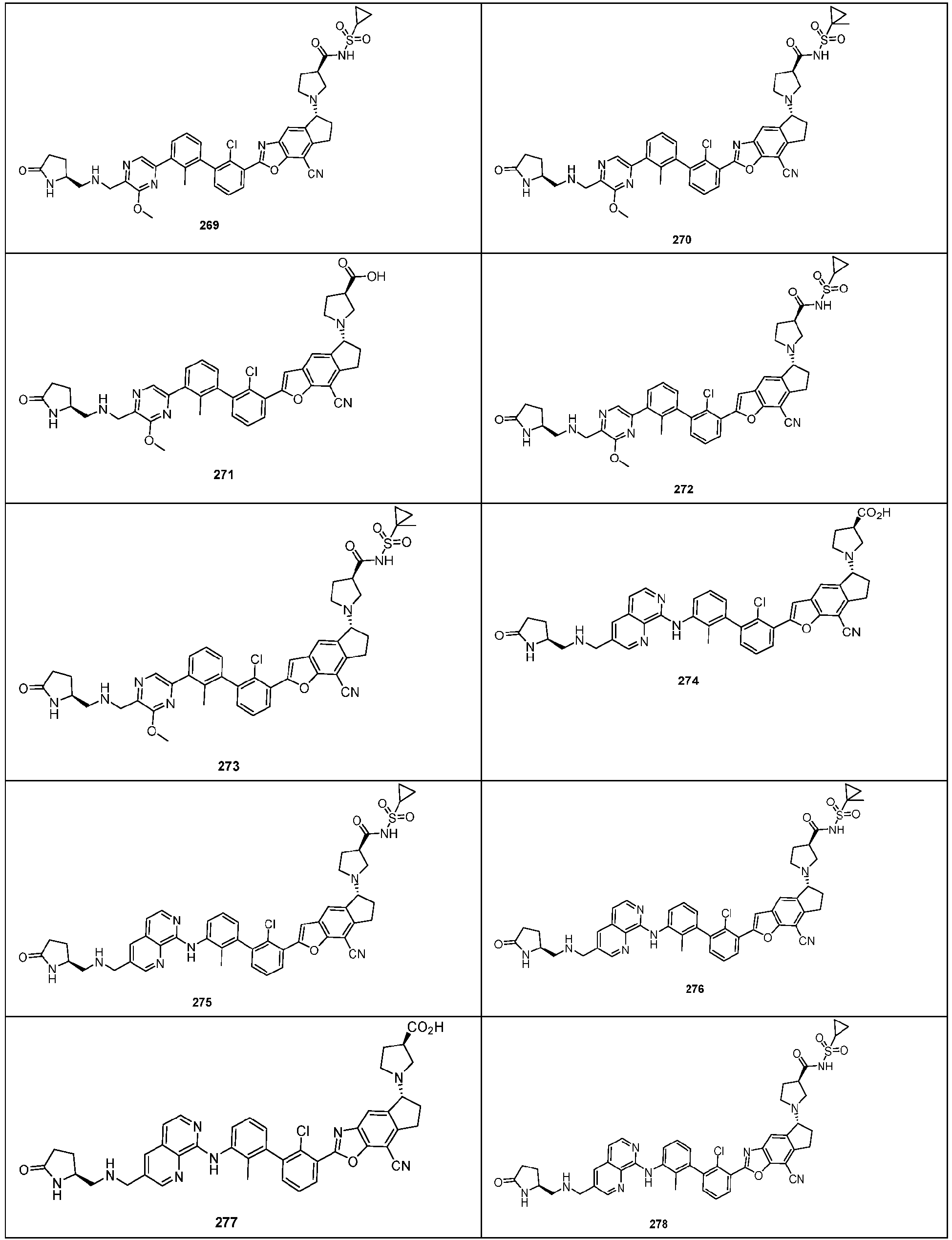

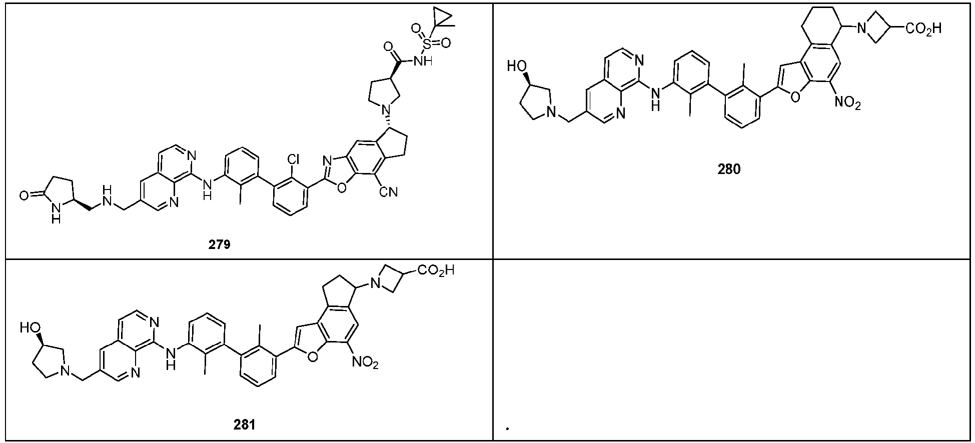

The second aspect of the present invention provides a preparation method of the compound of formula I according to the first aspect of the present invention, comprising steps selected from the steps shown in Synthesis Scheme 1, 2 or 3:
Synthesis Scheme 1: 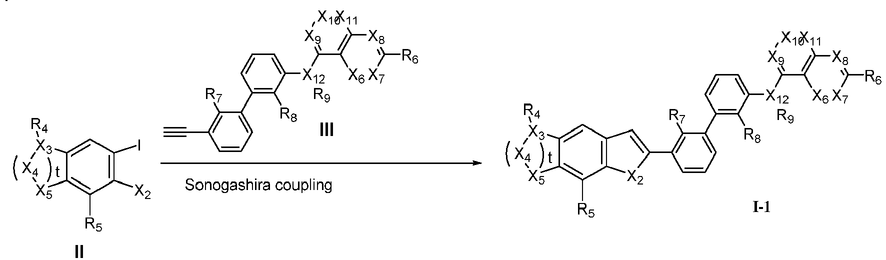
   (a) subjecting intermediates **II** and **III** as raw materials to Sonogashira coupling reaction catalyzed by palladium catalyst to obtain target product **1-1;**
   preferably, the preparation method of intermediate **II-2** is as follows: 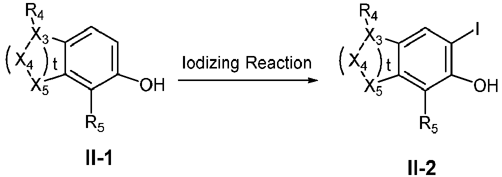
   (a) subjecting II-1 as a raw material to a halogenation reaction under the catalysis of Lewis acid to obtain intermediate **II-2;**
   preferably, the preparation method of intermediate **III** is as follows: 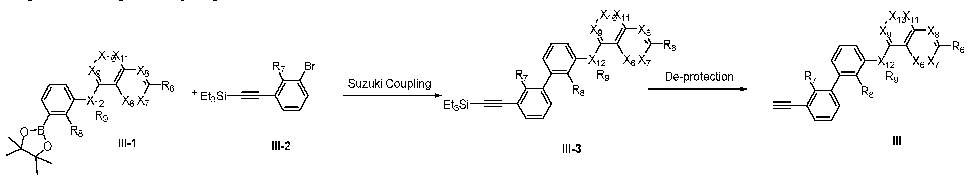
   (a) subjecting compound **III-1** and **III-2** as raw materials to a coupling reaction (such as Suzuki, Buchwald, etc.) under the condition of palladium catalyst and ligand to obtain intermediate **III-3;**
   (b) removing the silicon-based protecting group from **III-3** as a raw material by using a suitable reagent to obtain intermediate **III;**
Synthesis Scheme 2: 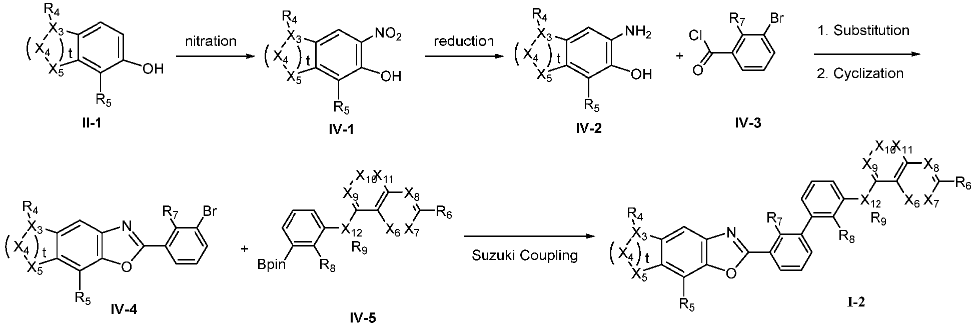
   (a) reacting compound **II-1** as a raw material with nitrifying agent (such as concentrated sulfuric acid/NaNO₃, concentrated sulfuric acid/fuming nitric acid, etc.) to obtain intermediate **IV-1;**
   (b) subjecting **IV-1** as a raw material to a reduction reaction under reducing condition (Pd-C/H₂; zinc powder/ammonium chloride; iron powder/acetic acid etc.) to form intermediate **IV-2;**
   (c) subjecting **IV-2** and **IV-3** as raw materials to an affinity substitution reaction under alkaline condition to obtain an amide intermediate; then to a cyclization reaction in the presence of a suitable dehydration reagent (such as PPh₃/DDQ) to obtain intermediate **IV-4;**
   (d) subjecting **IV-4** and **IV-5** as raw materials to a coupling reaction under the conditions of catalyst and ligand to form target intermediate **1-2;**
Synthesis Scheme 3: 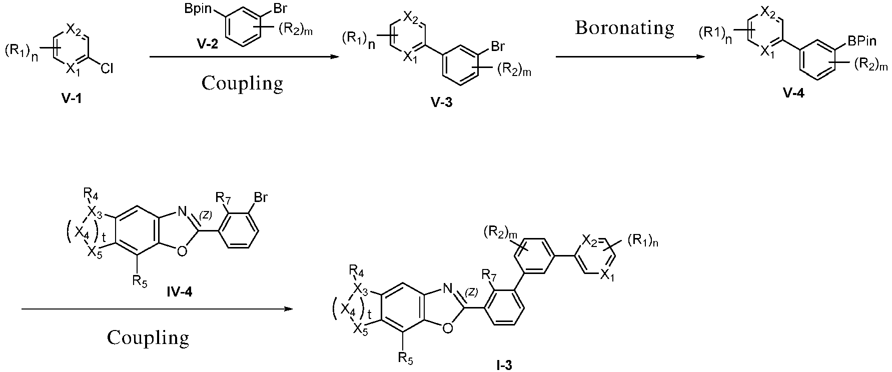
   (a) subjecting **V-1** and **V-2** as raw materials to a coupling reaction under the conditions of catalyst and ligand to form intermediate **V-3;**
   (d) subjecting **IV-4** and a suitable boron source (such as B₂Pin₂) as raw materials to a coupling reaction under the conditions of catalyst and ligand to form intermediate **V-4;**
   (d) subjecting **V-4** and **IV-4** as raw materials to a coupling reaction under the conditions of catalyst and ligand to form target product **1-3;**
   X₁-X₁₂, R₁-R₉, t, m, and n are defined as above.

The third aspect of the present invention provides a pharmaceutical composition, which comprises (1) the compound according to the first aspect of the present invention or a stereoisomer or a tautomer thereof, or a pharmaceutically acceptable salt, a hydrate or a solvate thereof; (2) a pharmaceutically acceptable carrier.

The fourth aspect of the present invention provides ause of the compound according to the first aspect of the present invention or a stereoisomer or a tautomer thereof or a pharmaceutically acceptable salt, a hydrate or a solvate thereof, or a pharmaceutical composition according to the third aspect of the present invention, for the preparation of a pharmaceutical composition for preventing and/or treating a disease related to the activity or expression of PD-1/PD-L1(HBV, HCV, solid tumors, hematological tumors, etc.).

The fifth aspect of the present invention provides a PD-1/PD-L1 modulator, which comprises the compound according to the first aspect of the present invention, or a stereoisomer or a tautomer thereof, or a pharmaceutically acceptable salt, hydrate or solvate thereof.

In another preferred embodiment, the pharmaceutical composition is used to treat a disease selected from the group consisting of cancer, infectious disease, and autoimmune disease.

In another preferred embodiment, the cancer is selected from the group consisting of pancreatic cancer, bladder cancer, colorectal cancer, breast cancer, prostate cancer, kidney cancer, hepatocellular carcinoma, lung cancer, ovary cancer, cervical cancer, stomach cancer, esophageal cancer, melanoma, neuroendocrine cancer, central nervous system cancer, brain cancer, bone cancer, soft tissue sarcoma, non-small cell lung cancer, small cell lung cancer or colon cancer, skin cancer, lung cancer, urinary system tumor, blood tumor, glioma, digestive system tumor, reproductive system tumor, lymphoma, nervous system tumor, brain tumor, head and neck cancer.

In another preferred embodiment, the cancer is selected from the group consisting of acute lymphocytic leukemia (ALL), acute myeloid leukemia (AML), chronic lymphocytic leukemia (CLL), small lymphocytic lymphoma (SLL), myelodysplastic syndrome (MDS), myeloproliferative disorder (MPD), chronic myeloid leukemia (CML), multiple myeloma (MM), non-hodgkin lymphoma (NHL), mantle cell lymphoma (MCL), follicular lymphoma, waldestrom macroglobulinemia (WM), T-cell lymphoma, B-cell lymphoma, and diffuse large B-cell lymphoma (DLBCL).

In another preferred embodiment, the infectious disease is selected from bacterial infection and viral infection.

In another preferred embodiment, the autoimmune disease is selected from the group consisting of organ-specific autoimmune disease and systemic autoimmune disease.

In another preferred embodiment, the organ-specific autoimmune diseases include chronic lymphocytic thyroiditis, hyperthyroidism, insulin-dependent diabetes mellitus, myasthenia gravis, ulcerative colitis, pernicious anemia with chronic atrophic gastritis, pulmonary hemorrhagic nephritic syndrome, primary biliary cirrhosis, multiple cerebral sclerosis, acute idiopathic polyneuritis.

In another preferred embodiment, the systemic autoimmune diseases include rheumatoid arthritis, systemic lupus erythematosus, systemic vasculitis, scleroderma, pemphigus, dermatomyositis, mixed connective tissue disease, autoimmune hemolytic anemia.

In another preferred embodiment, the pharmaceutical composition is also used to improve T cell function in a patient with chronic hepatitis B (CHB).

In another preferred embodiment, the inhibitor further comprises at least one therapeutic agent selected from the group consisting of nivolumab, pembrolizumab, atezolizumab, and ipilimumab.

The sixth aspect of the present invention provides a method for regulating the interaction of PD-1/PD-L1 *in vivo,* wherein comprising the steps of: contacting the compound according to the first aspect of the present invention, or a stereoisomer or a tautomer thereof, or a pharmaceutically acceptable salt, a hydrate or a solvate thereof with a PD-L1 protein.

### DETAILED DESCRIPTION OF THE INVENTION

After extensive and intensive research, the present inventors discovered a class of PD-1/PD-L1 interaction modulators with excellent regulatory effect. The present invention has been completed on this basis.

### Definitions

As used herein, the term "alkyl" includes straight or branched alkyl groups. For example, C₁-C₈ alkyl refers to straight or branched alkyls having from 1-8 carbon atoms, such as methyl, ethyl, propyl, isopropyl, butyl, isobutyl, tert-butyl, and the like.

As used herein, the term "alkenyl" includes straight or branched alkenyl groups. For example, C2-C6 alkenyl refers to straight or branched alkenyl groups having 2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, "C₂-C₆ alkynyl" refers to straight or branched alkynyl group having 2-6 carbon atoms, such as ethynyl, propynyl, butynyl, and the like.

As used herein, the term "C₃-C₁₀ cycloalkyl" refers to cycloalkyl groups having 3 to 10 carbon atoms. It may be a monocyclic ring, such as cyclopropyl, cyclobutyl, cyclopentyl, cyclohexyl, and the like. It may also be in bicyclic form, such as bridged or spiro ring form.

As used herein, the term "C₁-C₈ alkylamino" refers to amine groups substituted with C1-C8 alkyl group, which may be mono- or di-substituted; for example, methylamino, ethylamino, propylamino, isopropylamine, butylamine, isobutylamine, tert-butylamine, dimethylamine, diethylamine, dipropylamine, diisopropylamine, dibutylamine, diisobutylamine, di-tert-butylamine, and the like.

As used herein, the term "C₁-C₈ alkoxy" refers to straight or branched alkoxy groups having 1-8 carbon atoms; for example, methoxy, ethoxy, propoxy, isopropoxy, butoxy, isobutoxy, tert-butoxy, and the like.

As used herein, the term "3-10 membered heterocycloalkyl having 1-3 heteroatoms selected from the group consisting of N, S and O" refers to a saturated or partially saturated cyclic group having 3-10 atoms, wherein 1-3 atoms are heteroatoms selected from the group consisting of N, S and O. It may be a monocyclic ring or in a bicyclic form, such as bridged or spiro ring form. Specific examples may be oxetane, azetidine, tetrahydro-2H-pyranyl, piperidinyl, tetrahydrofuranyl, morpholinyl, pyrrolidinyl, and the like.

As used herein, the term "C₆-C₁₀ aryl" refers to aryl groups having 6 to 10 carbon atoms, such as phenyl, naphthyl, and the like.

As used herein, the term "5-10 membered heteroaryl having 1-3 heteroatoms selected from the group consisitng of N, S and O" refers to cyclic aromatic groups having 5-10 atoms, of which 1-3 atoms are selected from the group consisting of N, S and O. It may be a monocyclic ring or in a fused ring form. Specific examples may be pyridyl, pyridazinyl, pyrimidinyl, pyrazinyl, triazinyl, pyrrolyl, pyrazolyl, imidazolyl, (1,2,3)-triazolyl and (1,2,4)-triazolyl, tetrazyl, furyl, thienyl, isoxazolyl, thiazolyl, oxazolyl and the like.

Unless otherwise specified, the group described in the present invention is "substituted or unsubstituted", the group of the present invention can be substituted by a substituent selected from the group consisting of halogen, nitrile, nitro, hydroxy, amino, C₁-C₆ alkyl-amine, C₁-C₆ alkyl, C2-C6 alkenyl, C2-C6 alkynyl, C₁-C₆ alkoxy, halogenated C₁-C₆ alkyl, halogenated C2-C6 alkenyl, halogenated C2-C6 alkynyl, halogenated C₁-C₆ alkoxy, allyl, benzyl, C6-C12 aryl, C₁-C₆ alkoxy-C₁-C₆ alkyl, C₁-C₆ alkoxy-carbonyl, phenoxycarbonyl, C2-C6 alkynyl-carbonyl, C2-C6 alkenyl-carbonyl, C3-C6 cycloalkyl-carbonyl, C₁-C₆ alkyl-sulfonyl, etc.

As used herein, "halogen" or "halogen atom" refers to F, Cl, Br, and I. More preferably, the halogen or halogen atom is selected from F, Cl and Br. "Halogenated" means substitution with an atom selected from F, Cl, Br, and I.

Unless otherwise specified, the structural formula described herein are intended to include all isomeric forms (such as enantiomeric, diastereomeric, and geometric isomers (or conformational isomers)): for example, R, S configuration of asymmetrical centers, (Z), (E) isomers of double bonds, etc. Therefore, the single stereochemical isomers or enantiomers, diastereomers or geometric isomers (or conformers) of the compounds of the invention, or mixtures thereof all fall within the scope of the invention.

Unless otherwise specified, the structural formulae described herein are intended to include all possible deuterated derivatives (ie, one or more hydrogen atoms in the molecule are substituted by D).

As used herein, the term "tautomer" means that structural isomers having different energies can exceed the low energy barrier and thereby transform between each other. For example, proton tautomers (proton shift) includes interconversion by proton transfer, such as 1H-carbazole and 2H-carbazole. Valence tautomers include interconversion through some bonding electron recombination.

As used herein, the term "solvate" refers to a complex formed by coordinating a compound of the invention with a solvent molecule in specific proportion.

As used herein, the term "hydrate" refers to a complex formed by coordinating a compound of the invention with water.

### Active ingredient

As used herein, "compounds of the present invention" refers to compounds of formula I, and also includes various crystalline forms, pharmaceutically acceptable salts, hydrates or solvates of the compounds of formula I.

Preferred compounds of the present invention include compounds 1-360 (including various Rand/or S-configuration stereoisomers, and/or E-/Z- cis-trans isomers of each compound).

In another preferred embodiment, the pharmaceutically acceptable salts include salts formed by combining with inorganic acids, organic acids, alkali metal ions, alkaline earth metal ions or organic bases capable of providing physiologically acceptable cations and ammonium salts.

In another preferred embodiment, the inorganic acids are selected from hydrochloric acid, hydrobromic acid, phosphoric acid or sulfuric acid; the organic acids are selected from methanesulfonic acid, p-toluenesulfonic acid, trifluoroacetic acid, medlaric acid, maleic acid, tartaric acid, fumaric acid, citric acid or lactic acid; the alkali metal ions are selected from lithium ion, sodium ion, potassium ion; the alkaline earth metal ions are selected from calcium ion, magnesium ion; and the organic bases capable of providing physiologically acceptable cations are selected from methylamine, dimethylamine, trimethylamine, piperidine, morpholine or tris(2-hydroxyethyl)amine.

All of these salts within the scope of the present invention can be prepared using conventional methods. During the preparation of the compounds of general formula I, solvates and salts thereof, polycrystalline or co-crystal may occur under different crystallization conditions.

The starting materials and intermediates during the preparation method of the present invention are easily obtained, and each step of the reaction can be easily synthesized according to the reported literature or by conventional methods in organic synthesis for those skilled in the art. The compounds of formula I can exist in the form of solvates or nonsolvates, and different solvates may be obtained by crystallization from different solvents.

### Pharmaceutical composition and administration method

Since the compounds herein have excellent regulatory activity against PD-1/PD-L1 interaction, the compound of the present invention and various crystal forms thereof, pharmaceutically acceptable inorganic or organic salts, hydrates or solvates thereof, and pharmaceutical composition containing the compound according to the present invention as main active ingredient can be used to prevent and/or treat (stabilize, alleviate or cure) diseases associated with PD-1/PD-L1 interaction (eg, cancer, infectious disease, autoimmune disease).

The pharmaceutical composition of the invention comprises the compound of the present invention in a safe and effective dosage range and pharmaceutically acceptable excipients or carriers. Wherein the "safe and effective dosage" means that the amount of compound is sufficient to significantly ameliorate the condition without causing significant side effects. Generally, the pharmaceutical composition contains 1-2000 mg compounds of the invention per dose, preferably, 10-200mg compounds of the invention per dose. Preferably, the "dose" is a capsule or tablet.

"Pharmaceutically acceptable carrier" means one or more compatible solids or liquid fillers, or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc.

There is no special limitation of administration mode for the compound or pharmaceutical compositions of the present invention, and the representative administration mode includes (but is not limited to): oral administration, parenteral (intravenous, intramuscular or subcutaneous) administration.

Solid dosage forms for oral administration include capsules, tablets, pills, powders and granules. In these solid dosage forms, the active compounds are mixed with at least one conventional inert excipient (or carrier), such as sodium citrate or CaHPO4, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets and pills, the dosage forms may also contain buffering agents.

The solid dosage forms such as tablets, sugar pills, capsules, pills and granules can be prepared by using coating and shell materials, such as enteric coatings and any other materials known in the art. They can contain an opaque agent. The release of the active compounds or compounds in the compositions can be released in a delayed mode in a given portion of the digestive tract. Examples of the embedding components include polymers and waxes. If necessary, the active compounds and one or more above excipients can form microcapsules.

Liquid dosage forms for oral administration include pharmaceutically acceptable emulsions, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl formamide, as well as oil, in particular, cottonseed oil, peanut oil, corn germ oil, olive oil, castor oil and sesame oil, or the combination thereof.

Besides these inert diluents, the composition may also contain additives such as wetting agents, emulsifiers, and suspending agent, sweetener, flavoring agents and perfume.

In addition to the active compounds, the suspension may contain suspending agent, for example, ethoxylated isooctadecanol, polyoxyethylene sorbitol and sorbitan esters, microcrystalline cellulose, methanol aluminum and agar, or a combination thereof.

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Compounds of the present invention can be administrated alone, or in combination with any other pharmaceutically acceptable compounds (such as other anticaner agents).

In the case of co-administration, the pharmaceutical composition can also include one or more (2, 3, 4, or more) other pharmaceutically acceptable compounds. One or more (2, 3, 4, or more) other pharmaceutically acceptable compounds may be used simultaneously, separately or sequentially with the compound of the present invention for the prevention and/or treatment of PD-1/PD-L1 interation related diseases.

When the pharmaceutical compositions are used, a safe and effective amount of compound of the present invention is applied to a mammal (such as human) in need thereof, wherein the dose of administration is a pharmaceutically effective dose. For a person weighed 60 kg, the daily dose is usually 1-2000 mg, preferably 20-500mg. Of course, the particular dose should also depend on various factors, such as the route of administration, patient healthy status, which are well within the skills of an experienced physician.

### The main advantages of the present invention include:

(1) The compounds of the present invention have high regulatory activity on PD-1/PD-L1 interaction, strong binding ability to PD-L1 protein, and the ability to relieve the inhibition of IFNγ by PD-L1.
(2) The compounds of the present invention have better solubility; low toxicity to normal cells, so they can be administered to a subject in a larger dosage range.
(3) Compared with the compounds of the prior art, the compounds of the present invention have better solubility, so they have good druggability. Compared with the existing compounds, the compounds of the present invention show good bioavailability in *in vivo* experiments. In addition, compared with existing compounds, the compounds of the present invention can be easily made into pharmaceutically acceptable salts, thus facilitating further formulation.
*(4) In vivo* pharmacodynamic studies show that the compounds of the present invention can significantly inhibit the growth of subcutaneous tumors in terms of tumor volume and weight, and can significantly increase the number of lymphocytes in the blood and spleen of mice.

The present invention will be further illustrated below with reference to the specific examples. It should be understood that these examples are only to illustrate the invention but not to limit the scope of the invention. The experimental methods with no specific conditions described in the following examples are generally performed under the conventional conditions, or according to the manufacturer's instructions. Unless indicated otherwise, parts and percentage are calculated by weight.

The experimental materials and reagents used in the following examples can be commercially available unless otherwise specified.

### General Materials and Test Methods:

The instruments and raw materials involved in the examples are described as follows:
H NMR spectra were obtained by Bruker AV-400 (400MHz) NMR analysis.

Chemical shifts are reported with tetramethylsilane as an internal standard and are expressed in ppm (CDCl₃: δ 7.26 ppm). The recorded data information is as follows: chemical shifts and their splitting and coupling constants (s: singlet; d: doublet; t: triplet; q: quartet; br: broad; m: multiplet).

Mass spectral data were analyzed, among other things, using a LC/MS spectrometer (Finnigan LCQ Advantage), and all reactions were run under dry argon protected anhydrous and oxygen-free conditions. The solid metal organic compounds were stored in an argon-protected dry box.

Tetrahydrofuran and ether were obtained by distillation, in which sodium metal and benzophenone were added. Dichloromethane, pentane and hexane were treated with calcium hydride.

The special raw materials and intermediates involved in the present invention are customized and provided by Tianjin Changsen Pharmaceutical Co., Ltd., etc., and all other chemical reagents are purchased from reagent suppliers such as Shanghai Chemical Reagent Company, Aldrich, and Acros, etc.. If the intermediates or products required for the reaction in the synthesis process are not enough for the next step test, the synthesis is repeated several times to sufficient amount.

Unless otherwise specified, the raw materials and reagents involved in the present invention can be commercially available or can be purchased through customized processing.

The compounds of the present invention may contain one or more asymmetric centers, and thus the series of compounds may be in racemic or single enantiomeric form. The compounds prepared in the present invention are heterocyclic compounds with a purity of more than 95%, and the structural characterization of each final product is determined by MS or/and hydrogen spectral nuclear magnetic resonance (¹H NMR) analysis, respectively. The following examples illustrate the synthesis of various compounds and intermediates of the present invention.

### Example 1 Synthesis of compound 001

### Step 1-1:

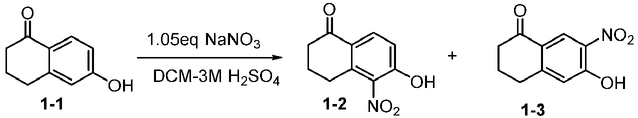

1.3 g of 1-1 was dissolved in 200 ML of DCM-3M H₂SO₄ (1:1). The reaction system was placed under ice bath, 0.71 g of sodium nitrate was added to the reaction system at 5 °C. Under nitrogen protection, the reaction was carried out overnight at room temperature. TLC spotting showed that 1-1 disappeared and LCMS analysis showed that the product was formed. The reaction mixture was purified by column chromatography to obtain 600 mg of product 1-2, MS-APCI: 208 [M+H]⁺ and 300 mg of 1-3, MS-APCI: 208 [M+H]⁺.

### Step 1-2:

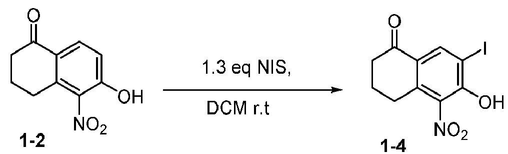

4.5 g of compound 1-2 was dissolved in 200 mL of DCM (in a 500 mL one-neck flask), into which 6.3 g of NIS was added, and argon was applied to protect. The reaction was carried out overnight at room temperature. TLC spotting showed that 1-2 was reacted completely. 100 mL of 2M HCl was added to the reaction solution. The resulting solution was separated and dried. The reaction solution was purified by spin-drying and subjected to column chromatography (Hep-DCM 1:1, then DCM) to obtain 6 g of product as a pale yellow solid. MS-APCI: 334 [M+H]⁺.

### Sts 1-3:

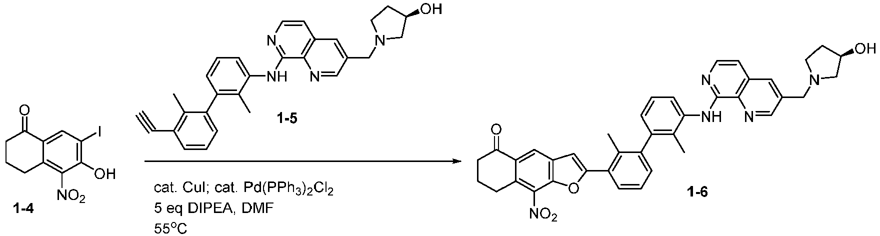

945 mg of compound 1-4, 636 mg of compound 1-5, 25 mg of CuI and 25 mg of Pd(PPh₃)Cl₂ were added to a 50 ml three-necked flask, under argon protection, 20 ml of DMF and 1.2 ml of DIPEA were added, and the reaction was carried out at 55°C for 3 hours. TLC spotting showed that raw material disappeared. The reaction solution was added to 300 ml of ice-water, then the resulting solution was extracted twice with 300 ml of ethyl acetate, washed once with 2M NaOH, dried and spin dried. The crude product was purified by column (DCM-MeOH 10:1) to obtain 220 mg of a pale yellow solid. MS-APCI: 654 [M+H]⁺.

### Step 1-4:

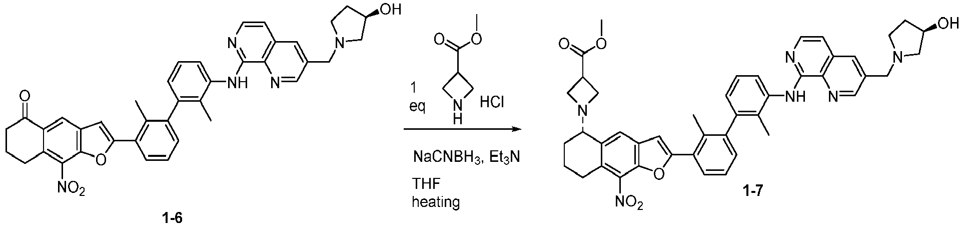

215 mg of compound 1-6, 248 mg of methyl azetidine-3-carboxylate hydrochloride, and 125 mg of Na(CN)BH3 were dissolved in 10 mL of THF, under argon protection, 167 mg of triethylamine was added, and the mixture was reacted at 70°C for 24 hours. The reaction solution was added to 100 ml of ice water, the resulting solution was extracted twice with 100 ml of EA and dried. The crude product was purified by column (DCM-MeOH 100, 50:1, 10:1, 5:1) to obtain 53 mg of 1-7 as a pale yellow solid, MS-APCI: 753 [M+H]⁺.

### Step 1-5:

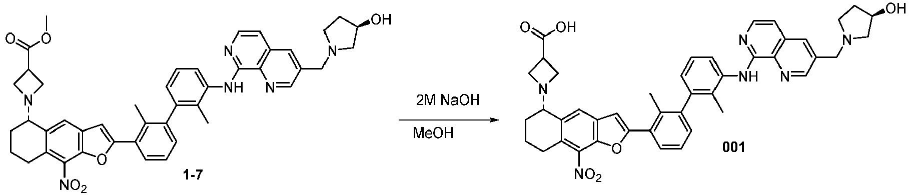

53 mg of raw material 1-7 was dissolved in 5 ml of MeOH, 2 ml of 2M NaOH was added to it, and the reaction was carried out at 26 °C for 5 hours. The reaction solution was spin-dried at 40 °C, 50 ml of water was added to the residual solid, and the pH value was adjusted to 7 with 2M HCl. The solid was filtered off, washed with water, washed with THF, dried, and further subjected to preparative purification to obtain 5 mg of a yellow solid. MS-APCI: 739 [M+H]⁺

¹H NMR (400 MHz, DMSO-*d*6) *δ* 9.34 (s, 1H), 9.00 (s, 1H), 8.46 (s, 1H), 8.34 (d, *J* = 8.0 Hz, 1H), 8.08 (d, J = 8.0 Hz, 1H), 7.77 (d, J = 8.0 Hz, 1H), 7.44 (t, J = 8.0 Hz, 1H), 7.35 - 7.25 (m, 3H), 7.19 (d, J = 8.0 Hz, 1H), 6.91 (d, J = 8.0 Hz, 1H), 6.52 (m, 1H), 4.75 (brs, 1H), 4.20 (brs, 1H), 3.84-3.74 (m, 2H), 3.37-2.83 (m, 3H), 2.66-2.28 (m, 2H), 2.21 (s, 3H), 2.18-2.06 (m, 1H), 2.06 (s, 3H), 1.96-1.58 (m, 5H), 1.43 (m, 1H).

### Step 1-6:

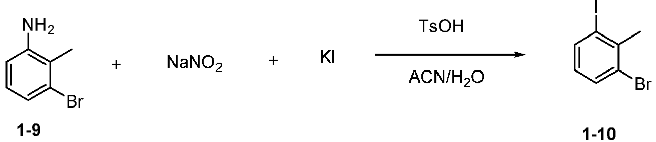

Amino raw material 1-9 (30 g) was dissolved in 400 ml of acetonitrile-water (1:1) under nitrogen protection and the system was clear. The reaction system was placed under ice bath, p-toluenesulfonic acid hydrate (901 g) was added at an internal temperature of 0-5 °C. The reaction mixture was stirred for 10 minutes, the system was pink and turbid. Aqueous NaNO₂ solution (22 g, 45 mL) was added dropwise at an internal temperature of 0 to 5 °C. The reaction was carried out for 30 minutes, and the system was clear; protected from light, potassium iodide aqueous solution (67 g, 60 mL) was added dropwise at 0 to 5 °C and naturally raised to room temperature for reaction. TLC tracking; post-treatment: saturated sodium sulfite was added under ice bath to quench, the mixture was extracted with HEP, washed once with saturated brine. The samples were mixed, passed through column: the product was eluted with HEP and concentrated to obtain 46.9 g of yellow-brown liquid, yield: 98.1%. MS-APCI: 296 [M+H]⁺.

### Step 1-7:

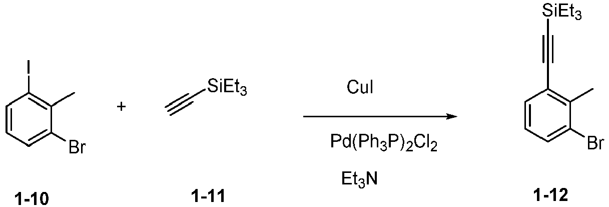

Iodine raw material 1-10 (1 g), CuI (0.03 g), Pd(Ph₃P)₂Cl₂ (0.12 g) were taken to add into a 50 ml reaction flask, the atomsphere was replaced with nitrogen for three times, anhydrous DMF (20 ml) was added, and the system was reddish brown and clear. Triethylamine (1.7 g) was added, the system was brownish yellow and clear; the atomsphere was replaced by nitrogen for 3 times, and the system was protected from light. Alkyne raw material 1-11 (0.52 g) was added, and the reaction was carried out at room temperature. TLC tracking; post-treatment: under ice bath, saturated ammonium chloride was added to quench, the mixture was extracted twice with MTBE, washed 5 times with saturated brine. The samples were mixed, passed through column: the product was eluted with HEP and concentrated to obtain 1.03 g of yellow-brown liquid, yield: 95%. MS-APCI: 309 [M+H]⁺.

### Step 1-8:

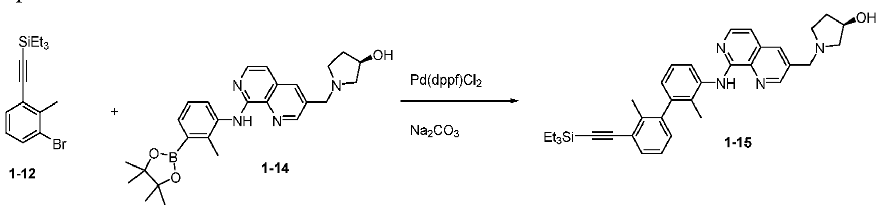

Bromine raw material **1-12** (1.1 g), borate raw material **1-14** (1 g, WO2018119286), Pd(dppf)Cl₂ (0.12 g) and sodium carbonate (0.62 g) were taken to add into a 50 ml reaction flask, the atomsphere was replaced with nitrogen for 3 times, 1,4-dioxane (8 ml) and 2 ml of water were added, and the mixture was reacted at 90 °C; TLC tracking; post-treatment: saturated ammonium chloride was added under ice bath to quench, the mixture was extracted twice with MTBE, washed 5 times with saturated brine. The samples were mixed, passed through column: (HEP-DCM 100, 50:1, 10:1, 5:1), eluted and concentrated to obtain 0.5 g of yellow solid, yield: 41%. MS-APCI: 563 [M+H]⁺

### Step 1-10:

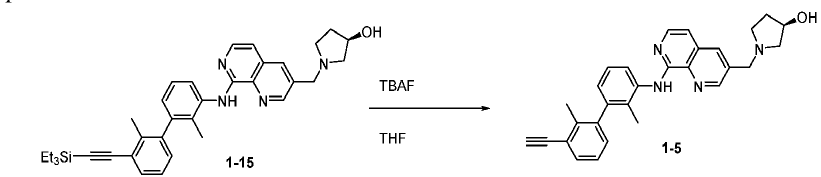

Alkyne raw material 1-15 (1.1 g) was added to THF (15 ml), the atomsphere was replaced with nitrogen for 3 times, 1 ml of 1M TBAF was added, and the reaction was carried out at room temperature. TLC tracking; post-treatment: THF was spin dried, 5 ml of water was added, the mixture was extracted twice with ethyl acetate, washed twice with saturated brine; the samples were mixed, passed through column: (DCM-MeOH 100, 50:1, 10:1, 5:1), eluted and concentrated to obtain 1.0 g of yellow solid, yield: 83 %. MS-APCI: 449 [M+H]⁺.

### Example 2 Synthesis of compound 002

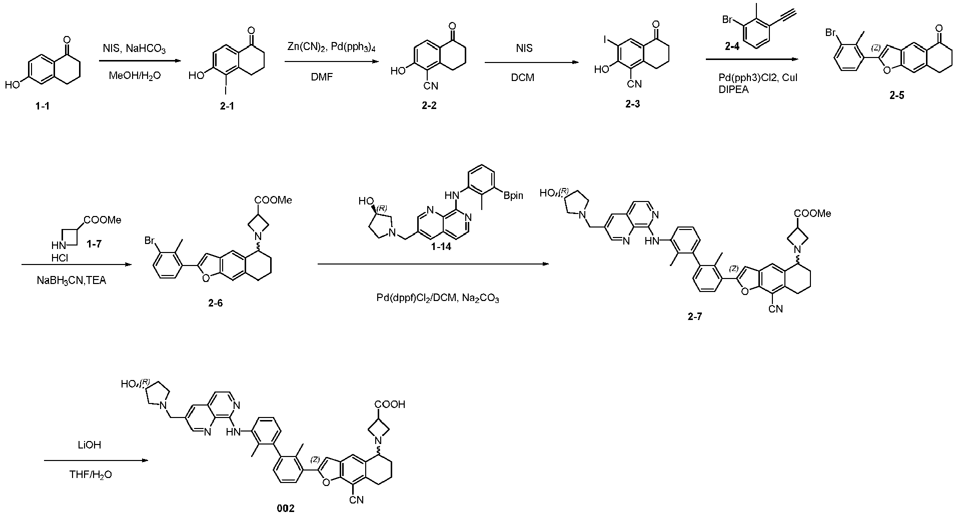

### Step 2-1:

**1-1** (1.1 g) and sodium bicarbonate (0.57 g) were added into a 100 mL three-neck flask, methanol (25 mL) and water (5 mL) were added to dissolve, and the resulting mixture was cooled to 5°C in an ice bath. NIS (1.52 g) was slowly added, and the temperature was naturally heated after all was added, and the reaction was carried out overnight. TLC showed that the raw materials were reacted completely. Methanol was spin dried, 1M hydrochloric acid was added to adjust pH to pH<3, and a large amount of white solid was precipitated. The mixture was filtrated, the filter cake solid was added to 0.5 M hydrochloric acid (100 mL) and the mixture was stirred for 30 minutes, filtered again, the obtained filter cake solid was dissolved with EA, and subjected to column chromatography to obtain a pale yellow/white solid product, 0.7 g. MS-APCI: 289 [M+H]⁺

### Step 2-2:

**2-1** (1.5 g) was dissolved in DMF (20 mL), Zn(CN)₂ (1.3 g), and Pd(PPh₃)₄ (1.2 g) were added to a 100 mL two-necked flask under the protection of N2, the reaction was carried out at 105 °C for 1 h. TLC showed that raw material was reacted completely. After the reaction mixture was cooled, the reaction solution was diluted with EA/HEP and filtered. The filtrate was washed with saturated NaCl and extracted with EA. After purified by column, 0.9 g of pure product was obtained as a yellow solid. MS-APCI: 188 [M+H]⁺

### Step 2-3:

In a 25 mL two-necked flask, **2-2** (1.5 g) was suspended in DCM (20 mL), NIS (2.0 g) was added at room temperature, and the reaction was carried out for 10 minutes. TLC showed that materials disappeared. The reaction solution was added with saturated NaHCO₃, the aqueous phase was adjusted to pH=2, extracted with DCM for 3 times. The DCM layer was washed with 10% Na₂S₂O₃, dried, and spin-dried to obtain 1.9 g of product as a brown solid. MS-APCI: 314 [M+H]⁺

### Step 2-4:

In a 15mL reaction tube, **2-3** (1.6 g), **2-4** (1.3g, WO2018195321), Pd(PPh₃)₂Cl₂ (350 mg), CuI (50 mg), DIPEA (3 g, 6.5 mmol) were dissolved in DMF (8 mL), under the protection of N2, and the reaction was carried out at 85°C for 2h. TLC showed that raw materials disappeared. After the reaction was completed, water/DCM were added to the reaction solution for extraction. The reaction solution was dried, spin dried. The residue was beaten to slurry with EA/HEP (20 mL, 1:1). The resulting solid was purified by column, eluted with pure DCM to give 800 mg of product as a yellow solid. MS-APCI: 355 [M+H]⁺

### Step 2-5:

In a 15mL reaction tube, **2-5** (150 mg), **1-7** (135 mg), NaBH₃CN (39 mg), TEA (83 mg) were added to THF (5 mL), and the reaction was carried out at 70 °C for 5h. The detection of TLC showed that 1-6 disappeared. The reaction solution was spin-dried, water and EA were added into the solution for extraction. EA was dried, spin dried, and the residue was purified by column to obtain pure product **1-8** (120 mg) as a yellow solid. MS-APCI: 454 [M+H]⁺

### Step 2-6:

Compound **2-6** (100 mg), **1-14** (100 mg, WO2018119286), Pd(dppf)Cl₂/DCM (16.7 mg), Na₂CO₃ (57.2 mg) were placed in a reaction flask, protected by N₂ and degassed. 3 mL dioxane/0.6 mL of water was injected into the reaction flask, and the reaction was carried out at 90 °C for 1 hour. The detection of TLC spotting showed that the reaction was completed. The reaction solution was added with 2 mL of water and extracted with EA. EA was dried, spin dried, and the residue was purified by column to obtain 80 mg of product as a yellow solid. MS-APCI: 733 [M+H]⁺

### Step 2-7:

**2-7** (80 mg) was dissolved in 2 mL of THF, 2 mL of water was added, LiOH (7.8 mg) was heated, and the mixture was reacted at room temperature for 1 h. The detection of TLC showed that the reaction was completed. The reaction solution was spin-dried at 40 °C, and 2M HCl was added to adjust the pH to 7. The solid was filtered out, washed with water, dried, and further subjected to preparative purification to obtain 50 mg of yellow solid. MS-APCI: 719 [M+H]⁺

1H NMR (400 MHz, DMSO-d6) δ 9.30 (s, 1H), 8.84 (d, J = 2.0 Hz, 1H), 8.43 (d, J = 8.1 Hz, 1H), 8.16 (d, J = 1.9 Hz, 1H), 8.04 (d, J = 5.8 Hz, 1H), 7.84 (s, 1H), 7.81 (dd, J = 7.9, 1.3 Hz, 1H), 7.46 (t, J = 7.7 Hz, 1H), 7.32 (t, J = 7.9 Hz, 1H), 7.29 (d, J = 1.3 Hz, 1H), 7.26 (dd, J = 7.6, 1.3 Hz, 1H), 7.16 (d, J = 5.8 Hz, 1H), 6.90 (d, J = 7.5 Hz, 1H), 4.69 (s, 1H), 4.20 (s, 1H), 3.88 - 3.71 (m, 2H), 3.50 - 3.39 (m, 11H), 3.19 - 2.79 (m, 3H), 2.76 - 2.58 (m, 2H), 2.40 - 2.28 (m, 1H), 2.22 (s, 3H), 2.08 (s, 3H), 2.00 (dq, J = 14.1, 7.4 Hz, 1H), 1.87 (d, J = 12.8 Hz, 1H), 1.71 (s, 1H), 1.57 (t, J = 13.0 Hz, 2H).

### Example 3 Synthesis of compound 003

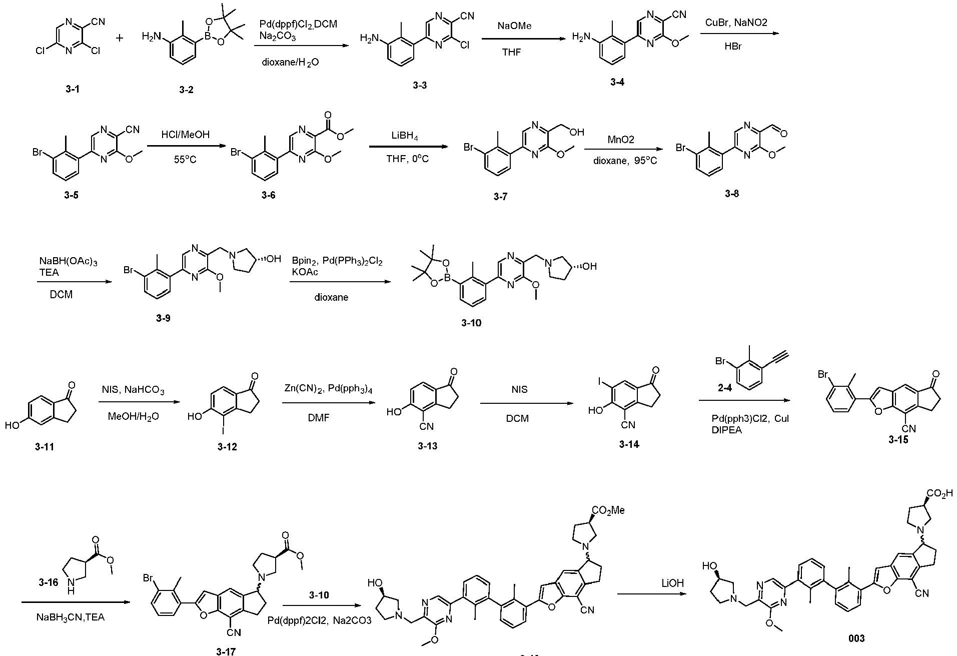

### Step 3-1:

Compounds **3-1** (40 g, 230.7 mmol), **3-2** (58.5 g, 230.7 mmol, WO2012031004), Pd(dppf)Cl₂/DCM (10.2 g, 0.05 eq), Na₂CO₃ (53 g, 2 eq) were sequentially added to dioxane/H₂O (300 mL, 5:1), the reaction system was degassed 3 times, protected by N2, and reacted at 80 °C for 1 hour. TLC showed that the reaction was complete. The reaction solution was filtered, and the filtrate was spin-dried. EA was added to dissolve, and 500 mL of saturated saline was added to EA. The layers were separated. The EA layer was dried and spin dried. The resulting solid was slurried with EA/HEP (200 mL, 1:3) and filtered to yield 40 g of a yellow solid.

### Step 3-2:

**3-3** (66 g, 249 mmol) was dissolved in 500 mL of THF, cooled to 0°C, and sodium methoxide solution (50 mL, 5N in MeOH) was added dropwise. After dropping, the reaction was carried out at 0 °C for 30 minutes. At 0°C, 4N HCl/MeOH was added dropwise to the reaction solution to pH=7. THF was spun away. The residue was dissolved in 1L of DCM, washed with 2L of water, and separated. The DCM layer was dried and spin dried. The resulting solid was slurried with EA/HEP (200 mL, 1:3) and filtered to obtain 60 g of a yellow solid.

### Step 3-3:

In a 3L of reaction flask, with mechanically stirring, CuBr (45.4 g, 316.5 mmol) was added to HBr (500 mL), cooled to 0 °C for further use. Then compound **3-4** (55 g, 211 mmol) was suspended in HBr (500 mL), cooled to 0 °C, and NaNO₂ aqueous solution (17.5 g, 253 mmol) (dissolved in 50 mL of water) was added dropwise. Brown smoke was produced. After the dropwise addition, the mixture was stirred for 30 minutes. The solution of compound 3-4 in HBr was poured into the solution of CuBr in HBr at one time, the reaction solution was black, and a lot of bubbles were generated. After stirring for 1 h, SM1 disappeared and the product spots formed by TLC monitoring. 2L of water was added to the reaction solution, and then 2L of EtOAc was added, followed by stirring, the solid was dissolved, and the layers were separated. The EA layer was dried, spin-dried, and purified by column (EA:DCM:HEP=1:1:4) to obtain 45 g of the product as a yellow solid.

### Step 3-4:

Compound **3-5** (15 g) was suspended in HCl/MeOH (250 mL, 4N) (stuffed reaction flask), and the mixture was heated and reacted at 60 °C overnight. The reaction solution became clear. The detection of TLC showed that 5 was reacted completely. After the reaction solution was cooled to room temperature, yellow solid was precipitated. The reaction solution was spin dried, saturated sodium bicarbonate (300 mL) was added to the remaining solid, and EA (200 mL x 3) was added for extraction. EA layer was dried and spin dried. The resulting solid was slurried with EA/HEP (240 mL, 1:5). The mixture was filtrated, and the filter cake solid was spin-dried to obtain 12 g of the product as a yellow solid.

### Step 3-5:

Compound **3-6** (31 g, 86.7 mmol) was dissolved in anhydrous THF (300 mL), cooled to 0 °C, LiBH4 (2.1 g, 95.4 mmol) was added in batches, and the reaction was carried out at 0 °C for 1 h. The completion of the reaction was detected by TLC. The reaction solution was filtered. The filter cake was rinsed with DCM. The filtrate was spin dried. 300 mL of water was added to the obtained solid, and the resulting solution was extracted with EA (200 mL×3). The EA layer was dried, spin dried, and the resulting solid was slurried with EA/HEP (240 mL, 1:5). The mixture was filtrated, and the filter cake solid was spin-dried to obtain 24 g of the product as a yellow solid.

### Step 3-6:

Compound **3-7** (32 g, 97.5 mmol) was dissolved in dioxane (500 mL), MnO₂ (51 g, 585.4 mmol) was added, degassed and protected by N2, and the reaction was carried out at 95 °C for 5 h. The completion of the reaction was detected by TLC. The reaction solution was filtered, and the filtrate was spin-dried. The resulting solid was slurried with EA/HEP (180 mL, 1:5). The mixture was filtrated, and the filter cake solid was spin-dried to obtain 24 g of the product as a yellow solid.

### Step 3-7:

Compound **3-8** (22 g, 67.5 mmol), R-3-hydroxypyrrolidine hydrochloride (16.6 g, 135 mmol), TEA (20.5 g, 202.5 mmol) were added to DCM (300 mL). After the reaction was carried out at room temperature for 1 hour, NaBH(OAc)3 (21.5 g, 101.25 mmol) was added and the reaction was carried out for another 1 hour. The completion of the reaction was detected by TLC. Post-treatment: water (300 mL) was added to the reaction solution, the layers were separated, and the DCM layer was washed with saturated brine. The layers was separated, DCM was dried, spin dried, and the residue was purified by column (DCM:MeOH=40:1) to obtain 15.3 g of product as a brown oil.

### Step 3-8:

Compound **3-9** (2.0 g, 5 mmol), Bpin2 (6.37 g, 25.1 mmol), Pd(PPh₃)₂Cl₂ (352 mg, 0.5 mmol), KOAc (984.6 mg, 10 mmol) were added to dioxane (40 mL), degassed 3 times and protected by N2, and the reaction was carried out at 90 °C overnight. After the reaction was completed, the reaction mixture was filtered. The filtrate was spin dried. 30 mL of DCM was added to dissolve and then 50 g of silica gel was added to mix the sample. The crude product was purified by silica gel column, elution gradient: EA=100% (500 mL), EA:MeOH=40:1 (2050 mL). The product was obtained as a brown oil, 900 mg.

### Step 3-9:

Raw material **3-11** (51 g, 0.34 mol) was dissolved in 500 ml of methanol, 300 ml of water was added, sodium bicarbonate (28.9 g, 0.34 mmol) was added, the solution of NIS in methanol was added at about 0°C, and the reaction was carried out overnight after complete addition. Post-treatment: the reaction solution was concentrated and dried, 500 ml of water was added, 3M hydrochloric acid was added to adjust pH to 3. the solid was filtered out , beaten to slurry with ethyl acetate/ethanol, filtered out and dried to obtain 146 g of gray solid.

### Step 3-10:

Raw material **3-12** (61 g, 230 mmol), ZnCN₂ (27 g, 230 mmol), Pd(PPh₃)₄(12.8 g, 11 mmol) were mixed in a 1000 mL there-necked flask, 1000 mL of DMF was added, and the atmosphere was replaced by N2 for 3 times, and the mixture was stirred at 90 °C for 3 h. A small amount of raw material remained by the detection of TLC spotting, and LCMS showed that the raw material was reacted completely.

### Step 3-11:

In a 25 mL two-necked flask, **3-13** (15 g) was suspended in DCM (20 mL), NIS (20 g) was added at room temperature. The reaction was carried out for 10 minutes, and TLC spotting showed that material disappeared. The reaction solution was added with saturated NaHCO₃, the aqueous phase was adjusted to pH=2, extracted with DCM for 3 times. DCM layer was washed with 10% Na₂S₂O₃, dried, and spin-dried to obtain 19 g of product as a brown solid.

### Step 3-12:

In a 15mL reaction tube, **3-14** (16 g), **2-4** (1.3 g, WO2018195321), Pd(PPh₃)₂Cl₂ (3.5 g), CuI (500 mg), DIPEA (30 g, 65 mmol) were dissolved in DMF (100 mL), protected by N2, and the reaction was carried out at 85°C for 2h. TLC spotting showed that the starting materials disappeared. After the reaction was completed, water/DCM was added to the reaction solution for extraction. The mixture was dried and spin-dried. The residue was slurried with EA/HEP (20 mL, 1:1). The resulting solid was purified by column and eluted with pure DCM to obtain 8 g of a yellow solid.

### Step 3-13:

In a 15mL reaction tube, **3-15** (150 mg), **3-16** (135 mg), NaBH₃CN (39 mg), TEA (83 mg) were added to THF (5 mL), and the reaction was carried out at 70 °C for 5h. 1-6 disappeared by the detection of TLC. The reaction solution was spin-dried, and water was added for EA extraction. EA was dried, spin-dried, and the residue was purified by column to obtain 100 mg of a yellow solid.

### Step 3-14:

120 mgof the product was obtained as a yellow solid according to the synthetic method of step 2-6 using compound **3-17** (200 mg) and **3-10** (180 mg) as raw materials.

### Step 3-15:

40 mg of the product was obtained as a yellow solid according to the synthesis method of steps 2-7 using compound **3-18** (120 mg) as raw material. MS (APCI): 684. ¹H NMR (400 MHz, DMSO-d6) δ 8.32 (s, 1H), 7.87 (d, J = 3.9 Hz, 1H), 7.80 (d, J = 8.0 Hz, 1H), 7.50 (d, J = 7.6 Hz, 1H), 7.45 (t, J = 7.6 Hz, 1H), 7.40 (t, J = 7.7 Hz, 1H), 7.31 (s, 1H), 7.27 (d, J = 7.6 Hz, 1H), 7.24 (d, J = 7.4 Hz, 1H), 5.29 (t, J = 5.0 Hz, 1H), 4.65 (d, J = 4.6 Hz, 1H), 4.29 (d, J = 7.0 Hz, 1H), 4.15 (s, 1H), 3.99 (q, J = 7.1 Hz, 2H), 3.92 (s, 3H), 3.71 (d, J = 4.3 Hz, 2H), 2.79 (t, J = 6.8 Hz, 1H), 2.68 - 2.60 (m, 4H), 2.40 (dd, J = 9.7, 4.0 Hz, 1H), 2.30 (q, J = 1.9 Hz, 2H), 2.21 (s, 3H), 2.04 (s, 3H), 1.96 (d, J = 7.0 Hz, 2H).

### Example 4 Synthesis of compound 004

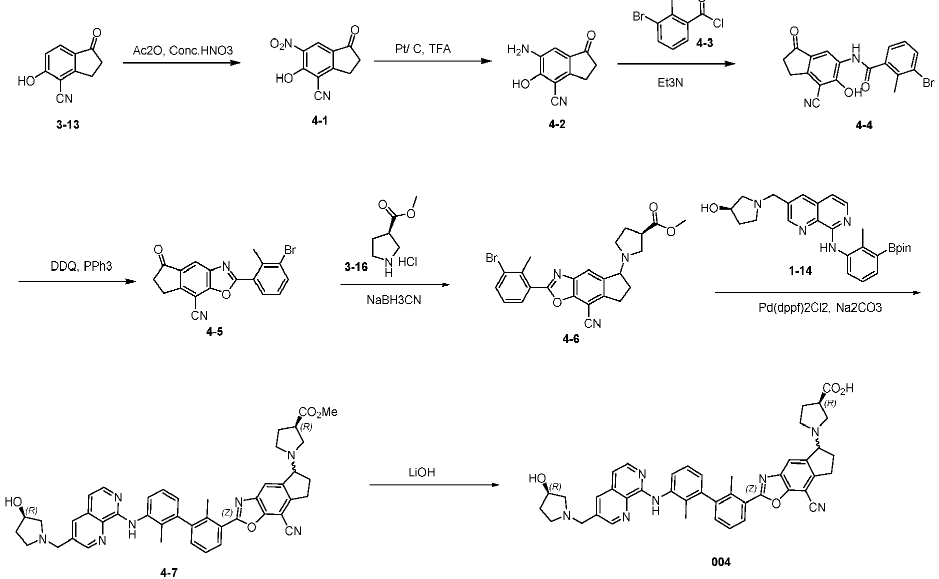

### Step 4-1:

Acetic anhydride (75 ml) and concentrated nitric acid (5 ml) were added to a 250 ml single-necked flask, raw material **3-13** (5.0 g, 28.9 mmol) was added in batches, and the mixture was stirred in a water bath. Precipitate formed after 20 mins. The reaction was monitored by TLC. After the reaction was completed, the precipitate was filtered out, drained, slurried, and pulled to dryness to obtain 4.5 g of white solid.

### Step 4-2:

THF (200 ml), raw material **4-1** (5.0 g, 23 mmol) and TFA (0.2 mL), Pt/C were added to a 500 ml single-necked flask, the atmosphere was replaced by H2 for 3 times, the mixture was stirred at room temperature, reacted for 1 hour. The reaction was monitored by TLC. After the raw materials was reacted completly, Pt/C was filtered out, and the filtrate was directly used in the next step.

### Step 4-3:

To the solution of the above filtrate **4-2** (10.0 g, 45.8 mmol) in THF was added 180 mmol Et₃N. **3-6** (10.74 g, 46 mmol, Organic Letters, 2019, 21, 5971-5976*)* was dissolved in 15 ml of DCM, and slowly added to the above solution. The reaction was carried out at room temperature for 1 hour, a lot of turbidity appeared, the solid was filtered out, and beaten to slurry and purified with ethyl acetate to obtain 18 g of off-white solid.

### Step 4-4:

PPh₃ (9.7 g, 37 mmol) was dissolved in toluene at room temperature, DDQ (8.4 g, 37 mmol) was slowly added and the mixture was stirred well, the system was suspended at this time. Raw material **4-4** (7.1 g, 18.5 mmol) was added, and the mixture was reacted at 110° C for 1 hour. The product accounted for the majority as the detection of TLC spotting. The supernatant was poured out, concentrated to dryness, and slurried with EA to obtain a white solid 3.7g.

### Step 4-5:

0.8 g of white solid was obtained according to the synthesis method of step 3-13 using compound **4-5** (1 g, 2.72 mmol) and **3-16** (902 mg, 5.45 mmol) as raw materials.

### Step 4-6:

180 mg of white solid was obtained according to the synthesis method of step 2-6 using compound **4-6** (200 mg, 0.416 mmol) and **1-14** (230 mg, 0.5 mmol, WO2018119286) as raw materials.

### Step 4-7:

38 mg of yellow solid was obtained according to the synthesis method of step 2-7 using compound **4-7** (120 mg) as a raw material. MS (APCI): 720. ¹H NMR (400 MHz, DMSO-d6) δ 9.30 (s, 1H), 8.83 (d, J = 2.0 Hz, 1H), 8.45 (d, J = 8.2 Hz, 1H), 8.15 (d, J = 1.9 Hz, 1H), 8.12 (d, J = 7.9 Hz, 1H), 8.04 (d, J = 5.8 Hz, 1H), 7.98 (d, J = 9.9 Hz, 1H), 7.52 (t, J = 7.7 Hz, 1H), 7.41 (d, J = 7.5 Hz, 1H), 7.33 (t, J = 7.9 Hz, 1H), 7.15 (d, J = 5.8 Hz, 1H), 6.89 (d, J = 7.5 Hz, 1H), 4.38 - 4.26 (m, 1H), 4.24 - 4.14 (m, 1H), 3.78 (q, J = 13.7 Hz, 2H), 3.24 - 3.15 (m, 2H), 3.08 - 2.99 (m, 2H), 2.93 - 2.76 (m, 2H), 2.76 - 2.64 (m, 2H), 2.63 - 2.54 (m, 3H), 2.44 (s, 3H), 2.35 (dd, J = 9.7, 3.6 Hz, 1H), 2.25 - 2.16 (m, 2H),2.10 - 2.05 (m, 3H), 2.00 (dt, J = 13.5, 7.3 Hz, 1H), 1.95 - 1.87 (m, 2H), 1.59 - 1.50 (m, 1H).

### Example 5 Synthesis of compound 005

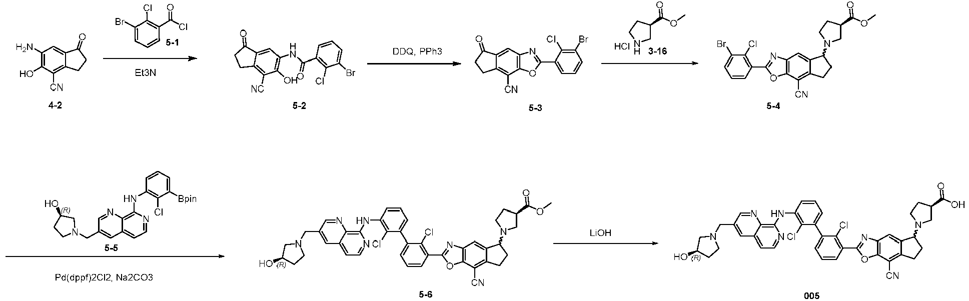

### Step 5-1:

15 g of off-white solid was obtained according to the synthesis method of step 4-3 using compound **4-2** (10 g, 45.8 mmol) and **5-1** (11.6 g, 45.8 mmol, WO2017059135) as raw materials.

### Step 5-2:

1.1 g of off-white solid was obtained according to the synthesis method of step 4-4 using compound **5-2** (2 g) as a raw material. MS (APCI): 387 [M+H]⁺

### Step 5-3:

0.9 g of off-white solid was obtained according to the synthesis method of step 3-13 using **5-3** (1 g, 2.58 mmol) and compound **3-16** (0.85 g, 5.16 mmol) as raw materials. MS (APCI): 500 [M+H]⁺

### Step 5-4:

210 mg of white solid was obtained according to the synthesis method of step 2-6 using compounds **5-4** (200 mg) and **5-5** (200 mg, WO2018119286) as raw materials.

### Step 5-5:

51 mg of yellow solid was obtained according to the synthesis method of step 2-7 using compound **5-6** (120 mg) as a raw material. MS (APCI): 760. ¹H NMR (400 MHz, DMSO-*d*₆) δ 9.89 (s, 1H), 9.15 (d, *J* = 8.4 Hz, 1H), 8.91 (s, 1H), 8.28 - 8.15 (m, 2H), 8.07 (d, *J* = 11.0 Hz, 1H), 7.71 (d, J = 7.2 Hz, 2H), 7.60 - 7.49 (m, 2H), 7.34 (d, J = 5.8 Hz, 1H), 7.09 (d, J = 7.5 Hz, 1H), 5.31 (s, 1H), 4.73 (s, 2H), 4.35 (dt, *J* = 20.7, 5.9 Hz, 2H), 4.20 (s, 1H), 3.93 (s, 1H), 3.89 - 3.72 (m, 2H), 2.87 (dd, J = 17.0, 9.6 Hz, 2H), 2.75 - 2.55 (m, 2H) , 2.40 - 2.29 (m, 2H), 2.16 - 1.85 (m, 4H), 1.56 (s, 2H), 1.22 (s, 4H), 0.84 (t, J = 6.8 Hz, 1H).

### Example 6 Synthesis of compound 006

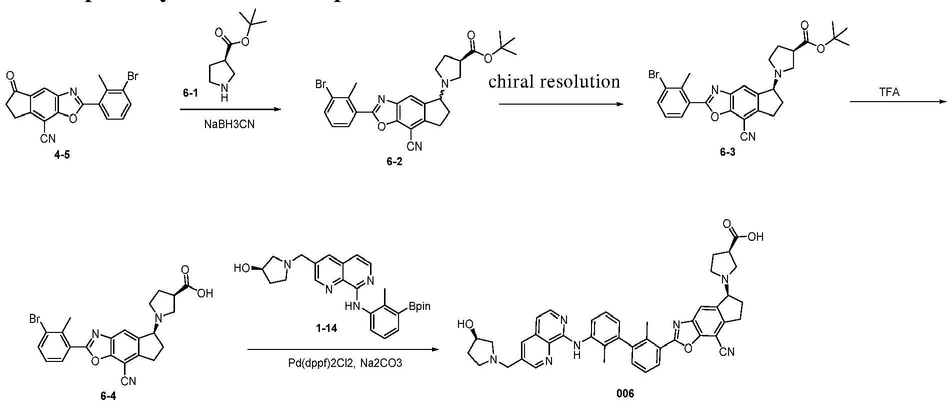

### Step 6-1:

8 g of white solid was obtained according to the synthesis method of step 3-13 using compounds **4-5** (10 g, 0.0272 mol) and **6-1** (9.3 g, 0.0544 mol, WO2018136935) as raw materials.

### Step 6-2:

Compound **6-2** (8 g) was subjected to chiral column resolution to obtain compound **6-3** (3.5 g).

### Step 6-3:

Compound **6-3** (3.5 g) was dissolved in TFA (10 mL), and then stirred at room temperature for 1 hour. TFA was spin-dried and azeotroped once with toluene. Saturated sodium bicarbonate solution and concentrated hydrochloric acid were added to the residual liquid, and the pH was adjusted to 2-3. A large amount of solid appeared, filtered and dried to obtain 2 g of light brown solid.

### Step 6-3:

32 mg of white solid was obtained according to the synthesis method of step 2-6 using compounds **6-4** (200 mg, 0.416 mmol) and **1-14** (230 mg, 0.5 mmol, WO2018119286) as raw materials. MS (APCI): 720. ¹H NMR (400 MHz, DMSO-d6) δ 9.32 (s, 1H), 8.86 (s, 1H), 8.47 (d, J = 8.2 Hz, 1H), 8.18 (s, 1H), 8.15 (d, J = 7.8 Hz, 1H), 8.06 (d, J = 5.8 Hz, 1H), 8.02 (s, 1H), 7.55 (s, 1H), 7.43 (d, J = 7.6 Hz, 1H), 7.35 (s, 1H), 7.18 (d, J = 5.8 Hz, 1H), 6.92 (d, J = 7.5 Hz, 1H), 4.32 (d, J = 5.8 Hz, 1H), 4.22 (tt, J = 6.9, 3.4 Hz, 1H), 3.81 (q, J = 13.7 Hz, 3H), 3.07 (q, J = 9.3, 8.0 Hz, 3H), 2.89 (s, 2H), 2.83 (d, J = 8.8 Hz, 2H), 2.74 (dd, J = 10.0, 6.3 Hz, 2H), 2.63 (dq, J = 15.1, 7.5 Hz, 3H), 2.46 (s, 3H), 2.37 (dd, J = 9.7, 3.6 Hz, 2H), 2.24 (q, J = 6.8 Hz, 2H), 2.09 (s, 3H), 2.01 (dt, J = 13.7, 6.8 Hz, 2H), 1.92 (d, J = 7.8 Hz, 2H), 1.57 (d, J = 3.7 Hz, 2H)

### Example 7 Synthesis of compound 007

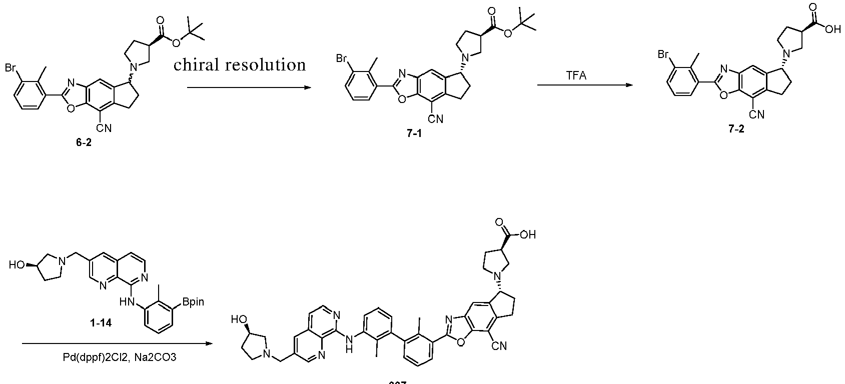

### Step 6-2:

Compound **6-2** (8 g) was subjected to chiral column resolution to obtain compound **7-1** (4 g).

### Step 6-3:

Compound **7-1** (4 g) was dissolved in TFA (10 mL) and then stirred at room temperature for 1 hour. TFA was suspended to dryness and azeotroped once with toluene. Saturated sodium bicarbonate solution and concentrated hydrochloric acid were added to the residual liquid, and the pH was adjusted to 2-3. A large amount of solid appeared, filtered and dried to obtain 3.2 g of light brown solid.

### Step 6-3:

30 mg of white solid was obtained according to the synthesis method of step 2-6 using compounds **7-2** (200 mg, 0.416 mmol) and **1-14** (230 mg, 0.5 mmol, WO2018119286) as raw materials. MS (APCI): 720. 1H NMR (400 MHz, DMSO-d6) δ 9.32 (s, 1H), 8.86 (d, J = 2.0 Hz, 1H), 8.46 (d, J = 8.1 Hz, 1H), 8.18 (s, 1H), 8.15 (d, J = 7.9 Hz, 1H), 8.06 (d, J = 5.8 Hz, 1H), 8.01 (s, 1H), 7.55 (t, J = 7.7 Hz, 1H), 7.43 (d, J = 7.4 Hz, 1H), 7.35 (t, J = 7.9 Hz, 1H), 7.18 (d, J = 5.8 Hz, 1H), 6.92 (d, J = 7.5 Hz, 1H), 4.36 (t, J = 6.0 Hz, 1H), 4.21 (dt, J = 6.9, 3.4 Hz, 1H), 3.81 (q, J = 13.8 Hz, 2H), 3.12 - 3.01 (m, 3H), 2.89 (p, J = 7.5 Hz, 2H), 2.79 (s, 1H), 2.75 (d, J = 6.1 Hz, 1H), 2.72 (dd, J = 6.3, 3.4 Hz, 1H), 2.62 (dt, J = 12.3, 7.5 Hz, 4H), 2.46 (s, 3H), 2.37 (dd, J = 9.7, 3.6 Hz, 1H), 2.22 (t, J = 7.2 Hz, 2H), 2.08 (s, 2H), 2.00 (dd, J = 11.0, 4.9 Hz, 2H), 1.94 (dd, J = 8.9, 6.0 Hz, 2H), 1.57 (qd, J = 8.2, 3.8 Hz, 2H).

### Example 8 Synthesis of compound 008

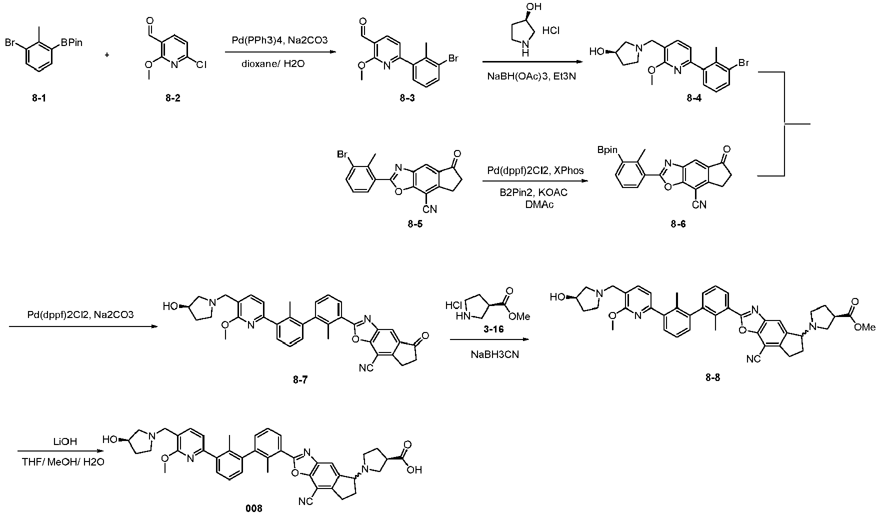

### Step 8-1:

Compound **8-1** (1.9 g, 6.41 mmol, WO2016207226), **8-2** (1.1 g, 6.41 mmol, WO2018006795), sodium carbonate (1.36 g, 12.82 mmol) and tetrakistriphenylphosphine palladium (222 mg, 0.19 mmol) were successively added to a single-necked flask containing dioxane/H₂O (4:1, 25 mL), protected by nitrogen, the mixture was stirred for 3 hours in an oil bath at 80 °C. The reaction was detected by LC-MS. After about 10% of the raw material remained, the reaction mixture was cooled, filtered, spin-dried, extracted with DCM/H₂O, dried over anhydrous sodium sulfate, and subjected to column chromatography (pure PE passed through the column) to obtain 650 mg of pale yellow solid. ESI (APCI): 306 [M+H]⁺.

### Step 8-2:

Compound **8-3** (200 mg, 0.653 mmol), R-3-hydroxypyrrolidine hydrochloride (121 mg, 0.98 mmol) and triethylamine (0.09 mL, 0.653 mmol) were successively added to a single-necked flask containing DCM (10 mL). After the solution was stirred at room temperature for 2 hours, NaBH(OAc)3 (415 mg, 1.96 mmol) was added to the reaction solution, the resulting solution was stirred at room temperature overnight, and the reaction was detected by TLC. After the reaction was completed, the reaction mixture was quenched by adding water, washed with brine, dried over anhydrous sodium sulfate, and subjected to column chromatography to obtain 200 mg of oil. ESI (APCI): 377 [M+H]⁺.

### Step 8-3:

Compound **8-5** (1 g, 2.72 mmol), B2Pin2 (1.04 g, 4.08 mmol), KOAc (668 mg, 6.81 mmol), X-phos (258 mg, 0.544 mmol) and Pd(dppf)₂Cl₂ (221 mg , 0.272 mmol) were successively added to a single-necked flask containing DMAc (20 mL), protected by nitrogen, the mixture was stirred for 3.5 hours in an oil bath at 90 °C, and the reaction was detected by LC-MS. After the reaction was completed, the solvent was spin-dried, the reaction solution was extracted with DCM/H₂O, dried over anhydrous sodium sulfate, and subjected to column chromatography to obtain 1 g of a yellow solid. ESI (APCI): 415 [M+H]⁺.

### Step 8-4:

Compounds **8-4** (200 mg, 0.53 mmol), **8-6** (240 mg, 0.58 mmol), sodium carbonate (168 mg, 1.59 mmol) and Pd(dppf)₂Cl₂ (44 mg) were successively added to a single-necked flask containing dioxane/H₂O (4:1, 5 mL), the mixture was stirred in an oil bath at 100 °C for 3 hours, and the reaction was monitored by LC-MS. After the reaction was completed, the mixture was filtered and spin-dried to remove the solvent. Tthe reaction solution was extracted with DCM/H₂O, dried over anhydrous sodium sulfate, and subjected to column chromatography to obtain 120 mg of oil. ESI (APCI): 585 [M+H]⁺.

### Step 8-5:

Compounds **8-7** (120 mg, 0.205 mmol), **3-16** (51 mg, 0.308 mmol), triethylamine (0.03 mL, 0.205 mmol) and NaBH₃CN (126 mg, 0.616 mmol) were successively added to a single-necked flask containing THF (5 mL). The mixture was stirred at 70°C in an oil bath overnight, and the reaction was monitored by LC-MS. After the reaction was completed, water was added to quench, the solvent was spin-dried, the residue was extracted with DCM/H₂O, dried over anhydrous sodium sulfate, and subjected to column chromatography to obtain70 mg of oil. ESI (APCI): 698 [M+H]⁺.

### Step 8-6:

Compound **8-8** (70 mg, 0.1 mmol) and LiOH (5 mg, 0.2 mmol) were successively added to a single-necked flask containing THF/MeOH/H₂O (2:1:1, 4 mL), and the mixture was stirred at room temperature for 1 hour. The reaction was monitored by TLC. After the reaction was completed, TFA was added to adjust the pH to neutrality, and 10 mg of white solid was prepared by reversed-phase column. ESI (APCI): 684 [M+H]⁺. ¹H NMR (400 MHz, DMSO-*d6)* δ 8.13 (d, *J* = 7.9 Hz, 1H), 7.99 (d, *J* = 9.6 Hz, 1H), 7.74 (d, *J* = 7.5 Hz, 1H), 7.52 (t, *J* = 7.7 Hz, 1H), 7.48 - 7.40 (m, 2H), 7.36 (t, J = 7.5 Hz, 1H), 7.18 (d, J = 7.7 Hz, 1H), 7.14 (d, J = 7.4 Hz, 1H), 4.68 (br, 1H), 4.40 - 4.26 (m, 1H), 4.22 - 4.15 (m, 1H), 3.86 (s, 3H), 3.67 - 3.48 (m, 2H), 3.28 - 3.14 (m, 2H), 3.09 - 2.98 (m, 1H), 2.93 - 2.74 (m, 4H), 2.74 - 2.51 (m, 3H), 2.44 (s, 3H), 2.44 - 2.34 (m, 3H), 2.25 - 2.17 (m, 2H), 2.02 (s, 3H), 2.00 - 1.85 (m, 3H), 1.58 - 1.51 (m, 1H).

### Example 9 Synthesis of compound 009

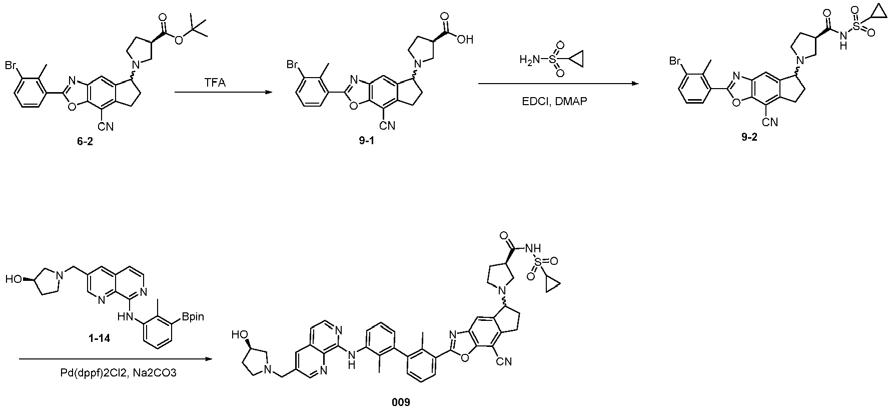

### Step 9-1:

Compound **6-2** (1 g) was dissolved in TFA (5 mL) and then stirred at room temperature for 1 hour. TFA was spin-dired and azeotroped once with toluene. Saturated sodium bicarbonate solution and concentrated hydrochloric acid were added to the residual liquid, and the pH was adjusted to 2-3. A large amount of solid appeared, filtered and dried to obtain 0.5 g of a light brown solid.

### Step 9-2:

Compound **9-1** (0.5 g, 1.07 mmol), cyclopropylsulfonamide (260 mg, 2.14 mmol), DMAP (261 mg, 2.14 mmol) and EDCI (410 mg, 2.14 mmol) were added in one portion to a single-necked flask containing DCM (15 mL), the mixture was stirred at room temperature overnight, and the reaction was detected by TLC. After the reaction was completed, the reaction mixture was washed with saturated brine and 0.5 M dilute hydrochloric acid aqueous solution, then the organic layer was dried over anhydrous sodium sulfate, and subjected to column chromatography to obtain 0.3 g of a pale yellow solid.

### Step 9-3:

60 mg of pale yellow solid was obtained according to the synthesis method of step 8-4 using compounds **9-2** (200 mg) and **1-14** (240 mg) as raw materials. ESI (APCI): 823 [M+H]⁺. ¹H NMR (400 MHz, DMSO-*d*₆) δ 9.30 (s, 1H), 8.86 (d, J = 2.0 Hz, 1H), 8.42 (d, J = 8.1 Hz, 1H), 8.20 (d, J = 1.9 Hz, 1H), 8.15 - 8.09 (m, 1H), 8.04 (t, J = 6.0 Hz, 2H), 7.53 (t, *J* = 7.7 Hz, 1H), 7.41 (d, J = 7.5 Hz, 1H), 7.33 (t, J = 7.9 Hz, 1H), 7.16 (d, J = 5.8 Hz, 1H), 6.91 (d, J = 7.5 Hz, 1H), 4.42 (d, J = 6.3 Hz, 1H), 4.23 (tt, J = 6.5, 3.1 Hz, 1H), 4.01 - 3.84 (m, 2H), 3.30 - 3.17 (m, 3H), 3.06 (dt, J = 16.6, 6.8 Hz, 2H), 2.98 - 2.75 (m, 5H), 2.74 - 2.54 (m, 4H), 2.44 (s, 3H), 2.31 - 2.19 (m, 3H),2.07 (s, 3H), 2.05 - 1.85 (m, 4H), 1.64 - 1.55 (m, 1H).

### Example 10 Synthesis of compound 010 (222)

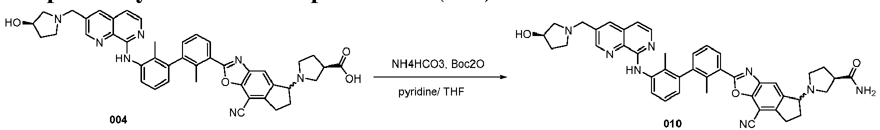

### Step 10-1:

**004** (30 mg, 0.0471 mmol), NH4HCO3 (7 mg, 0.0834 mmol) and Boc2O (18 mg, 0.0834 mmol) were successively added to a single-neck flask containing THF/pyridine (6:1, 3.5 mL), and the mixture was stirred at room temperature for 1 hour. The reaction was detected by LC-MS. After the reaction was completed, TFA was added to adjust the pH to 7-8, and then 10 mg of pale yellow solid was obtained by preparative reversed-phase column. ESI (APCI): 719 [M+H]⁺. ¹H NMR (400 MHz, DMSO-*d*₆) δ 9.35 (s, 1H), 8.97 (s, 1H), 8.39 (s, 1H), 8.32 (d, *J* = 8.2 Hz, 1H), 8.16 (d, J = 7.9 Hz, 1H), 8.08 (d, *J* = 5.7 Hz, 1H), 7.58 - 7.47 (m, 2H), 7.43 (d, *J* = 7.5 Hz, 1H), 7.35 (t, *J* = 7.8 Hz, 1H), 7.17 (d, *J* = 5.8 Hz, 1H), 6.94 (d, *J* = 7.5 Hz, 1H), 5.40 - 5.26 (m, 1H), 4.57 - 4.32 (m, 2H), 3.20 - 2.89 (m, 7H), 2.46 (s, 3H), 2.31 - 2.21 (m, 1H), 2.19 - 2.08 (m, 2H), 2.06 (s, 3H), 2.01 - 1.89 (m, 2H), 1.85 - 1.75 (m, 1H).

### Example 11 Synthesis of compound 011

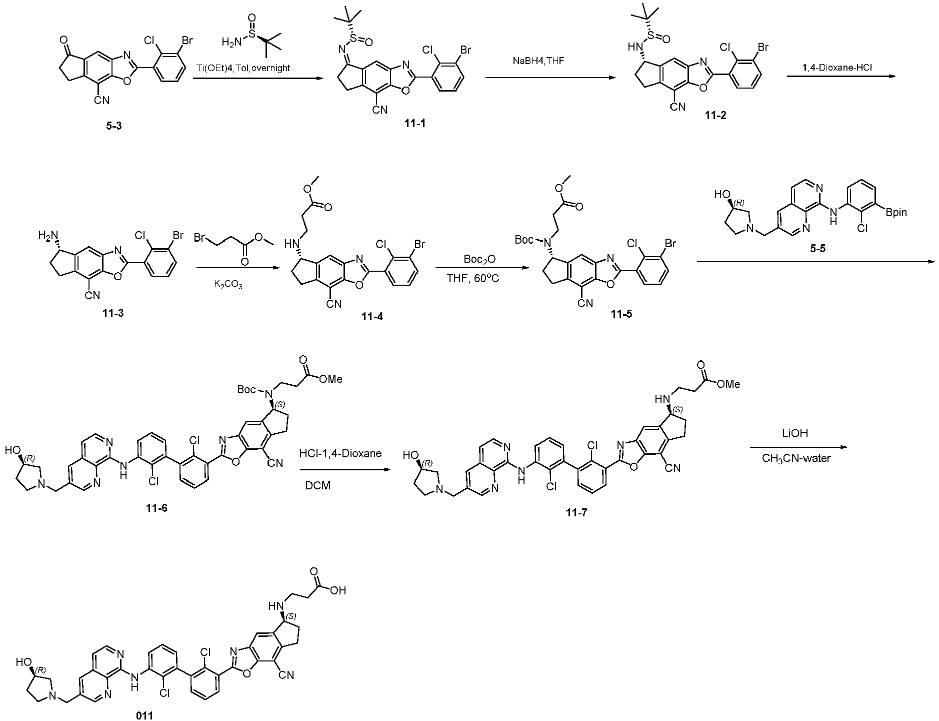

### Step 11-1:

Compound **5-3** (5 g, 12.9 mmol), S-tert-butylsulfinamide (4.69 g, 38.7 mmol) and ethyl tetratitanate (11.77 g, 51.60 mmol) were successively added to toluene (50 mL), protected by nitrogen, the mixture was heated to 70°C and stirred overnight, and the reaction was detected by TLC. After the reaction was completed, the reaction solution was cooled to room temperature and used directly in the next step.

### Step 11-2:

The above reaction solution was cooled to -70 °C, then THF (20 mL) and NaBH₄ (3.9 g, 103.2 mmol) were added thereto, and the mixture was kept stirring for 1 hour. Then it was slowly raised to room temperature and stirred for 1 hour, and the reaction was detected by TLC. After the reaction was completed, saturated aqueous ammonium chloride solution was added thereto. Then the reaction solution was extracted with ethyl acetate, dried over anhydrous sodium sulfate, and subjected to column chromatography to obtain 5 g of black-gray solid.

### Step 11-3:

Compound **11-2** (1 g, 2.03 mmol) was dissolved in DCM (5 mL) and the solution was stirred under an ice bath. Then, a dioxane solution of hydrogen chloride (3M, 1 mL) was added to the solution, the mixture was heated to room temperature and stirred for 1 hour, and the reaction was detected by TLC. After the reaction was completed, the mixture was filtered, the solid was rinsed with ethyl acetate and dried to obtain 788 mg of black-gray solid.

### Step 11-4:

Compound **11-3** (300 mg, 0.77 mmol), potassium carbonate (213 mg) were added to DMF (5 mL), then methyl 3-bromopropionate (129 mg) was added to the reaction solution, and then methyl 3-bromopropionate (129 mg) was added every 1 hour, three times in total. The reaction was detected by LC-MS until the product no longer increased. The reaction solution was extracted with DCM and water, dried over anhydrous sodium sulfate, and subjected to column chromatography to obtain 100 mg of a mixture of product and starting material.

### Step 11-5:

The above mixture was dissolved in THF (30 mL), and Boc₂O (413 mg) was added. Under nitrogen protection, the mixture was heated to 60 °C with stirring. The reaction was detected by TLC, and product of the previous step and the Boc on the raw material could be separated on TLC. Then the mixture was extracted with DCM/H₂O and dried over anhydrous sodium sulfate to obtain 20 mg of the target product as a gray solid.

### Step 11-6:

20 mg of pale yellow solid was obtained according to the synthesis method of step 8-4 using compounds **11-5** (20 mg, 0.035 mmol) and **5-5** (15 mg) as raw materials.

### Step 11-7:

To a 15 mL Schlenk tube, **11-6** (93 mg), DCM (2 mL) were added, HCl-dioxane (0.4 mL) was added dropwise, under N2 protection at room temperature, and the reaction mixture was stirred (yellow turbidity). The reaction was completed after 1 h by LC-MS. The mixtre was dried by oil pump to obtian 83 mg of a yellow solid.

### Steps 11-8:

34 mg of pale yellow solid was obtained according to the synthesis method of step 8-6 using compound **11-7** (83 mg) as a raw material. ESI (APCI): 734 [M+H]⁺. ¹H NMR (400 MHz, DMSO-d6) δ 9.88 (s, 1H), 9.12 - 9.00 (m, 2H), 8.53 (s, 1H), 8.39 (s, 1H), 8.31 - 8.19 (m, 2H), 7.73 (q, J = 7.0, 6.4 Hz, 2H), 7.56 (t, J = 8.0 Hz, 1H), 7.38 (d, J = 5.8 Hz, 1H), 7.11 (d, J = 7.7 Hz, 1H), 5.00 (s, 1H), 4.61 (s, 1H), 4.43 (d, J = 27.4 Hz, 1H), 3.30 - 3.14 (m, 2H), 2.71 - 2.58 (m, 3H), 2.39 (dd, J = 9.4, 4.3 Hz, 0H), 2.07 - 1.75 (m, 2H).

### Example 12 Synthesis of compound 012

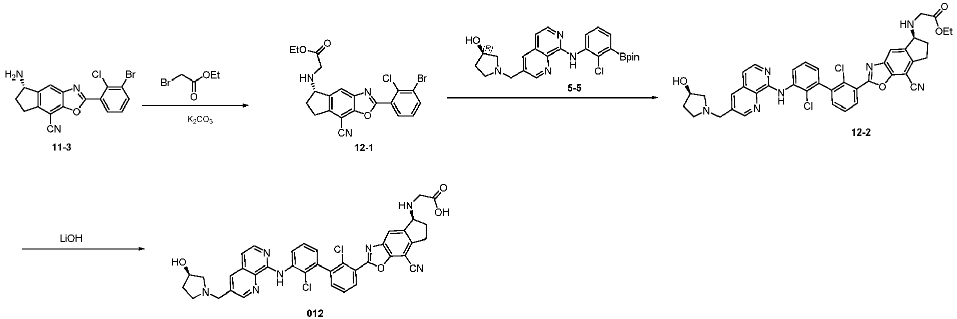

### Step 12-1:

Compound **11-3** (1 g, 2.35 mmol) was dissolved in THF (30 mL), then potassium carbonate (975 mg, 7.06 mmol) and ethyl bromoacetate (393 mg, 2.35 mmol) were added, and the mixture was heated and stirred at 60 °C overnight. The reaction was detected by LC-MS. After the reaction was completed, the mixture was extracted with DCM/H₂O, dried over anhydrous sodium sulfate, and subjected to column chromatography to obtain 600 mg of gray-brown solid.

### Step 12-2:

52 mg of light yellow solid was obtained according to the synthesis method of step 8-4 using compounds **12-1** (200 mg) and **5-5** (280 mg) as raw materials.

### Step 12-3:

21 mg of pale yellow solid was obtained according to the synthesis method of step 8-6 using compound **11-7** (52 mg) as a raw material. ESI (APCI): 720 [M+H]⁺. ¹H NMR (400 MHz, DMSO-d6) δ 9.89 (s, 1H), 9.12 - 8.98 (m, 2H), 8.54 (d, J = 2.0 Hz, 1H), 8.41 (s, 1H), 8.29 - 8.17 (m, 2H), 7.79 - 7.68 (m, 2H), 7.56 (t, J = 8.0 Hz, 1H), 7.38 (d, J = 5.8 Hz, 1H), 7.12 (dd, J = 7.6, 1.5 Hz, 1H), 5.01 (dd, J = 8.1, 3.5 Hz, 1H), 4.62 (s, 1H), 3.96 (d, J = 6.2 Hz, 2H), 3.29 (s, 2H), 2.69 - 2.52 (m, 1H), 2.47 (s, 2H), 1.90 (d, J = 55.7 Hz, 2H).

### Example 13 Synthesis of compound 013

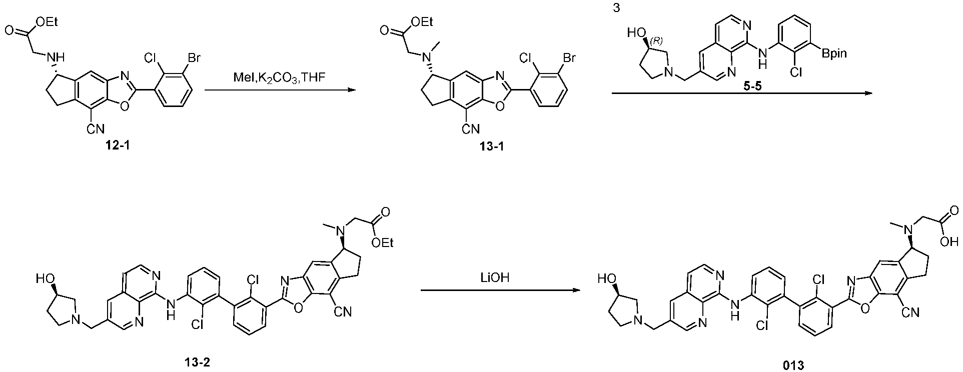

### Step 13-1:

Into a 100mL round-bottomed flask were added **12-1** (150mg, 0.3159mmol), Mel (54mg, 0.3791mmol), K₂CO₃ (87mg, 0.6318mmol), THF (30mL), and the reaction mixture was stirred at room temperature overnight (dark yellow clear) under the protection of N2, and the reaction was detected by LC-MS. After the reaction was completed, THF was spin dried, the mixture was extracted with DCM (20 mL^{∗}2), and subjected to column chromatography to obtain 76 mg of gray solid.

### Step 13-2:

70 mg of pale yellow solid was obtained according to the synthesis method of step 8-4 using compounds **13-1** (50 mg) and **5-5** (52 mg) as raw materials.

### Step 13-3:

20.5 mg of pale yellow solid was obtained according to the synthetic method of step 8-6 using compound **13-2** (62 mg) as a raw material. ESI (APCI): 734 [M+H]⁺. ¹H NMR (400 MHz, DMSO-*d*₆) δ 9.89 (s, 1H), 9.19 - 8.92 (m, 2H), 8.54 (s, 1H), 8.37 (s, 1H), 8.33 - 8.12 (m, 2H), 7.88 - 7.63 (m, 2H), 7.56 (t, *J* = 8.0 Hz, 1H), 7.38 (d, *J* = 5.9 Hz, 1H), 7.13 (dd, *J* = 7.6, 1.5 Hz, 1H), 5.47 (s, 2H), 5.12 (s, 1H), 4.64 (d, *J* = 17.9 Hz, 2H), 4.43 (d, *J* = 26.4 Hz, 1H), 3.28 (s, 2H), 3.25 - 3.15 (m, 2H), 2.68 (s, 2H), 2.08 - 1.90 (m, 2H), 1.83 (s, 2H).

### Example 14 Synthesis of compound 014

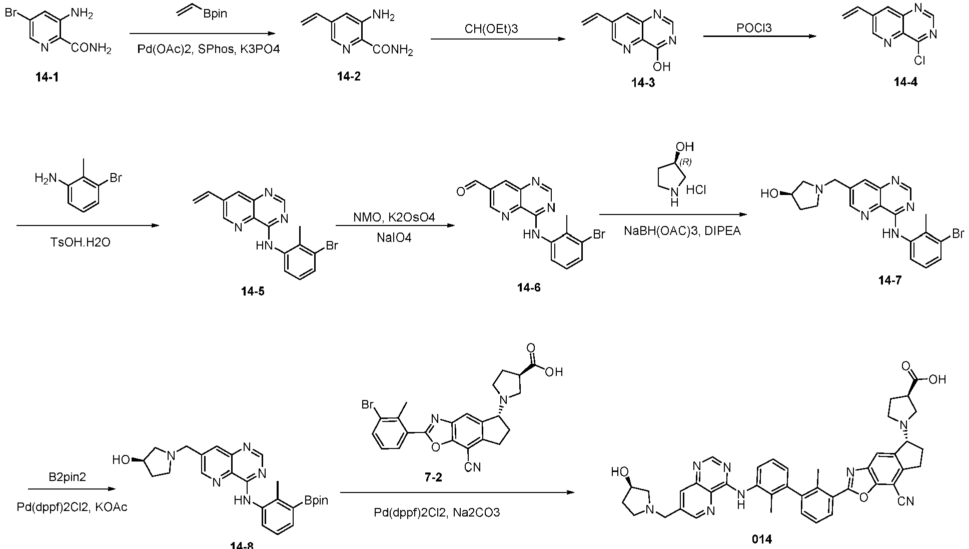

### Step 14-1:

Compound **14-1** (5 g, 0.0231 mol, WO2019148036), pinacol vinylboronate (7.7 mL, 0.0463 mmol), potassium phosphate (14.7 g, 0.0693 mol), Pd(OAc)2 (0.52 g, 0.0023 mol) ) and SPhos (1.89 g, 0.0046 mol) were successively added to a single-necked flask containing 1,4-dioxane/H₂O (4:1, 50 mL), under protection of nitrogen, the mixture was stirred overnight in an oil bath at 85 °C, and the reaction was detected by TLC. When the raw materials disappeared, the reaction solution was spin-dried and subjected to column chromatography to obtain 2.4 g of a pale yellow solid.

### Step 14-2:

Compound **14-2** (1.4 g, 9 mmol) was suspended in triethyl orthoformate (20 mL), the mixture was refluxed in an oil bath at 145 °C for 6 hours, and the reaction was detected by TLC. When the raw materials disappeared, the reaction solution was cooled, n-heptane was added, the solution was stirred and washed for 10 minutes, filtered. The above operation was repeated twice. The mixture was filtered and dried to obtain 1.35 g of a pale yellow solid.

### Step 14-3:

Compound **14-3** (1.25 g, 7.22 mmol) and **DIPEA** (1.4 g, 10.83 mmol) were added to toluene (75 mL), stirred at 100 °C, and then phosphorus oxychloride (1.1 g, 7.22 mmol) was added, and the reaction solution was stirred in an oil bath at 100 °C for 1.5 hours, and the reaction was detected by TLC. When the raw materials disappeared, the reaction solution was slowly poured into ice cubes (50 g), the mixture was stirred for 20 minutes, filtered, separated, washed twice with brine. The organic layer was dried over anhydrous sodium sulfate, and spin-dried to obtain 820 mg of a gray-green solid.

### Step 14-4:

Compound **14-4** (820 mg, 4.44 mmol), 3-bromo-2-methylaniline (908 mg, 4.88 mmol) and TsOH.H₂O (844 mg, 4.44 mmol) were added to isopropanol (20 mL). The mixture was heated to 85°C, refluxed and stirred overnight, and the reaction was detected by TLC. When the raw materials disappeared, the solvent was spin-dried, the mixture was extracted with DCM/H₂O. The organic layers were combined, washed with saturated sodium bicarbonate solution, dried over anhydrous sodium sulfate, and subjected to column chromatography to obtain 600 mg of yellow solid.

### Step 14-5:

Compound **14-5** (600 mg, 1.76 mmol), NMO (412 mg, 3.52 mmol), K₂OsO₄.2H₂O (65 mg, 0.176 mmol) and NaIO₄ (2.63 g, 12.31 mmol) were dissolved in THF/H₂O (10 mL/ 5 mL), the mixture was stirred at room temperature for 3 hours, and the reaction was detected by TLC. When the starting material disappeared, the mixture was filtered and the solid was rinsed with ethyl acetate. The filtrate was taken, washed with 10% sodium thiosulfate and brine, dried over anhydrous sodium sulfate, and concentrated to obtain 600 mg of pale yellow oil, which was used directly in the next reaction.

### Steps 14-6:

Compound **14-6** (600 mg, 1.75 mmol), (R)-3-hydroxypyrrole hydrochloride (949 mg) and DIPEA (678 mg, 5.24 mmol) were dissolved in DCM (20 mL) and the mixture was stirred at room temperature 1 hour. Then NaBH(OAc)3 (741 mg, 3.5 mmol) was added to the above reaction solution and the mixture was stirred at room temperature overnight. When the raw materials disappeared, saturated sodium bicarbonate solution was added dropwise to quench the reaction, the reaction slution was extracted with ethyl acetate/water. The organic layers were combined, dried over anhydrous sodium sulfate, and subjected to column chromatography to obtain 350 mg of pale yellow solid.

### Steps 14-7:

300 mg of light yellow solid was obtained according to the synthesis method of step 8-3 using compound **14-7** (350 mg) as a raw material.

### Steps 14-8:

300 mg of light yellow solid was obtained according to the synthesis method of step 8-4 using compounds **14-8** (200 mg) and **7-2** (180 mg) as raw materials. MS (APCI): 721 [M+H]⁺

### Example 15 Synthesis of compound 015

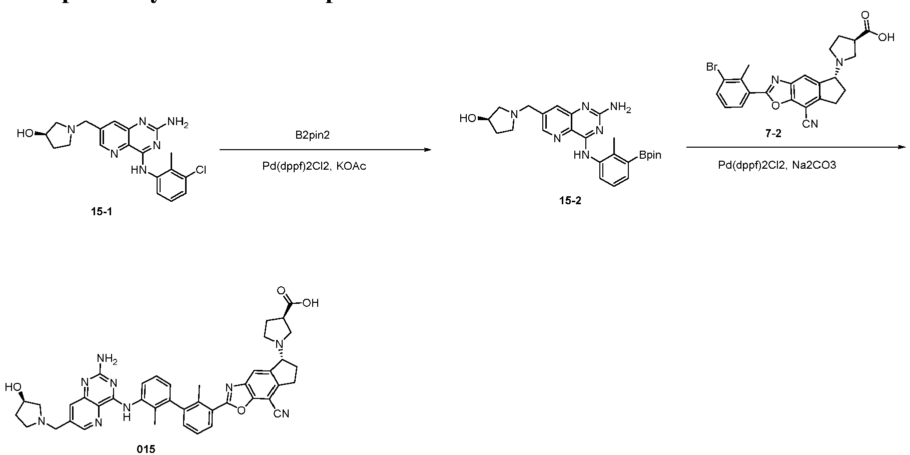

### Step 15-1:

150 mg of light yellow solid was obtained according to the synthesis method of step 8-3 using compound **15-1** (200 mg) as a raw material.

### Step 15-2:

21 mg of light yellow solid was obtained according to the synthesis method of step 8-4 using compounds **15-1** (100 mg, WO2019191707) and **7-2** (120 mg) as raw materials. MS (APCI): 736 [M+H]⁺.

### Example 16 Synthesis of compound 016

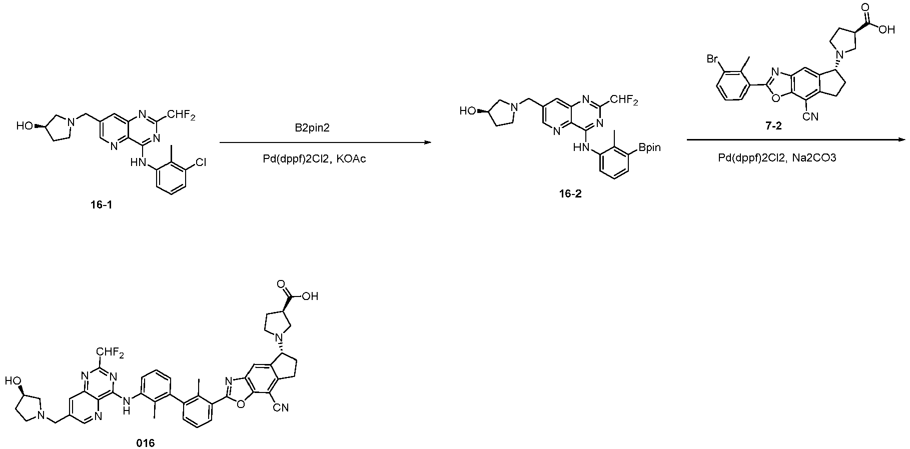

### Step 16-1:

100 mg of light yellow solid was obtained according to the synthesis method of step 8-3 using compound **16-1** (200 mg) as a raw material.

### Step 16-2:

25 mg of light yellow solid was obtained according to the synthesis method of step 8-4 using compounds **16-1** (50 mg, WO2019191707) and **7-2** (56 mg) as raw materials. MS (APCI): 771 [M+H]⁺.

According to the synthetic method of compound 001-016, the compounds in the following table were synthesized with corresponding raw materials and reagents:

| Compound | LCMS [M+H]⁺ | Compound | LCMS [M+H]⁺ | Compound | LCMS [M+H]⁺ |
|---|---|---|---|---|---|
| **017** | 733 | **114** | 880 | **196** | 885 |
| **018** | 719 | **115** | 896 | **197** | 901 |
| **019** | 705 | **116** | 878 | **198** | 883 |
| **020** | 691 | **117** | 857 | **199** | 862 |
| **021** | 759 | **118** | 846 | **200** | 851 |
| **022** | 621 | **119** | 816 | **201** | 821 |
| **023** | 679 | **120** | 866 | **202** | 871 |
| **024** | 741 | **121** | 882 | **203** | 887 |
| **025** | 706 | **122** | 864 | **204** | 869 |
| **026** | 740 | **123** | 843 | **205** | 848 |
| **027** | 740 | **124** | 832 | **206** | 837 |
| **028** | 694 | **125** | 870 | **207** | 801 |
| **029** | 760 | **126** | 920 | **208** | 851 |
| **030** | 760 | **127** | 897 | **209** | 867 |
| **031** | 734 | **128** | 856 | **210** | 849 |
| **032** | 823 | **129** | 906 | **211** | 828 |
| **033** | 863 | **130** | 883 | **212** | 817 |
| **034** | 787 | **131** | 850 | **213** | 801 |
| **035** | 827 | **132** | 900 | **214** | 851 |
| **036** | 788 | **133** | 916 | **215** | 867 |
| **037** | 828 | **134** | 898 | **216** | 849 |
| **038** | 699 | **135** | 877 | **217** | 828 |
| **039** | 699 | **136** | 866 | **218** | 817 |
| **040** | 740 | **137** | 836 | **219** | 815 |
| **041** | 720 | **138** | 886 | **220** | 865 |
| **042** | 823 | **139** | 902 | **221** | 881 |
| **043** | 748 | **140** | 884 | **222** | 863 |
| **044** | 696 | **141** | 863 | **223** | 842 |
| **045** | 733 | **142** | 852 | **224** | 831 |
| **046** | 734 | **143** | 850 | **225** | 815 |
| **047** | 622 | **144** | 900 | **226** | 865 |
| **048** | 706 | **145** | 916 | **227** | 881 |
| **049** | 764 | **146** | 898 | **228** | 863 |
| **050** | 736 | **147** | 876 | **229** | 842 |
| **051** | 695 | **148** | 866 | **230** | 831 |
| **052** | 723 | **149** | 836 | **231** | 808 |
| **053** | 693 | **150** | 886 | **232** | 808 |
| **054** | 641 | **151** | 902 | **233** | 822 |
| **057** | 719 | **152** | 884 | **234** | 822 |
| **058** | 731 | **153** | 863 | **235** | 740 |
| **059** | 745 | **154** | 852 | **236** | 863 |
| **060** | 742 | **155** | 685 | **237** | 843 |
| **061** | 729 | **156** | 678 | **238** | 843 |
| **062** | 679 | **157** | 728 | **239** | 837 |
| **063** | 837 | **158** | 744 | **240** | 877 |
| **064** | 751 | **159** | 726 | **241** | 857 |
| **065** | 711 | **160** | 705 | **242** | 857 |
| **066** | 613 | **161** | 694 | **243** | 719 |
| **067** | 604 | **162** | 725 | **244** | 719 |
| **068** | 752 | **163** | 718 | **245** | 759 |
| **069** | 724 | **164** | 768 | **246** | 739 |
| **070** | 649 | **165** | 784 | **247** | 739 |
| **071** | 666 | **166** | 766 | **248** | 822 |
| **083** | 811 | **167** | 745 | **249** | 862 |
| **085** | 877 | **168** | 734 | **250** | 842 |
| **087** | 914 | **169** | 698 | **251** | 842 |
| **088** | 928 | **170** | 748 | **252** | 836 |
| **089** | 713 | **171** | 764 | **253** | 876 |
| **090** | 763 | **172** | 746 | **254** | 856 |
| **091** | 779 | **173** | 725 | **255** | 856 |
| **092** | 761 | **174** | 714 | **256** | 684 |
| **093** | 740 | **175** | 705 | **257** | 724 |
| **094** | 729 | **176** | 698 | **258** | 704 |
| **095** | 753 | **177** | 748 | **259** | 704 |
| **096** | 803 | **178** | 764 | **260** | 787 |
| **097** | 819 | **179** | 746 | **261** | 827 |
| **098** | 801 | **180** | 725 | **262** | 807 |
| **099** | 780 | **181** | 714 | **263** | 807 |
| **100** | 769 | **182** | 705 | **264** | 801 |
| **101** | 733 | **183** | 781 | **265** | 841 |
| **102** | 783 | **184** | 830 | **266** | 821 |
| **103** | 799 | **185** | 847 | **267** | 821 |
| **104** | 781 | **186** | 829 | **268** | 732 |
| **105** | 760 | **187** | 808 | **269** | 835 |
| **106** | 749 | **188** | 797 | **270** | 849 |
| **107** | 733 | **189** | 795 | **271** | 731 |
| **108** | 783 | **190** | 845 | **272** | 834 |
| **109** | 799 | **191** | 861 | **273** | 848 |
| **110** | 781 | **192** | 843 | **274** | 766 |
| **111** | 760 | **193** | 822 | **275** | 869 |
| **112** | 749 | **194** | 811 | **276** | 883 |
| **113** | 830 | **195** | 835 | **277** | 767 |
| **278** | 870 | **279** | 884 | | |

### Example 280 Synthesis of Compound 280

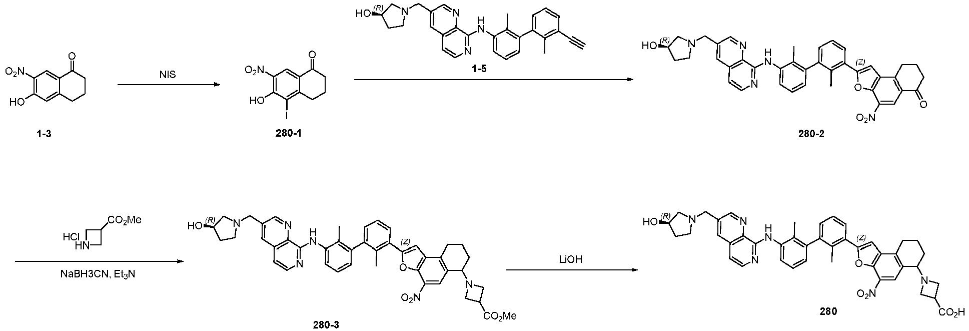

### Step 280-1:

3.1 g of light yellow solid was obtained according to the synthesis method of step 1-2 using compound **1-3** (2.4 g) as a raw material. MS-APCI: 334 [M+H]⁺.

### Step 280-2:

200 mg of light yellow solid was obtained according to the synthesis method of step 1-3 using compound **280-1** (500 mg) as a raw material. MS-APCI: 654 [M+H]⁺.

### Step 280-3:

60 mg of light yellow solid was obtained according to the synthesis method of step 1-4 using compound **280-2** (200 mg) as a raw material. MS-APCI: 753 [M+H]⁺.

### Step 280-4:

16 mg of light yellow solid was obtained according to the synthesis method of step 1-5 using compound **280-3** (60 mg) as a raw material. MS-APCI: 654 [M+H]⁺.

¹H NMR (400 MHz, DMSO-*d*6) *δ* 9.34 (s, 1H), 9.00 (s, 1H), 8.46 (s, 1H), 8.34 (d, *J* = 8.0 Hz, 1H), 8.08 (d, *J* = 8.0 Hz, 1H), 7.77 (d, *J* = 8.0 Hz, 1H), 7.44 (t, *J* = 8.0 Hz, 1H), 7.35 - 7.25 (m, 3H), 7.19 (d, *J* = 8.0 Hz, 1H), 6.91 (d, *J* = 8.0 Hz, 1H), 6.52 (m, 1H), 4.49-4.39 (m, 3H), 3.37-2.83 (m, 3H), 2.66-2.28 (m, 2H), 2.21 (s, 3H), 2.18-2.06 (m, 1H), 2.06 (s, 3H), 1.96-1.58 (m, 5H), 1.43 (m, 1H).

### Example 281 Synthesis of Compound 280

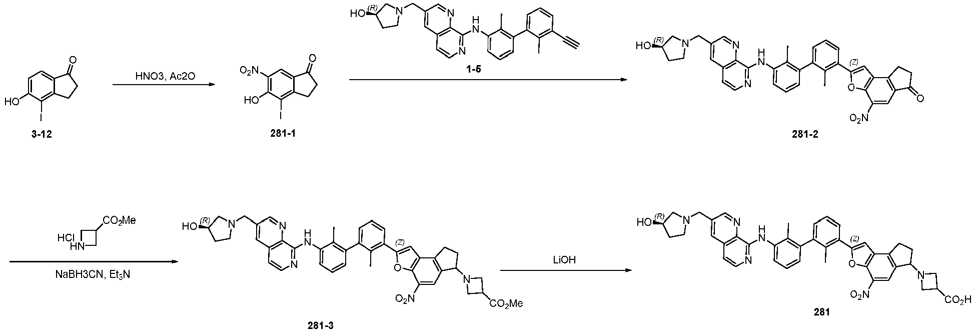

### Step 281-1:

3 g of light yellow solid was obtained according to the synthesis method of step 4-1 using compound **3-12** (5 g) as a raw material. MS-APCI: 320 [M+H]⁺.

### Step 281-2:

400 mg of light yellow solid was obtained according to the synthesis method of step 1-3 using compound **280-1** (1 g) as a raw material. MS-APCI: 640 [M+H]⁺.

### Step 281-3:

10 mg of light yellow solid was obtained according to the synthesis method of step 1-4 using compound **280-2** (400 mg) as a raw material. MS-APCI: 739 [M+H]⁺.

### Step 281-4:

36 mg of light yellow solid was obtained according to the synthesis method of step 1-5 using compound **280-3** (100 mg) as a raw material. MS-APCI: 725 [M+H]

### Biological Test

### Example A: PD-1/PD-L1 Homogeneous Time-Resolved Fluorescence (HTRF) Binding Assay

Assays were performed in standard black 384-well polystyrene plates and a final volume was 20 µL. The inhibitor was first serially diluted with DMSO and added to the wells of the plate, followed by the addition of other reaction components. The final concentration of DMSO in the assay was 1%. Assays were performed at 25°C in PBS buffer (pH 7.4) containing 0.05% Tween-20 and 0.1% BSA. Recombinant human PD-L1 protein (19-238) with a His-tagged at the C-terminus was purchased from AcroBiosystems (PD1-H5229). Recombinant human PD-1 protein (25-167) with an Fc tag at the C-terminus was also purchased from AcroBiosystems (PD1-H5257). The PD-L1 and PD-1 proteins were diluted in assay buffer and then 0.1 µl of the solution was extracted and added to the wells of the plate. Plates were centrifuged and proteins and inhibitors were preincubated for 40 minutes. After incubation, 0.1 µl of HTRF detection buffer containing europium blocking labeled anti-human IgG (PerkinElmer-AD0212), Fc-specific and anti-His SureLight^{®}-Allophycocyanin (APC, PerkinElmer-AD0059H) conjugated antibodies was added. After centrifugation, the plates were incubated at 25°C for 60 minutes. Data was read in a PHERAstar FS plate reader (665nm/620nm ratio). Final concentrations in the assay were ~3 nM PD1, 10 nM PD-L1, 1 nM europium anti-human IgG, and 20 nM anti-His-allophycocyanin. Activity data were fitted using GraphPad Prism 5_0 software to derive IC50 values for inhibitors.

The IC50 values of the compounds exemplified in the examples are expressed in the following manner: IC50: +: ≤ 10 nM; ++: 10 nM~100 nM; +++: > 100 nM.

The data of the example compounds obtained using the PD-1/PD-L1 homogeneous time-resolved fluorescence (HTRF) binding assay described in Example A showed that the IC50 values of most of the tested compounds of the present invention are less than 10 nm, and the IC50 values of some compounds (about 25 %) are even less than 1 nM. The activity test data of some preferred compounds were provided in Table 1.

| Compound | IC₅₀ | Compound | IC₅₀ | Compound | IC₅₀ |
|---|---|---|---|---|---|
| **001** | + | **035** | + | **015** | + |
| **002** | + | **036** | + | **016** | + |
| **003** | + | **037** | + | **243** | + |
| **004** | + | **038** | + | **244** | + |
| **005** | + | **039** | + | **245** | + |
| **006** | + | **040** | + | **246** | + |
| **007** | + | **041** | + | **247** | + |
| **008** | + | **042** | + | **248** | + |
| **009** | + | **043** | ++ | **249** | + |
| **010** | + | **081** | + | **250** | + |
| **011** | + | **082** | + | **251** | + |
| **012** | + | **083** | + | **252** | + |
| **013** | + | **084** | + | **253** | + |
| **017** | + | **086** | + | **254** | + |
| **018** | + | **087** | + | **255** | + |
| **019** | + | **088** | + | **256** | + |
| **020** | + | **162** | + | **257** | + |
| **021** | + | **175** | + | **258** | + |
| **022** | + | **182** | + | **259** | + |
| **023** | + | **231** | + | **260** | + |
| **024** | + | **232** | + | **261** | + |
| **025** | + | **233** | + | **262** | + |
| **026** | + | **234** | + | **263** | + |
| **027** | + | **235** | + | **264** | + |
| **028** | + | **236** | + | **265** | + |
| **029** | + | **237** | + | **266** | + |
| **030** | + | **238** | + | **267** | + |
| **031** | + | **239** | + | **268** | + |
| **032** | + | **240** | + | **271** | + |
| **033** | + | **241** | + | **274** | + |
| **034** | + | **242** | + | **280** | +++ |

All documents mentioned herein are incorporated by reference in the present invention as if each document was individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A compound represented by the following formula I, or an optical isomer, a hydrate, a solvate thereof, or a pharmaceutically acceptable salt thereof: 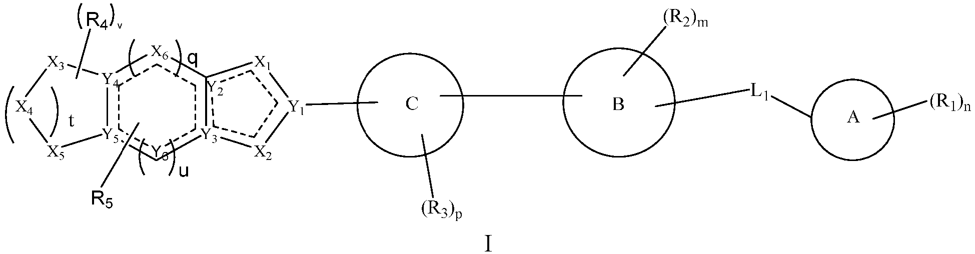 wherein, n is 0, 1, 2, 3, 4, or 5;
m, p, q, t, v, and u are each independently selected from 0, 1, 2, 3 or 4;
X₁, X₂, X₃, X₄, X₅ and X₆ are each independently selected from the group consisting of N, O, S, SO, SO₂, C(R)₂, CHR, and NR;
Y₁, Y₂, Y₃, Y₄, Y₅ and Y₆ are each independently selected from the group consisting of N, CH, and C;
wherein, a hydrogen (if present) on a carbon atom of X₃, X₄, X₅ may be each independently substituted by deuterium; 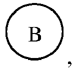 and 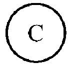 are each independently selected from the group consisting of substituted or unsubstituted C6-C10 arylene, or substituted or unsubstituted 5-12 membered (preferably 5-7 membered) heteroarylene having 1-3 heteroatoms, substituted or unsubstituted 5-12 membered heterocyclylene, and substituted or unsubstituted 5-12 membered C3-C12 (preferably C5-C12) cycloalkylene; 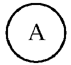 is selected from the group consisting of substituted or unsubstituted 5-12 membered heteroaryl, substituted or unsubstituted C6-C10 aryl, substituted or unsubstituted 5-12 membered heterocyclyl, substituted or unsubstituted 5-12 membered C3-C12 (preferably C5-C12) cyclic group, wherein the heterocyclyl has 1-3 heteroatoms;
L₁ is selected from the group consisting of chemical bond, substituted or unsubstituted C1-C4 alkylene, substituted or unsubstituted C2-C4 alkenylene, substituted or unsubstituted C2-C4 alkynylene, -S-, -O-, substituted or unsubstituted -NH-, -S(O)-, -S(O)₂-, substituted or unsubstituted -NHC(O)NH-, 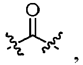 substituted or unsubstituted 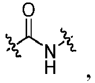 substituted or unsubstituted 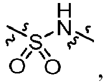 and substituted or unsubstituted 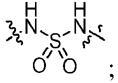 and 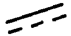 represents single bond or double bond;
R, R₁, R₂, R₃, R₄, and R₅ are each independently selected from the group consisting of H, -CN, trifluoromethyl, -CHF2, -OCF3, -OCHF2, sulfonamido, nitro, hydroxyl, halogen, -S-R₈, - S(O)-R₈, -S(O)₂-R₈, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NR_{d}Rₑ (eg amino), substituted or unsubstituted C1-C6 amino, substituted or unsubstituted - (C1-C6 alkylene)-NH-(C1-C6 alkylene), carboxy, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms, wherein a hydrogen on a carbon atom of substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (i.e. =O), =NRf, -CN, hydroxyl, NR_{d}Rₑ (e.g. amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12 membered heterocyclyl with 1-4 heteroatoms may be each independently substituted by deuterium; substituted or unsubstituted 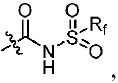 substituted or unsubstituted 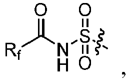 substituted or unsubstituted 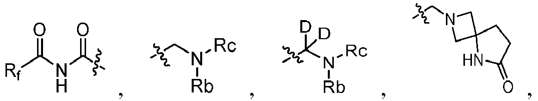 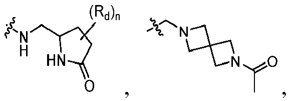 wherein, Rb and Rc are are each independently selected from the group consisting of H, substituted or substituted C₁-C₈ alkyl; or Rb and Rc together with adjacent N atom form substituted or unsubstituted 3-10 membered heterocyclyl having 1-3 heteroatoms selected from N, S and O, or Rb and Rc together with adjacent N atom form substituted or unsubstituted 4-10 membered lactam, the substituents include but are not limited to hydroxyl, carboxyl, sulfhydryl, amino, F, Cl, wherein, a hydrogen on a carbon atom of R_{b}, R_{c} and Rd may be each independently substituted by deuterium; or -(L₁ₐ)ᵣ-(L₂ₐ)ₛ-(L₃ₐ)ₛ-; -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉;
R₈ and R₉ are each independently selected from the group consisting of H, hydroxy, substituted or unsubstituted C1-C10 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NR_{d}Rₑ (eg amino), substituted or unsubstituted C1-C6 amino, substituted or unsubstituted-(C1-C6 alkylene)-NH-(C1-C6 alkylene), carboxyl, substituted or unsubstituted C6-C10 aryl, substituted or unsubstituted 5-12 membered heteroaryl with 1-3 heteroatoms, substituted or unsubstituted 3-12-membered heterocyclyl with 1-4 heteroatoms, substituted or unsubstituted 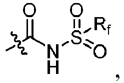 substituted or unsubstituted 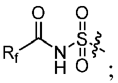 substituted or unsubstituted 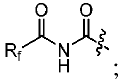 or -(L₁ₐ)ᵣ-(L₂ₐ)ₛ-(L₃ₐ)ₛ-;
each L₁ₐ is each independently the group selected from the group consisting of chemical bond, substituted or unsubstituted C₁-C₇ alkylene, substituted or unsubstituted C2-C4 alkenylene, substituted or unsubstituted C2-C4 alkynylene, -S-, -O-, substituted or unsubstituted -NH-, -S(O)-, and -S(O)₂-;
L2a is selected from the group consisting of substituted or unsubstituted C6-C12 arylene, substituted or unsubstituted 5-12 membered heteroarylene with 1-3 heteroatoms, substituted or unsubstituted C3-C8 cycloalkylene, and substituted or unsubstituted 3-10 membered heterocyclylene with 1-3 heteroatoms;
L3a is selected from the group consisting of substituted or unsubstituted C1-C10 alkyl, C1-C10 aryl, -CN, hydroxyl, amino, carboxyl, -CO-NH-SO₂-R_{g}, -NH-SO₂-R_{g}, -SO₂-NH-CO-R_{g}, - OR_{g}, -N(R_{g})₂, -CO₂R_{g}, -CON(R_{g})₂, -CONHCOR_{g}, NR_{g}-CO-N(R_{g})₂, and -NRg-SO₂-N(R_{g})₂;
r is 1, 2, 3, 4, 5, 6;
s is 0, 1, 2 respectively;
R_{d}, Rₑ and R_{g} are each independently selected from the group consisting of H, substituted or unsubstituted C₁-C₆ alkyl, substituted or unsubstituted C₃-C₁₀ cycloalkyl, substituted or unsubstituted C₆-C₁₀ aryl; or Rd and Re together form substituted or unsubstituted 4-10 (preferably 5-10) membered heterocyclyl having 1-3 heteroatoms selected from N, S and O;
R_{f} is selected from the group consisting of H, substituted or unsubstituted C₁-C₆ alkyl, substituted or unsubstituted C₆-C₁₀ aryl, substituted or unsubstituted 5-10 membered heteroaryl, cyano, -C(=O)-NR_{d}Rₑ, -C(=O)-substituted or unsubstituted C₁-C₆ alkoxy, -C(=O)-substituted or unsubstituted C₁-C₆ alkyl, -C(=O)-substituted or unsubstituted C₃-C₁₀ cycloalkyl, -C(=O)-substituted or unsubstituted C₂-C₆ alkenyl, and -C(=O)-substituted or unsubstituted C₂-C₆ alkynyl;
unless otherwise specified, the "substituted" refers to substitution with one or more (eg 2, 3, 4, etc.) substituents selected from the group consisting of halogen (including -F, -Cl, -Br, - I), -CH₂Cl, -CHCl₂, -CCl₃, -CH₂F, -CHF₂, -CF₃, oxo (=O), -CN, hydroxyl, amino, C1-C6 alkylamino, carboxyl, -NHAc, 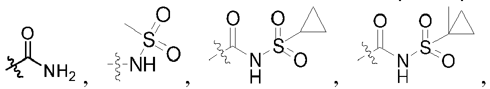 substituted or unsubstituted group selected from the group consisting of C1-C6 alkyl, C1-C6 alkoxy, C6-C10 aryl, C3-C8 cycloalkyl, halogenated C6-C10 aryl, 5-10 membered heteroaryl with 1-3 heteroatoms selected from N, S and O, 5-10 membered heterocyclyl with 1-3 heteroatoms selected from N, S and O; the substituent is selected from the group consisting of halogen, hydroxyl, carboxyl, oxo, cyano, C1-C6 alkoxy, and C1-C6 alkylamino;
in the above formulas, any one of the heteroatom is selected from the group consisting of B, P, N, S and O.

2. The compound according to claim 1, or the isomer, optical isomer, hydrate, solvate, or the pharmaceutically acceptable salt thereof, wherein 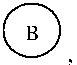 and 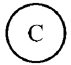 are each independently a divalent group formed by a ring selected from the following group: 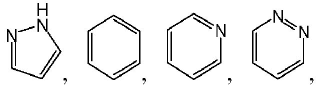 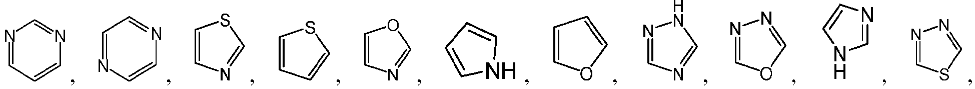 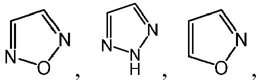 , wherein the bonding position of the ring can be N or C; preferrably, 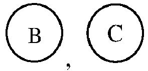 are each independently

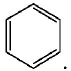

3. The compound according to claim 1, wherein 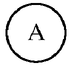 has a structure shown in the following formula: 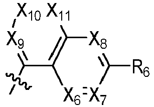 wherein,
X₆, X₇, X₈, X₉, X₁₀ and X₁₁ are each independently selected from the group consisting of N, CR;
R6 is selected from the group consisting of H, halogen, substituted or unsubstituted C1-C6 alkyl, substituted or unsubstituted C2-C6 alkenyl, substituted or unsubstituted C2-C6 alkynyl, substituted or unsubstituted C1-C6 alkoxy, substituted or unsubstituted C3-C8 cycloalkyl, oxo (ie =O), =NRf, -CN, hydroxyl, NR_{d}Rₑ (eg amino), substituted or unsubstituted C1-C6 amino, substituted or unsubstituted -(C1-C6 alkylene)-NH-(C1-C6 alkylene), carboxyl, substituted or unsubstituted C6-C10 aryl, substituted or unsubstituted 5-12 membered heteroaryl with 1-3 heteroatoms substituted or unsubstituted 5-12 membered heterocyclyl with 1-4 heteroatoms substituted or unsubstituted 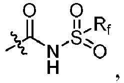 substituted or unsubstituted 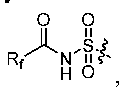 substituted or unsubstituted 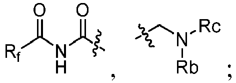 or -(L₁ₐ)ᵣ-(L₂ₐ)ₛ-(L₃ₐ)ₛ-, -C₀₋₈-O-R₈, -C₀₋₈-C(O)OR₈, -C₀₋₈-OC(O)OR₈, -C₀₋₈-NR₈R₉, -C₀₋₈-N(R₈)C(O)R₉, -C₀₋₈-C(O)NR₈R₉.

4. The compound according to claim 1, or the optical isomer, hydrate, solvate thereof, or the pharmaceutically acceptable salt thereof, wherein 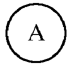 has a structure selected from the group consisting of 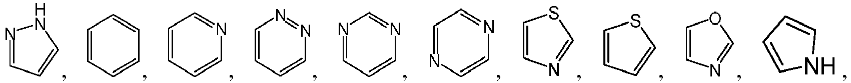 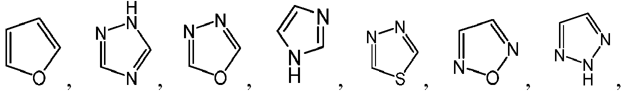 and 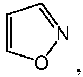 wherein the bonding position of the ring can be N or C.

5. The compound according to claim 1, or the optical isomer, hydrate, solvate thereof, or the pharmaceutically acceptable salt thereof, formula IV: 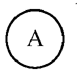 has a substitutent as shown in the following 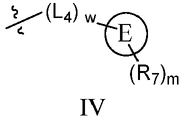 wherein, w is 0, 1, 2, 3, 4, 5, or 6;
each L₄ is independently selected from the group consisting of substituted or unsubstituted C1-C4 alkylene, -S-, -O-, NR_{f}, -S(O)-, -S(O)₂-; preferably substituted or unsubstituted C1-C4 alkylene, wherein, a hydrogen on a carbon atom of the substituted or unsubstituted C1-C4 alkylene may be each independently substituted by deuterium, provided that a structure formed by each L4 is chemically stable; 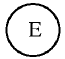 is selected from the group consisting of substituted or unsubstituted C3-C10 (preferrably C5-C10) cycloalkyl, substituted or unsubstituted 3-10 membered heterocyclyl having 1-3 heteroatoms selected from B, P, N, S and O; preferably, 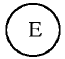 is substituted or unsubstituted 3-8 membered nitrogen-containing heterocyclyl, or substituted or unsubstituted 4-10 membered cyclic amido, wherein a hydrogen on the ring-forming carbon atom of 3-8 membered nitrogen-containing heterocyclyl, substituted or unsubstituted 4-10 membered cyclic amido may be each independently substituted by deuterium;
each R₇ is independently selected from the group consisting of substituted or unsubstituted C1-C6 alkyl, -CN, hydroxy, amino, carboxyl, -OR_{g}, -N(R_{g})₂, -CO-NH-SO₂-R_{g}, -NH-SO₂-R_{g}, -SO₂-NH-CO-R_{g}, -CO₂R_{g}, -CON(R_{g})₂, -CONHCOR_{g}, NR_{g}-CO-N(R_{g})₂, -NR_{g}-SO₂-N(R_{g})₂; R_{f} and R_{g} are defiend as above, wherein a hydrogen on a carbon atom of R_{f} and R_{g} may be independently substituted by deuterium; wherein, the substituents are selected from the group consisting of halogen, hydroxyl, carboxyl, cyano, and C1- C6 alkoxy.

6. The compound of claim 1, wherein consisting of: 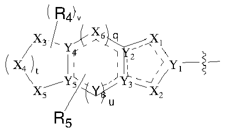 is selected from the group

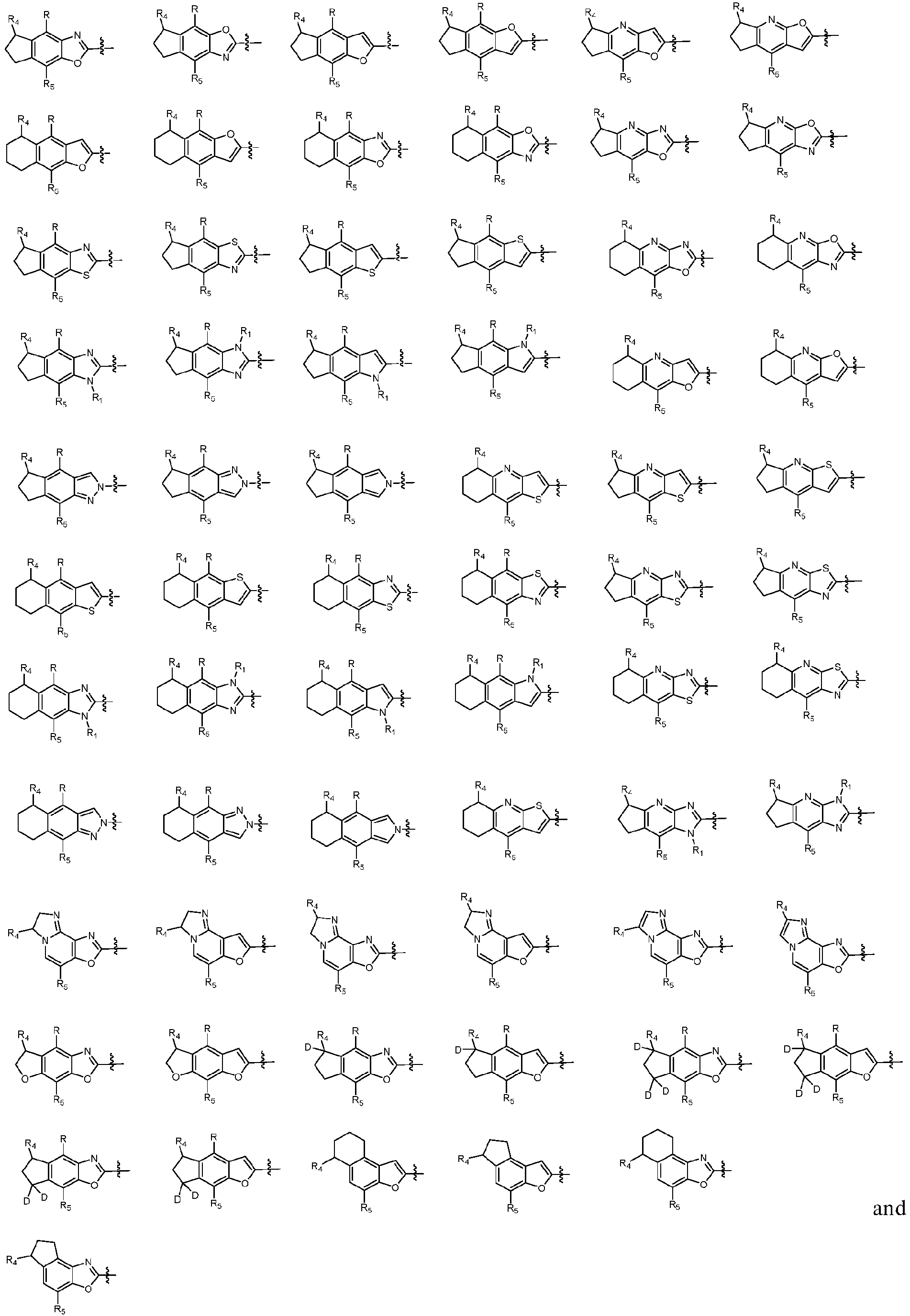

7. The compound according to claim 1, or the optical isomer, hydrate, solvate thereof, or the pharmaceutically acceptable salt thereof, wherein the compound is selected from the following list:

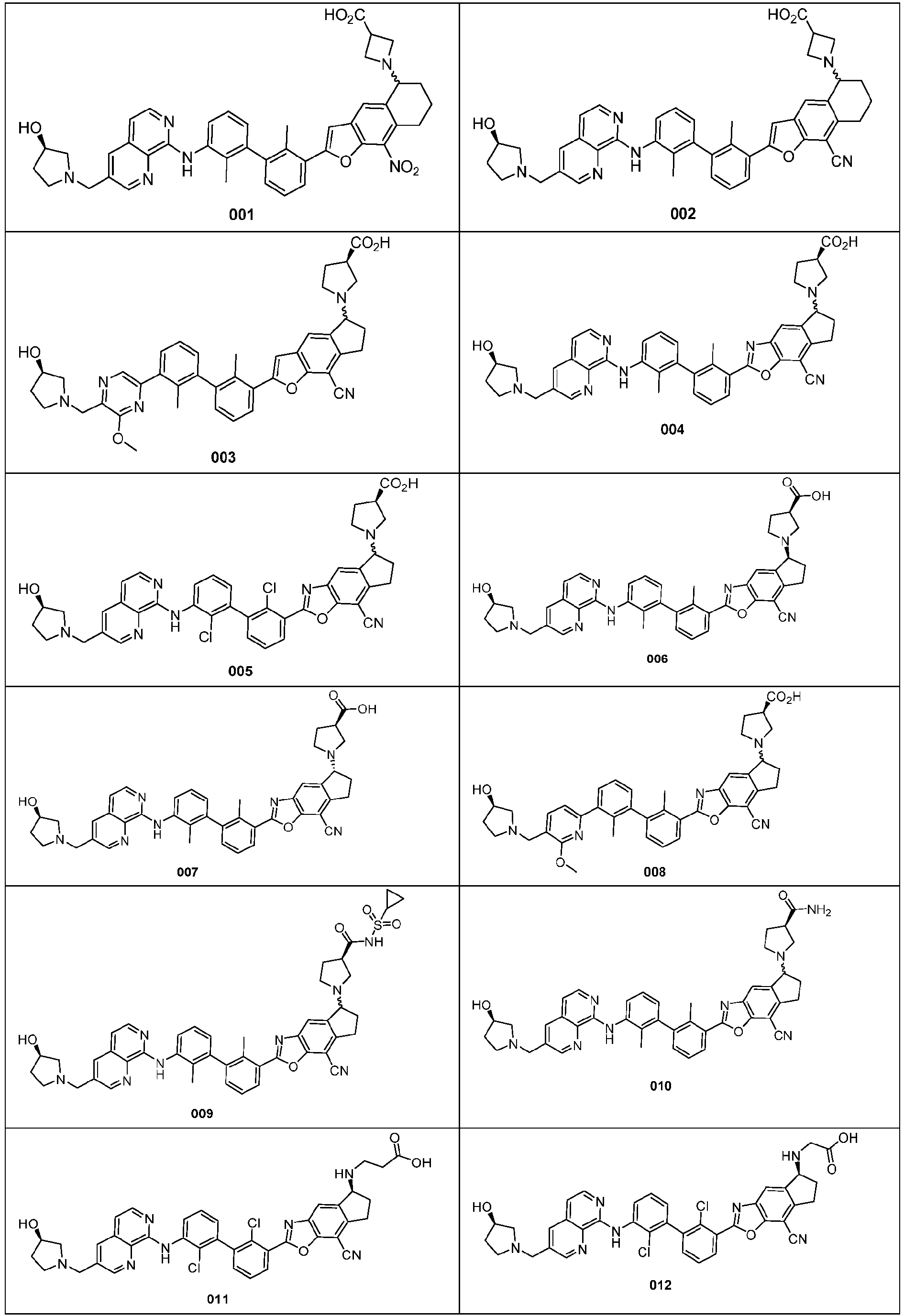

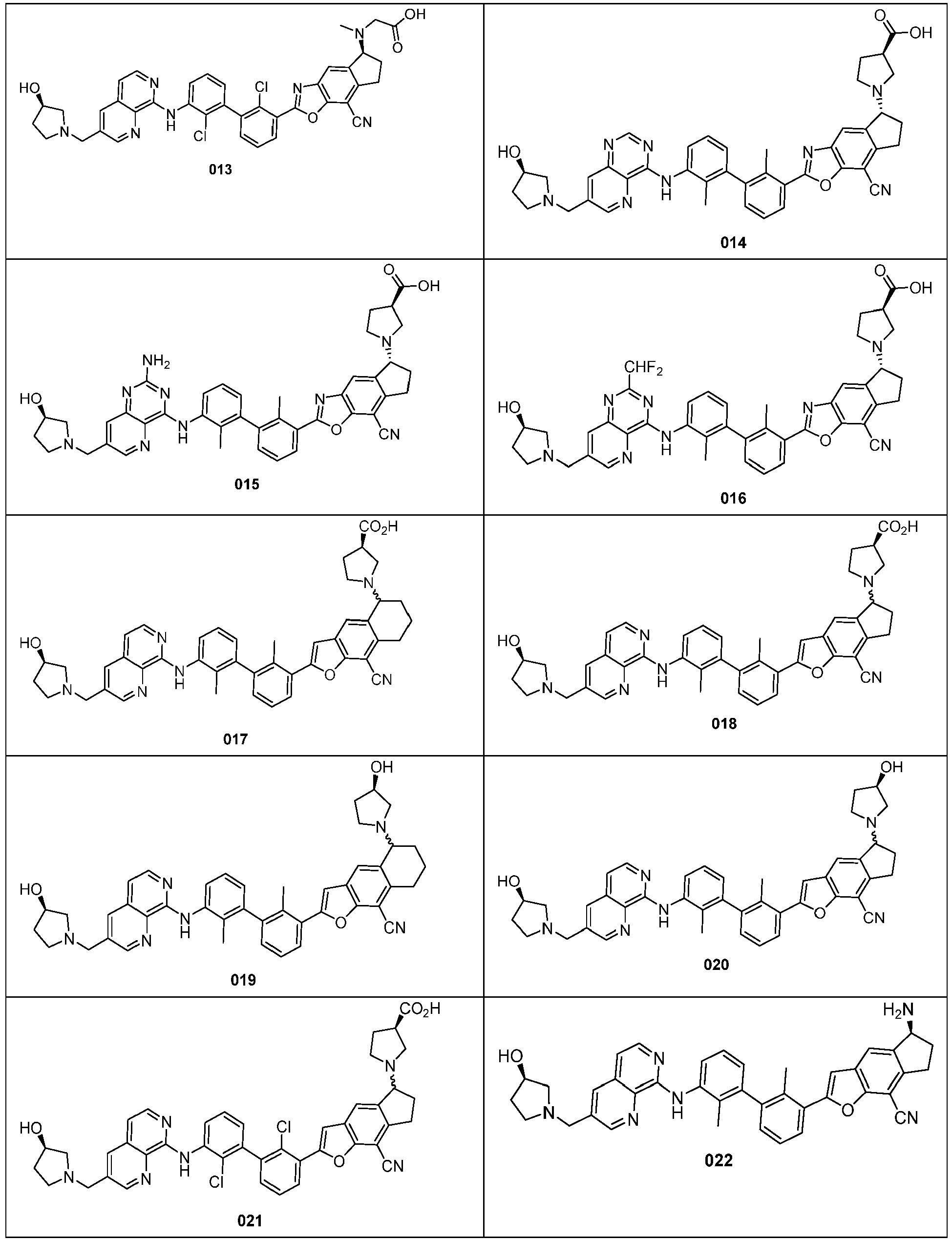

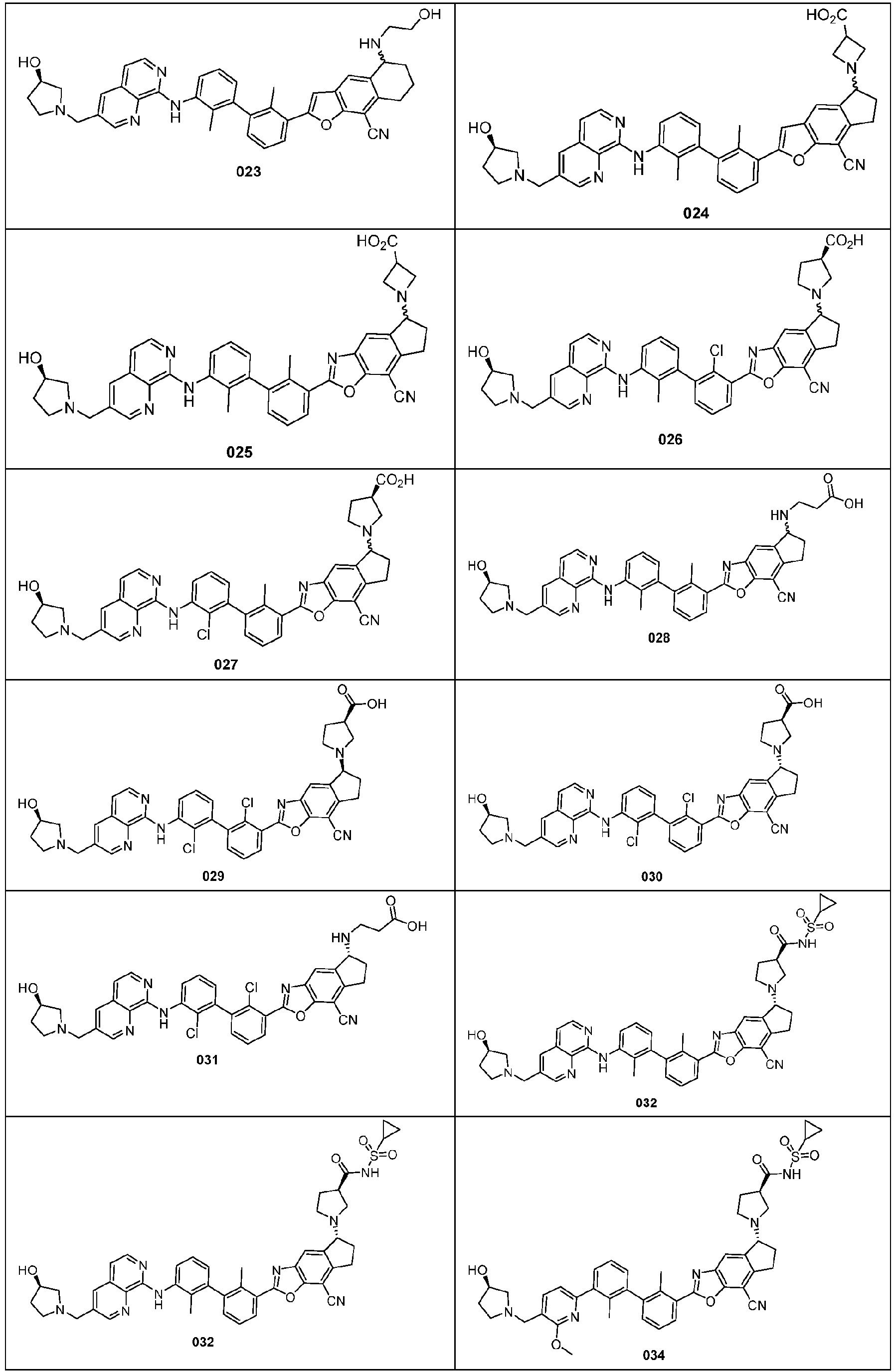

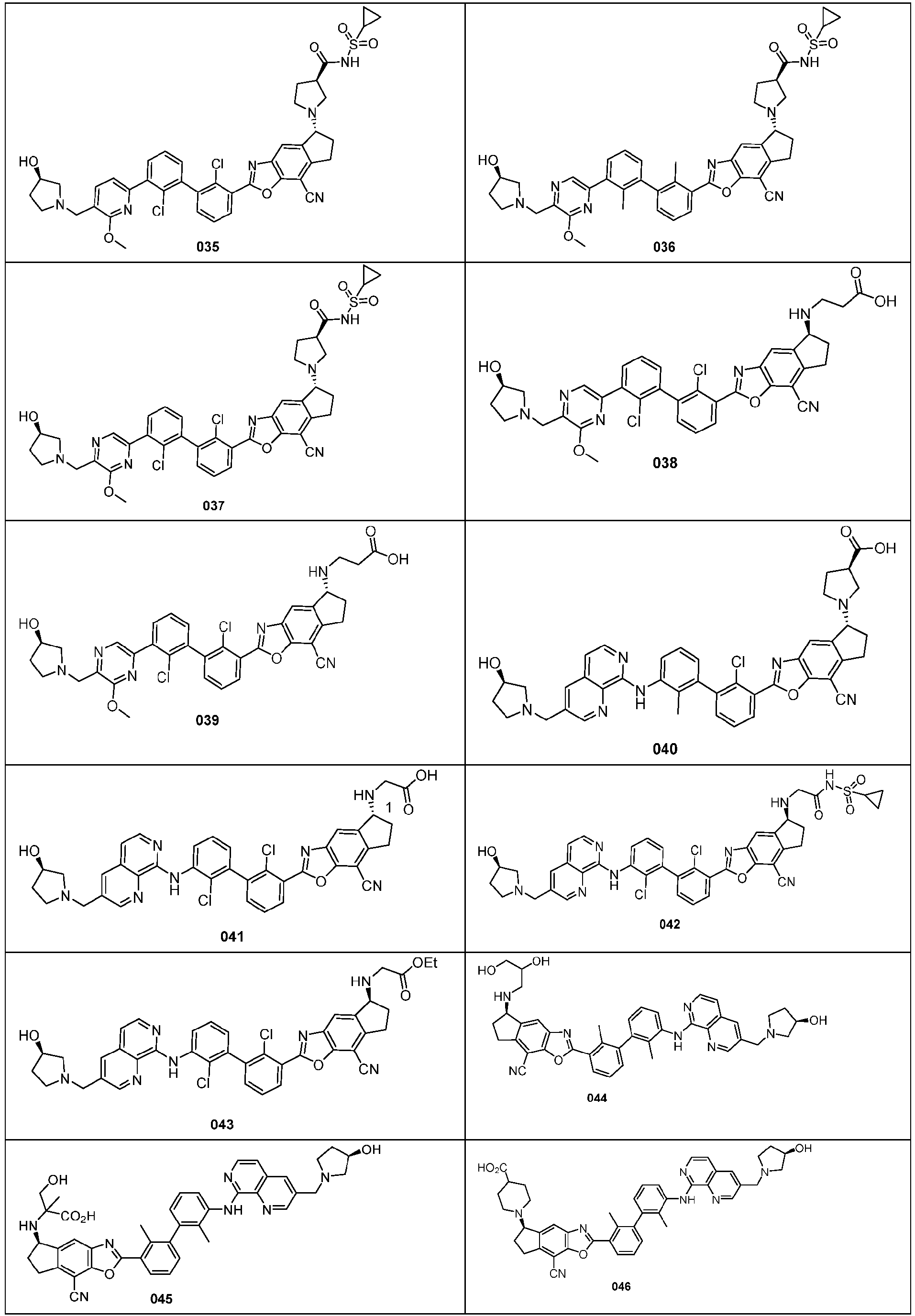

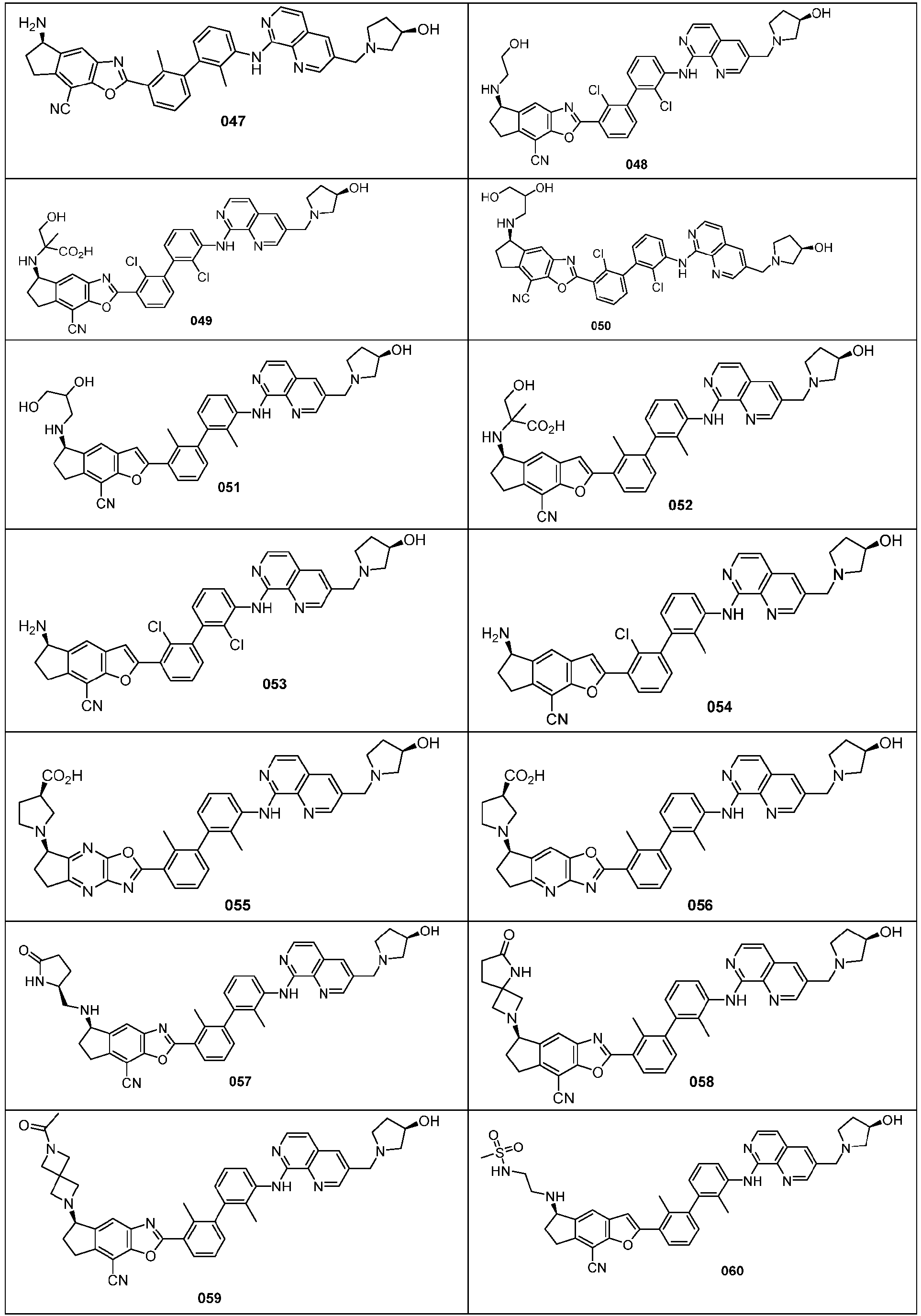

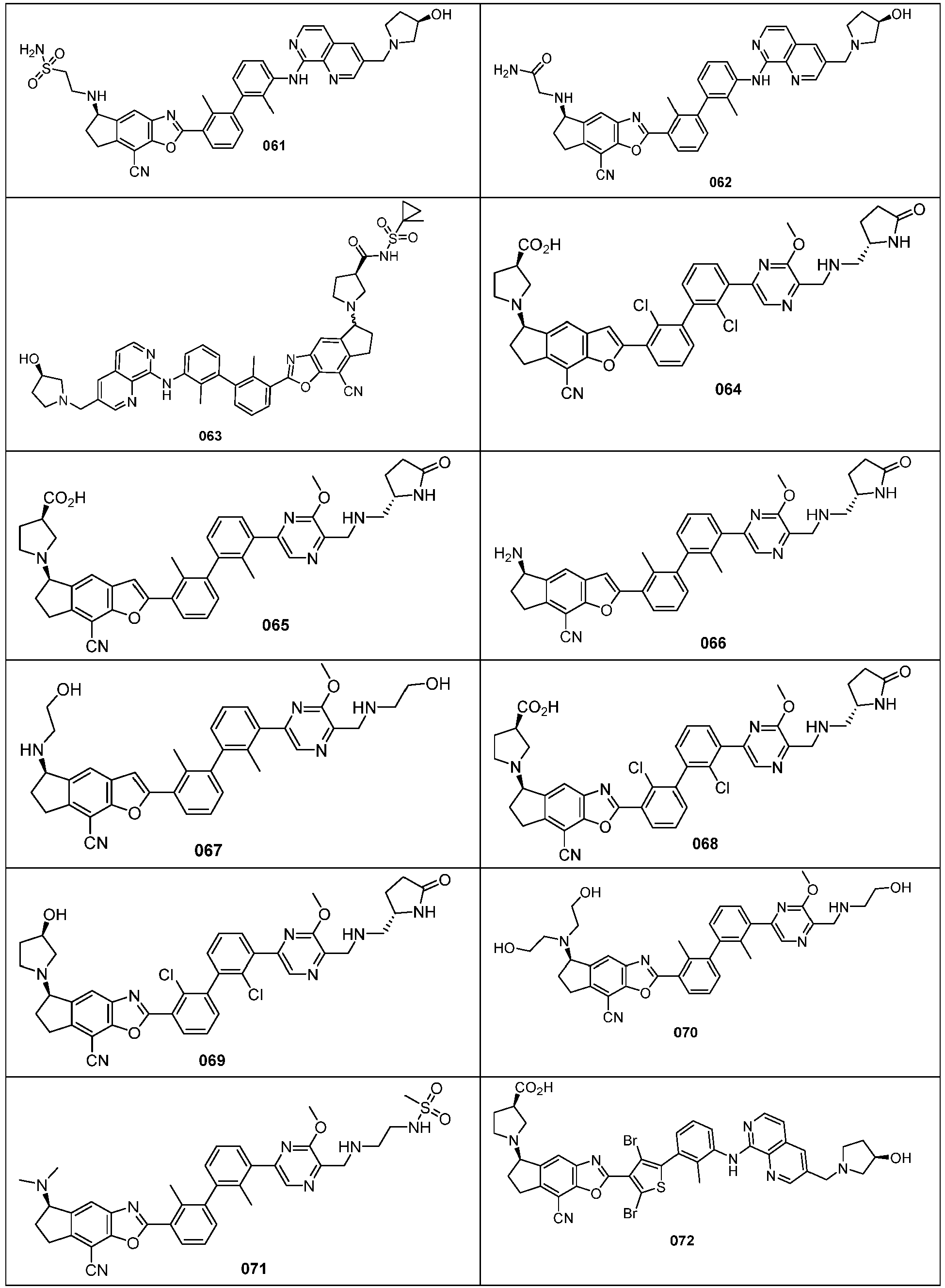

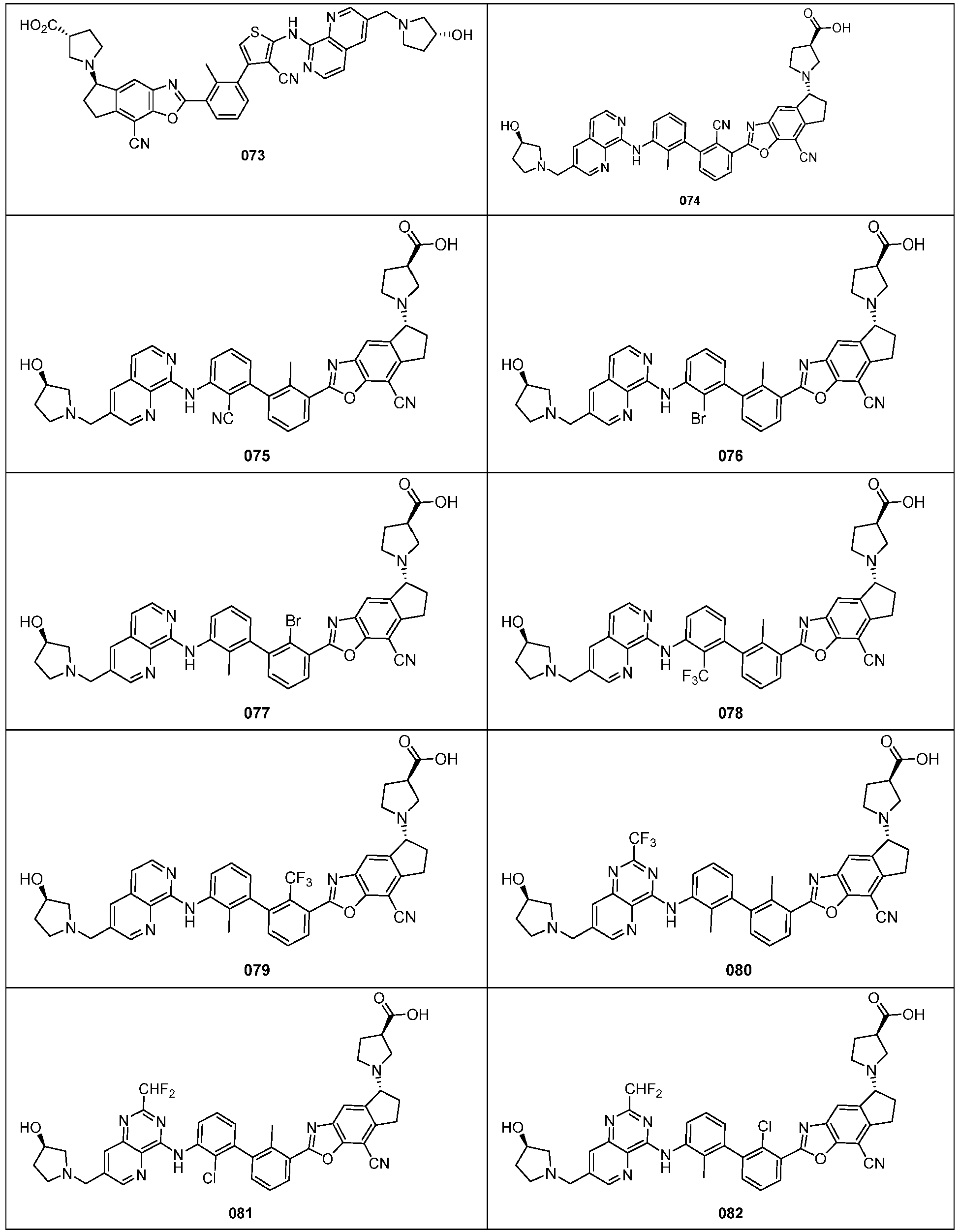

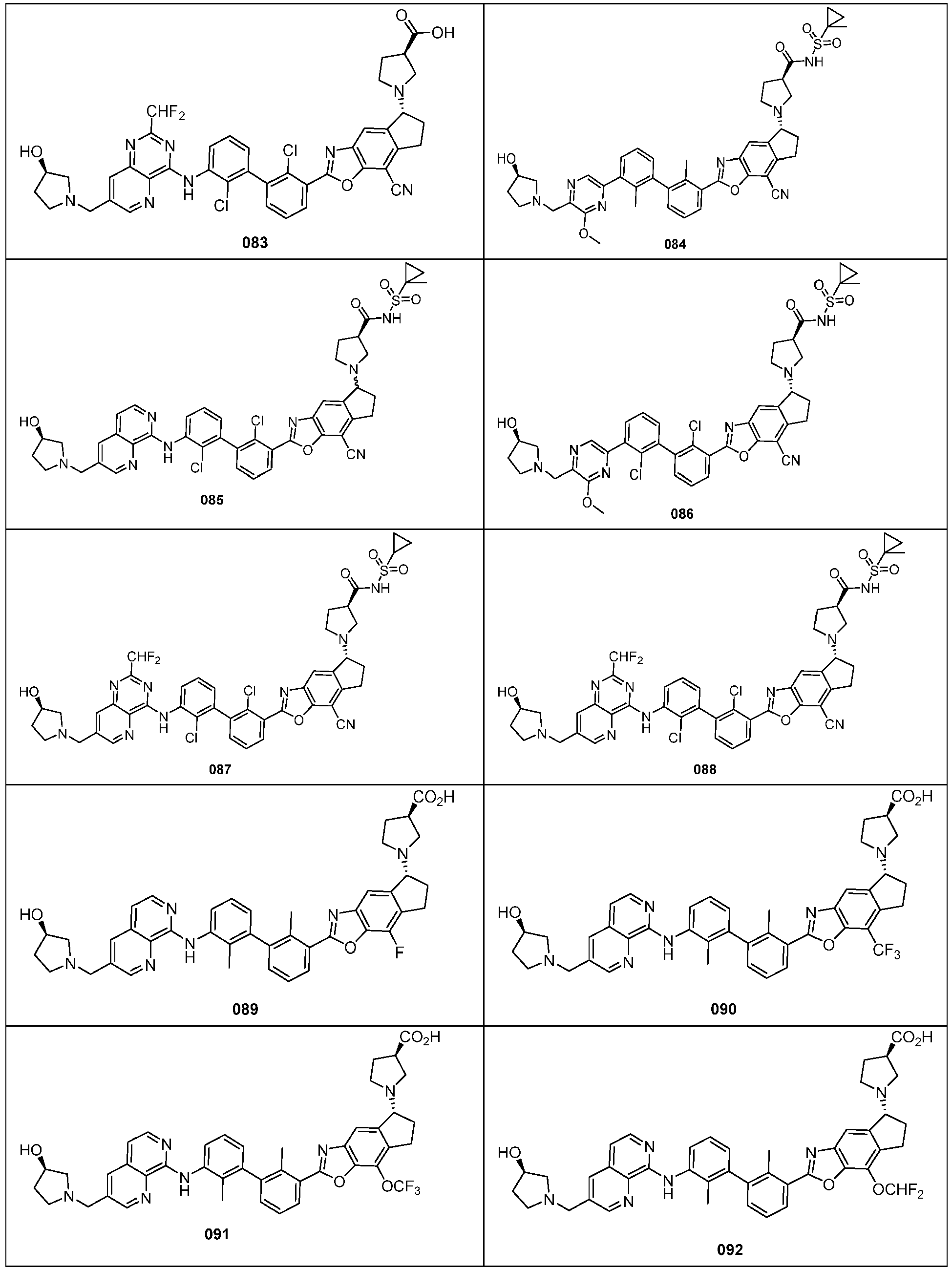

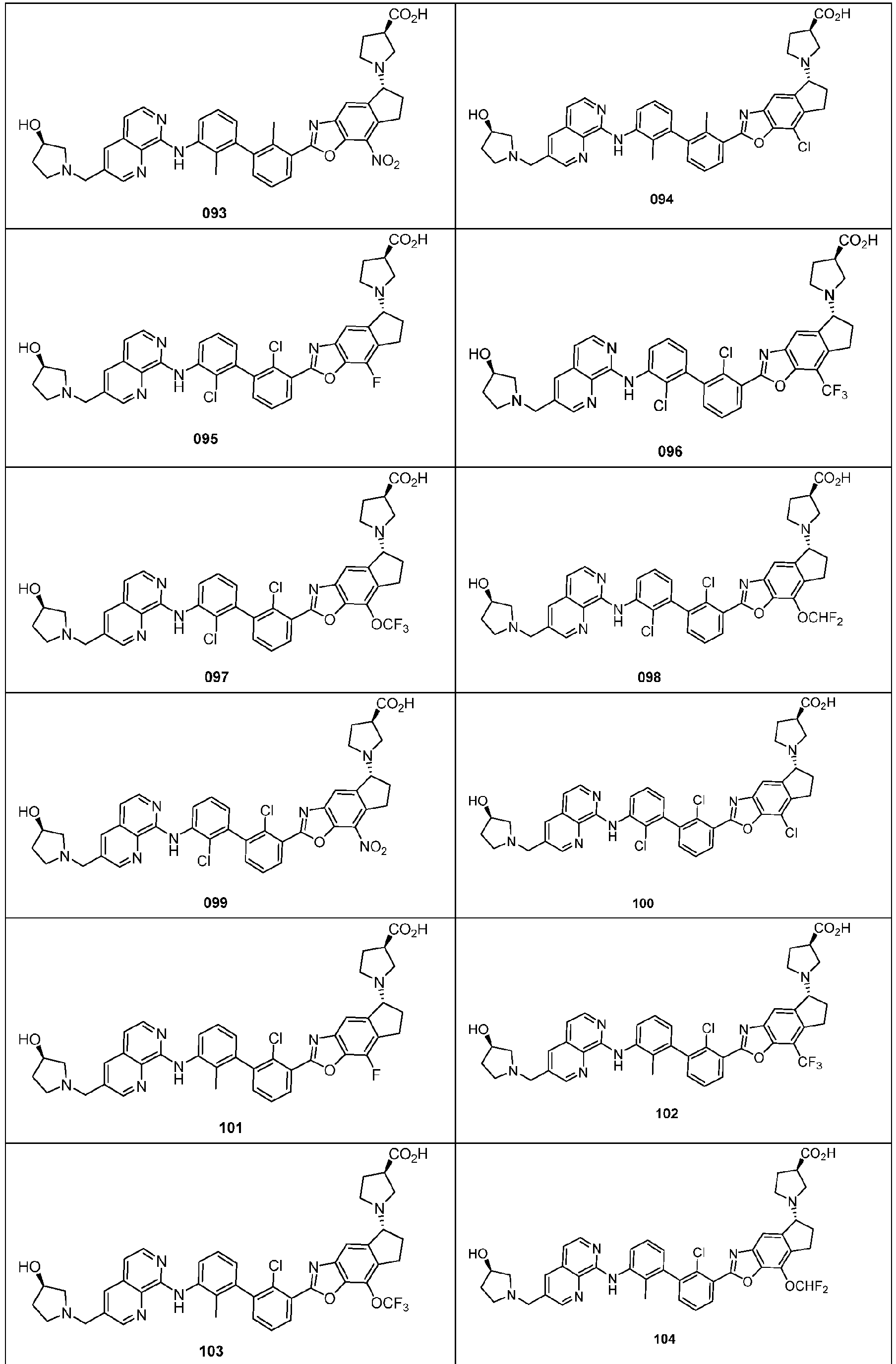

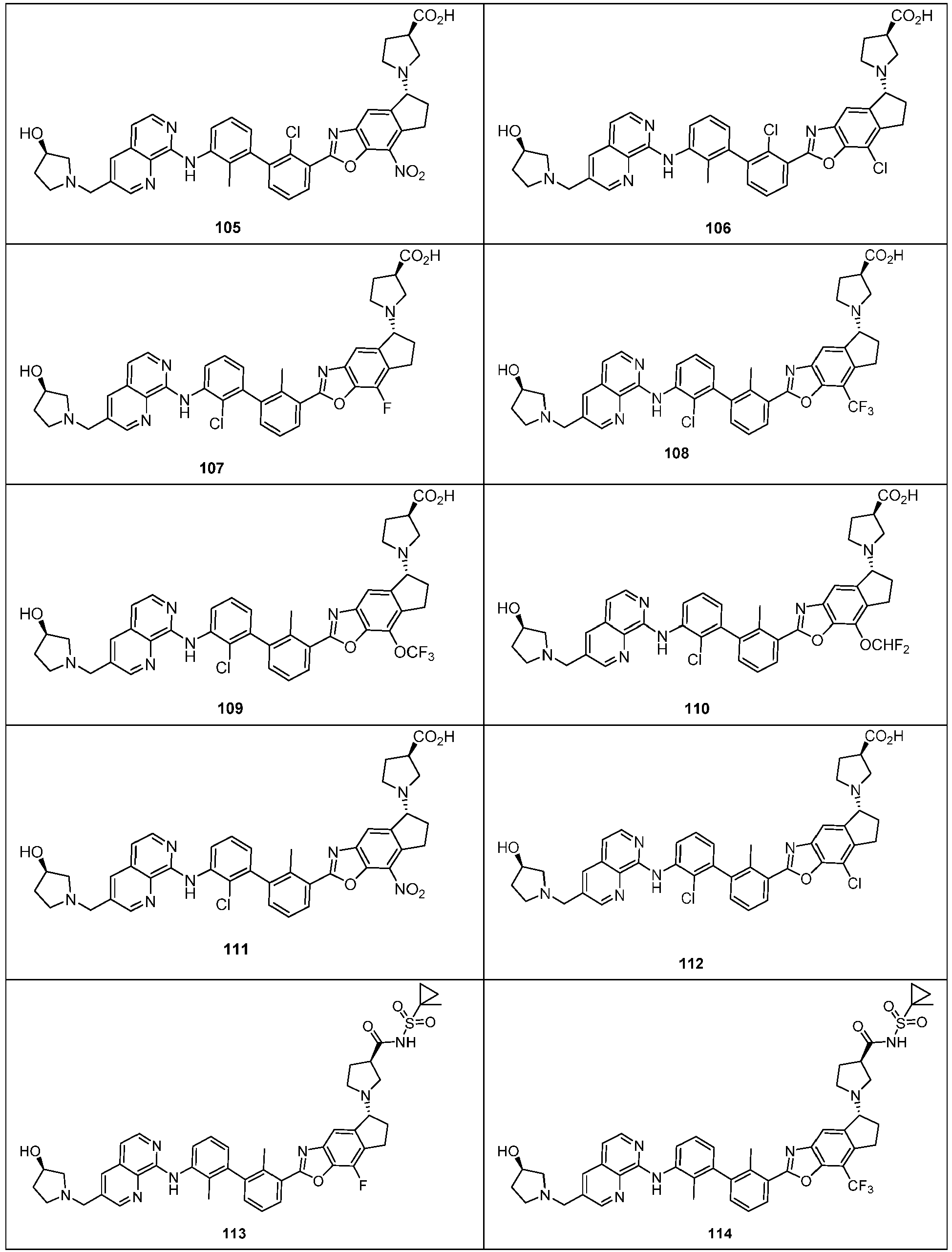

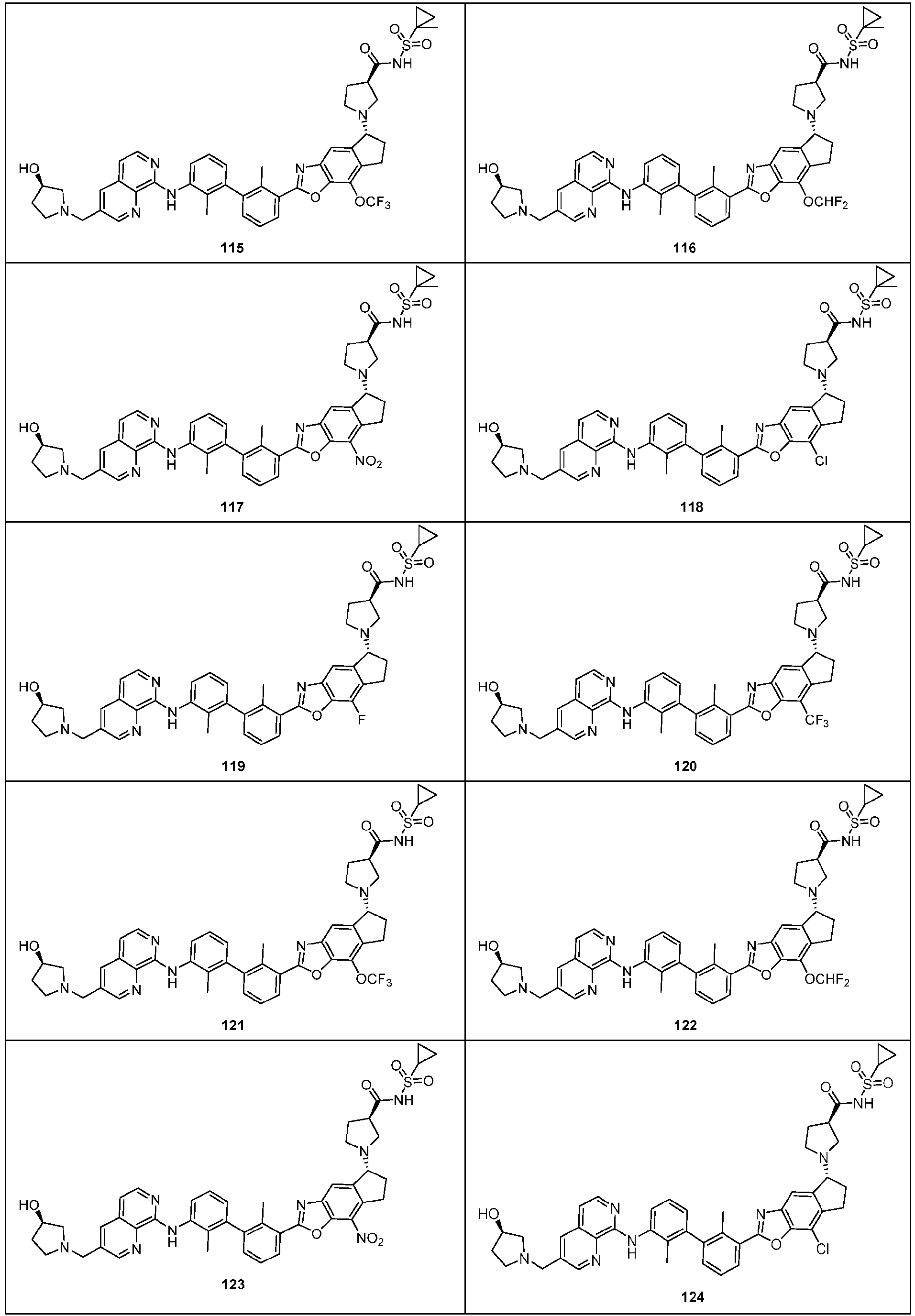

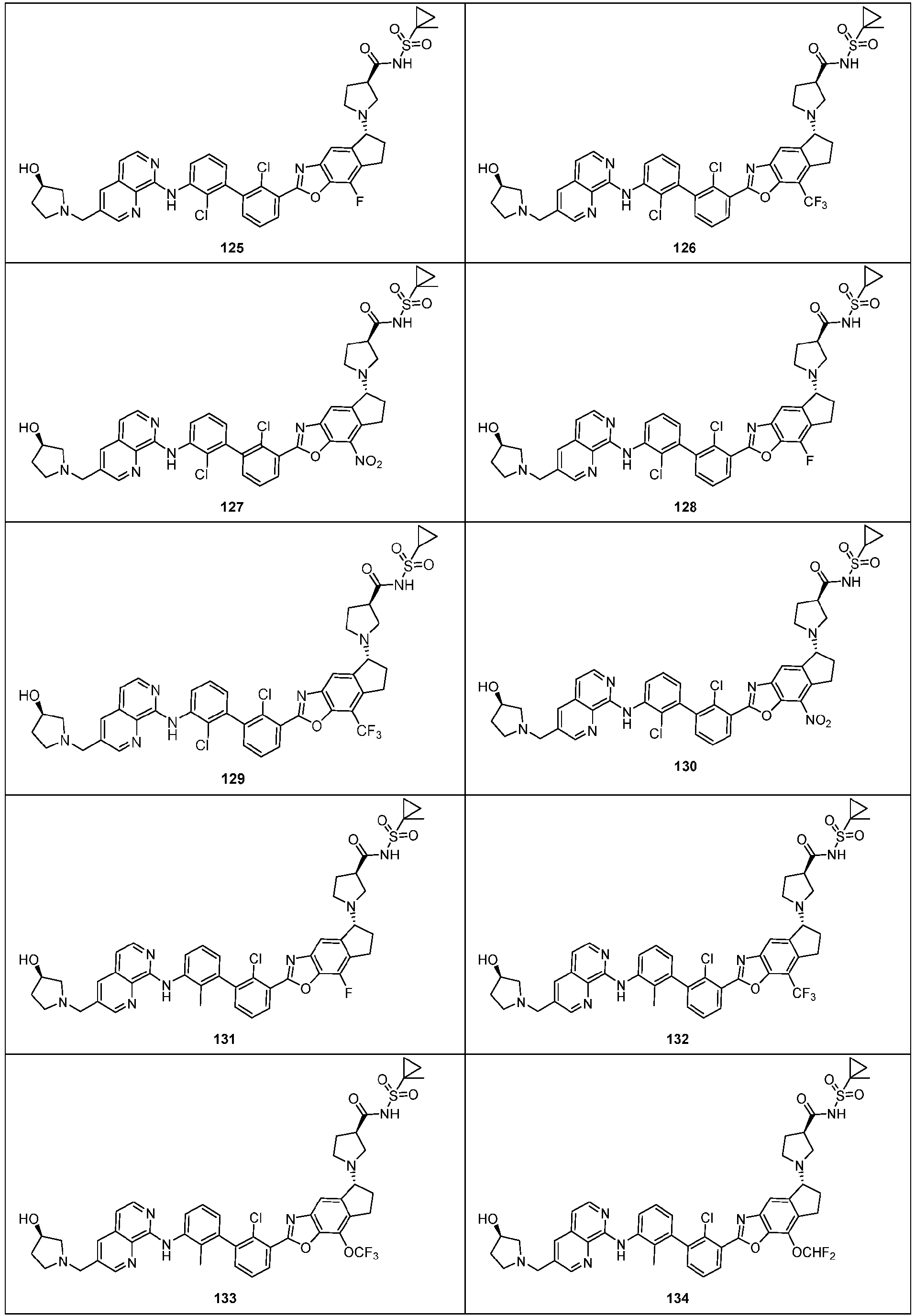

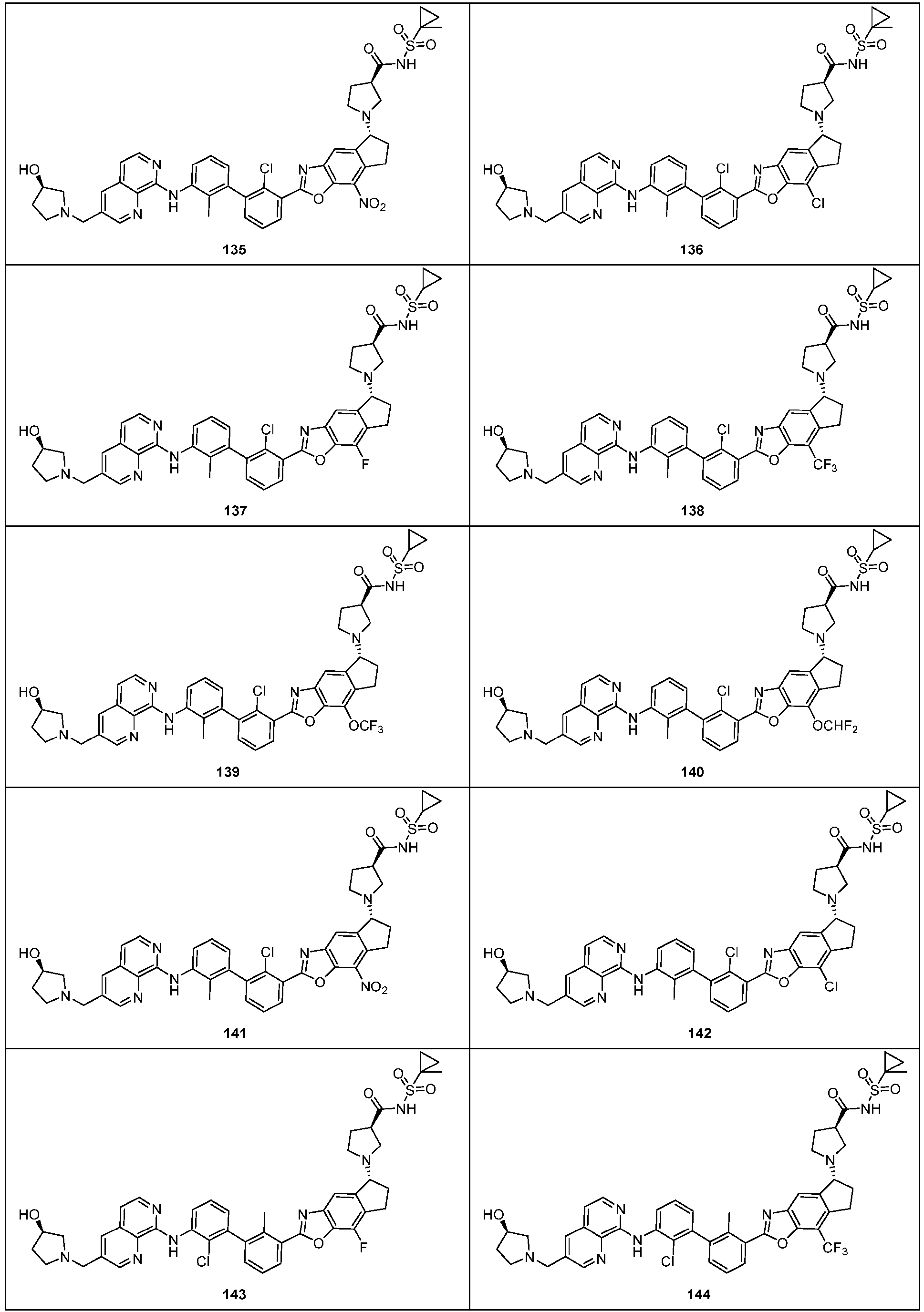

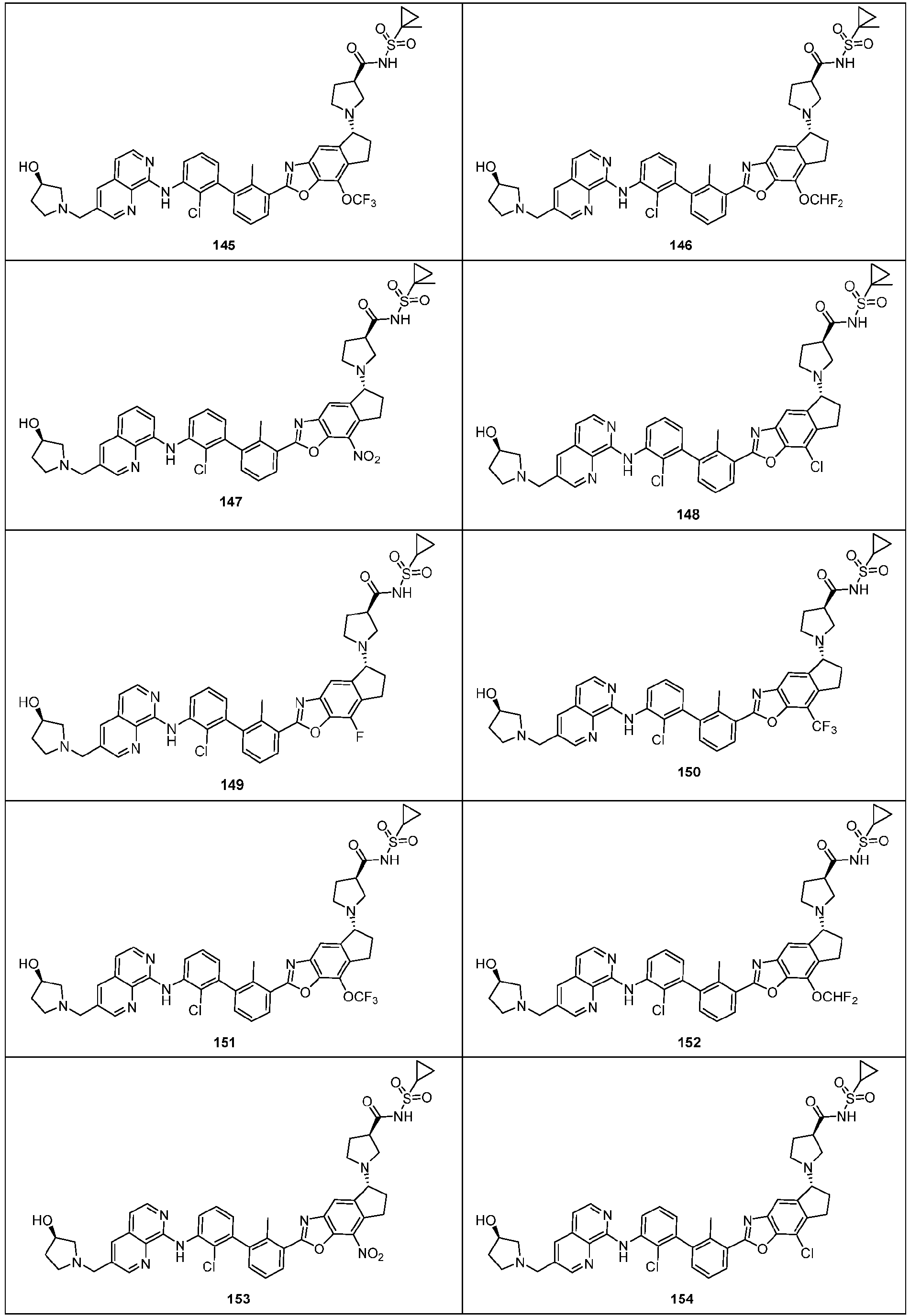

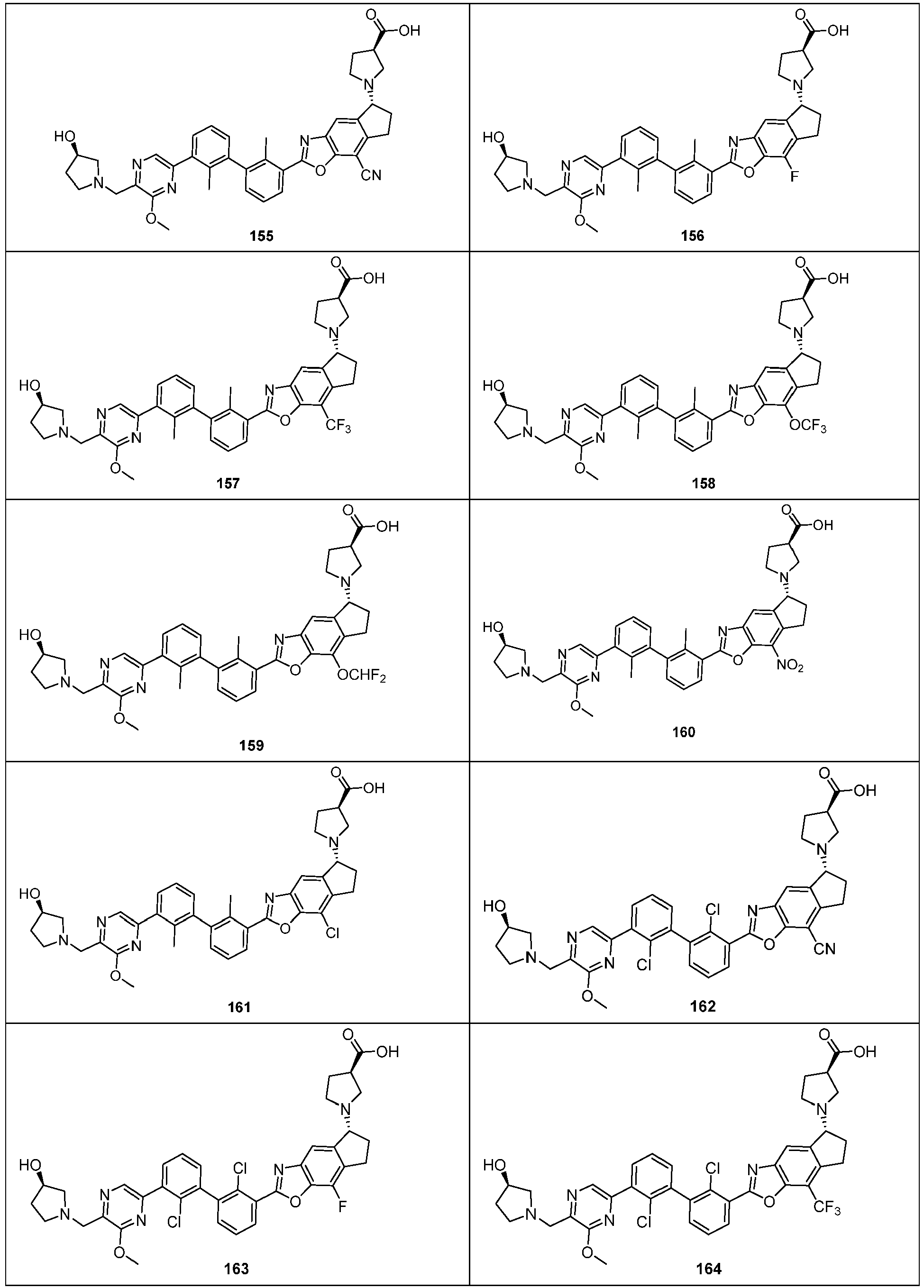

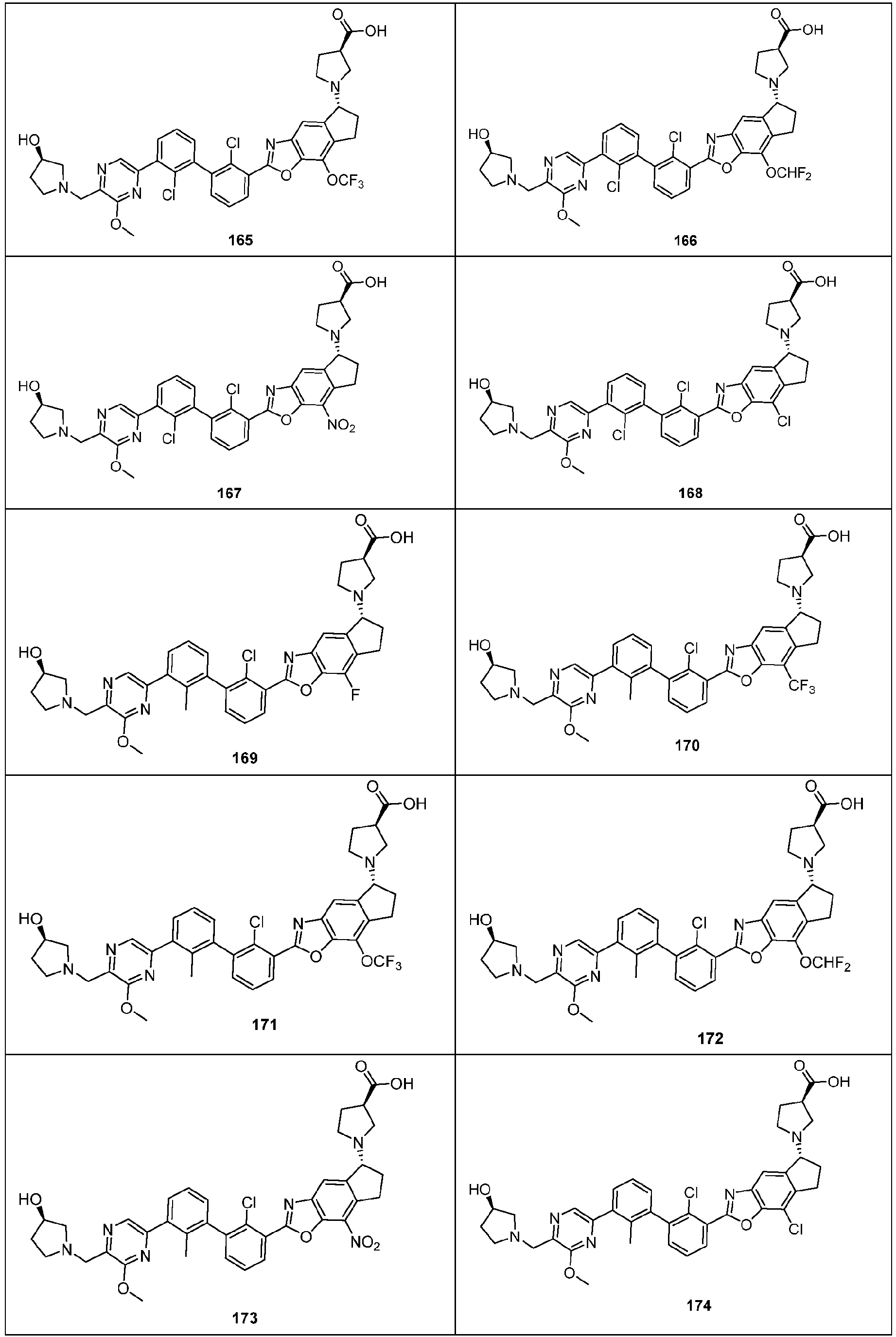

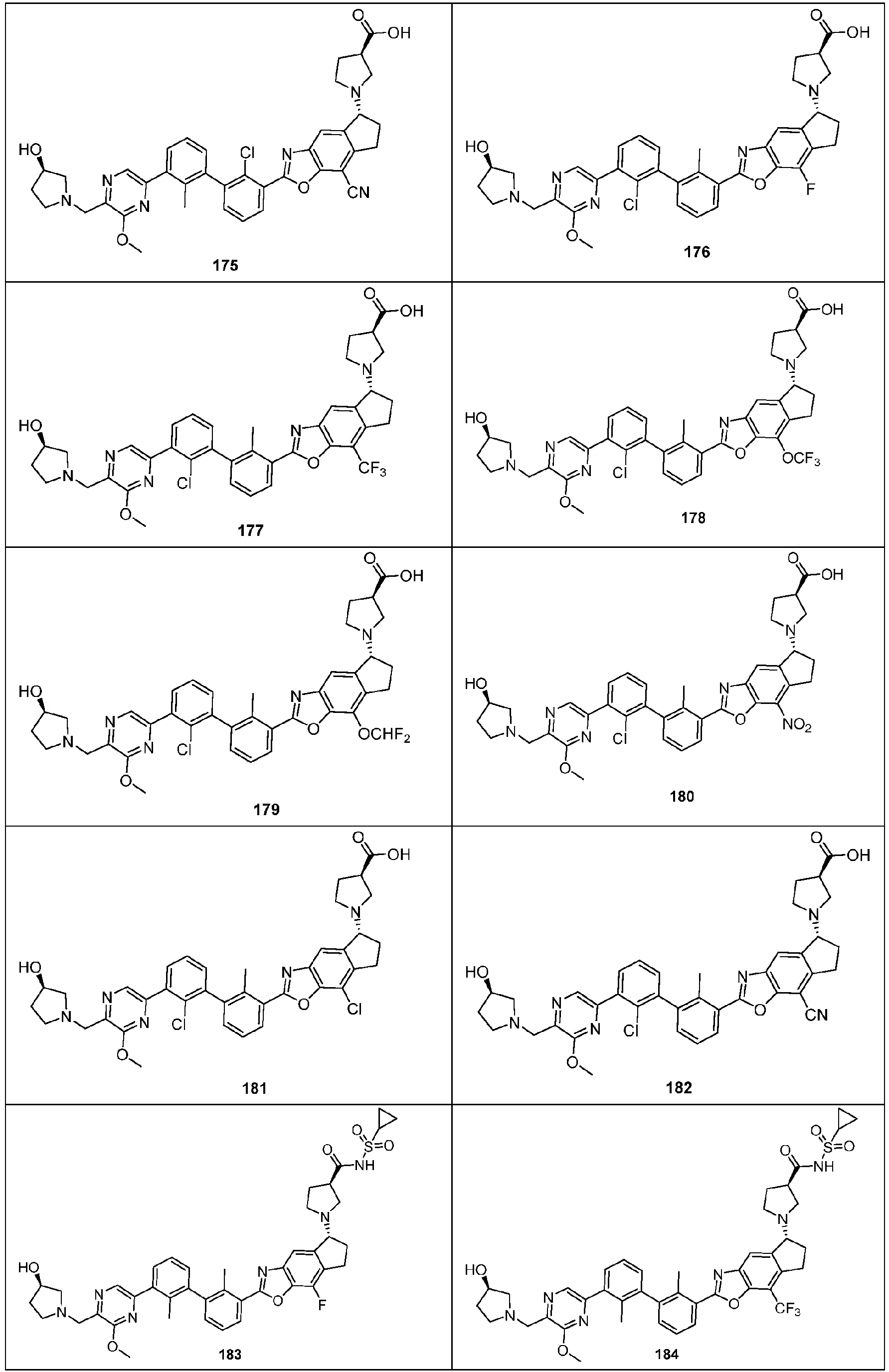

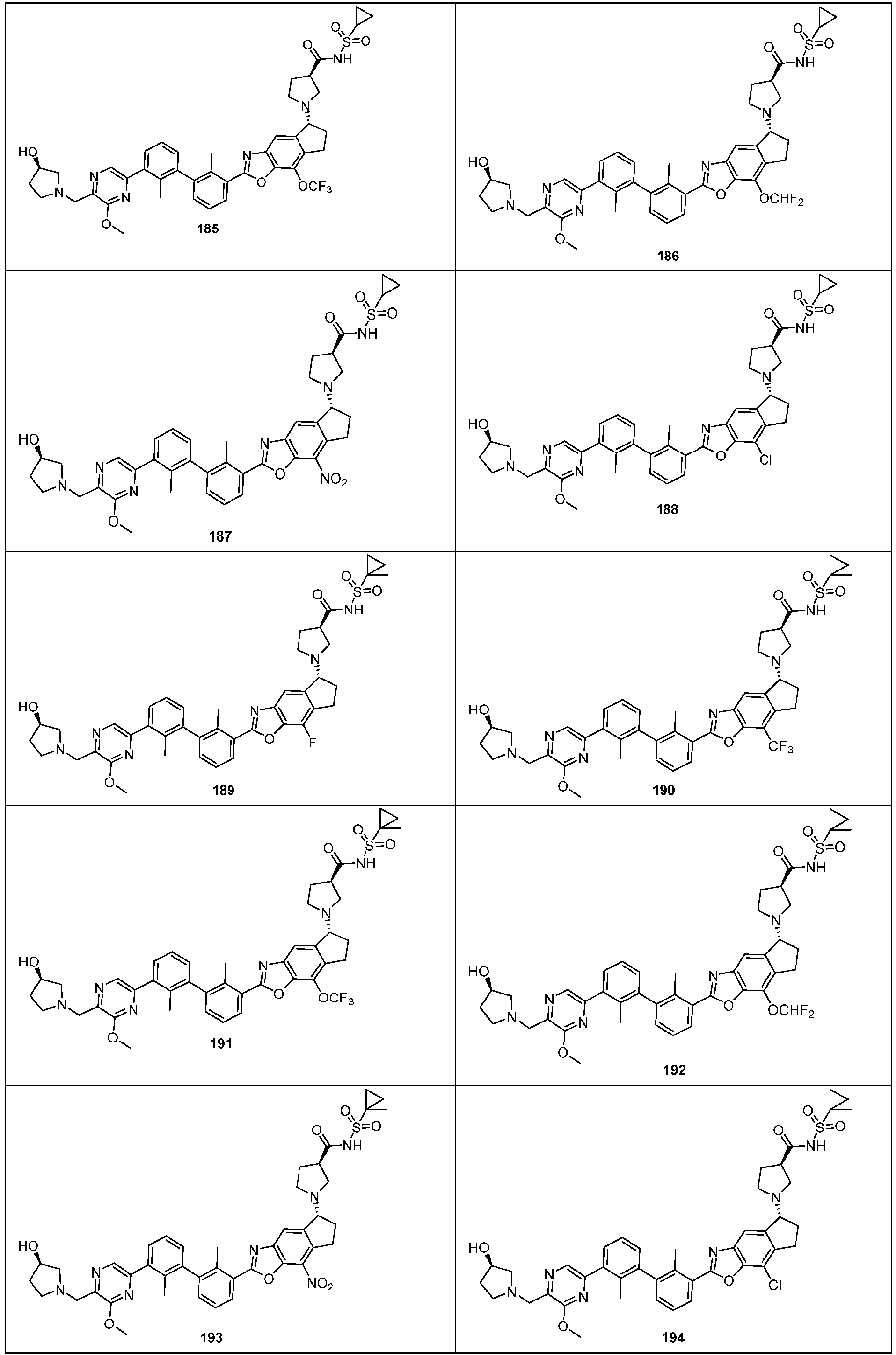

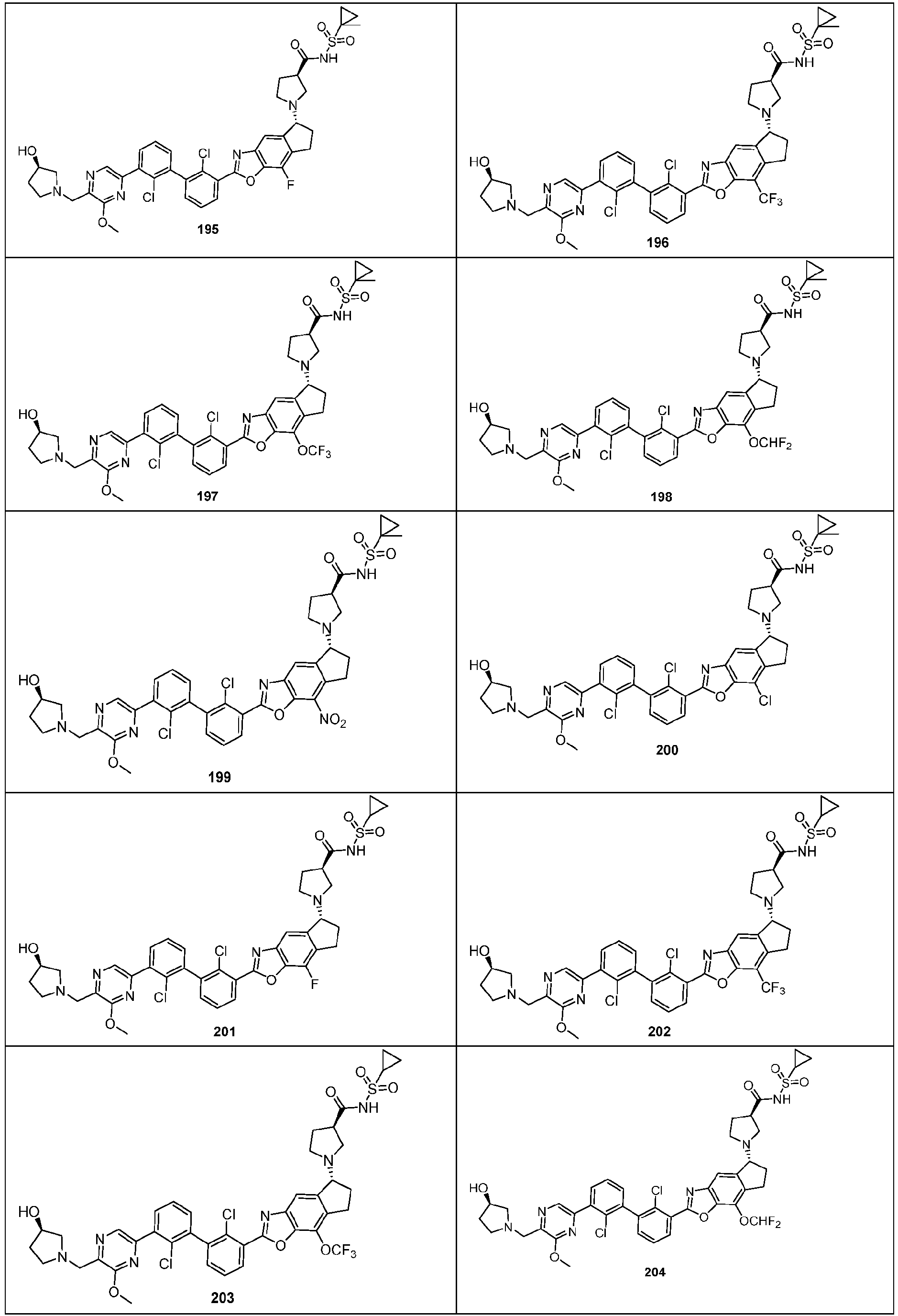

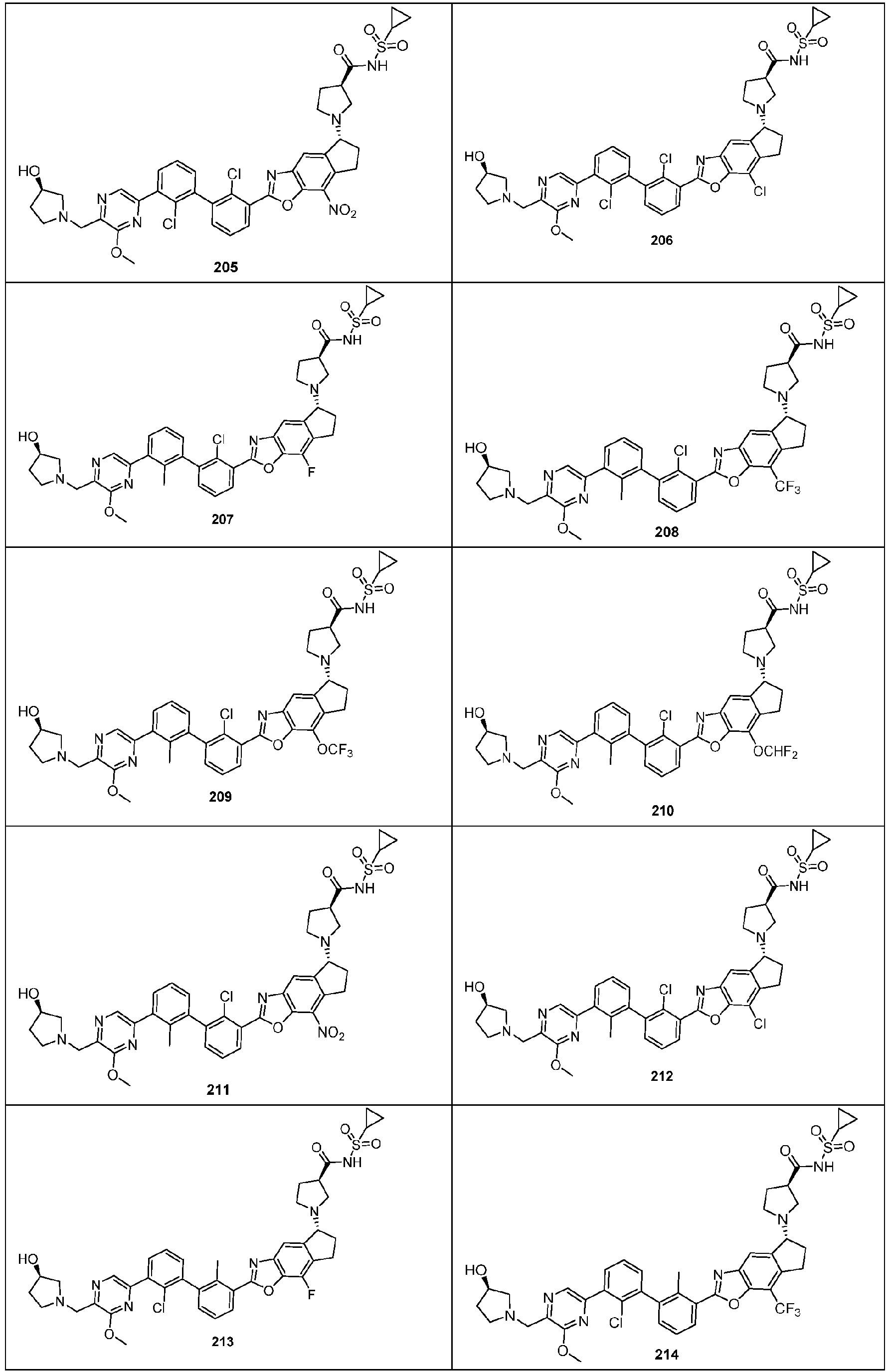

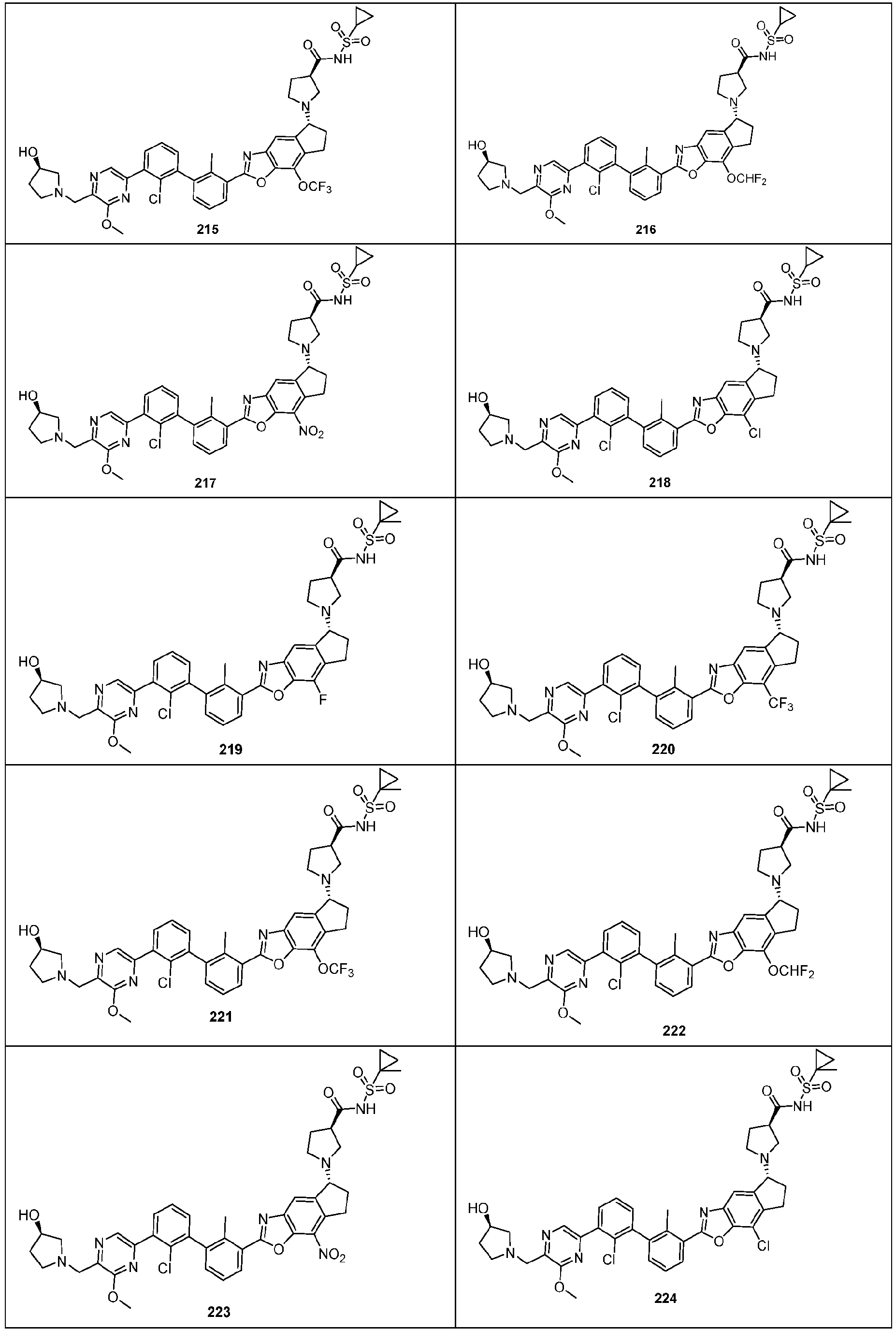

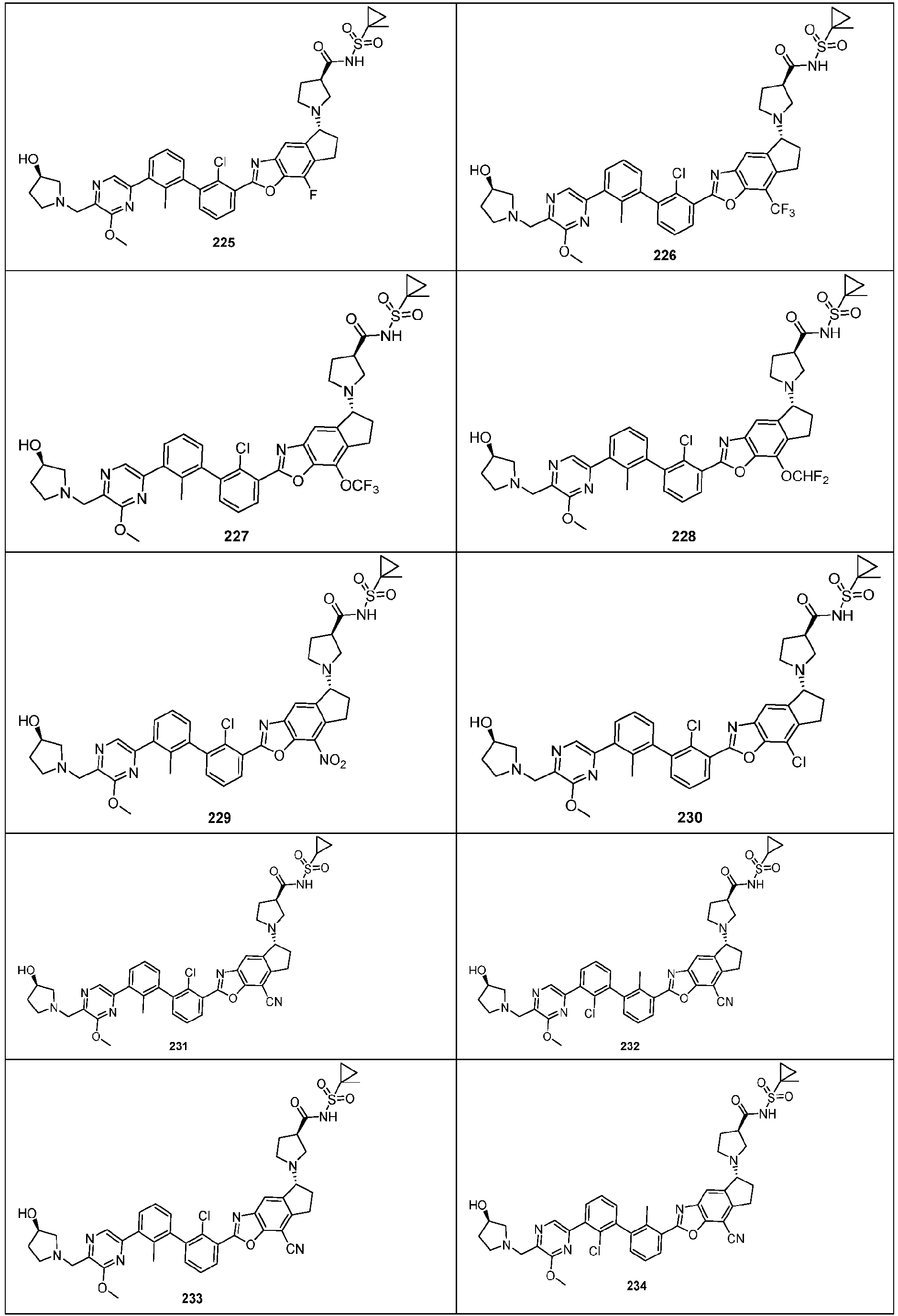

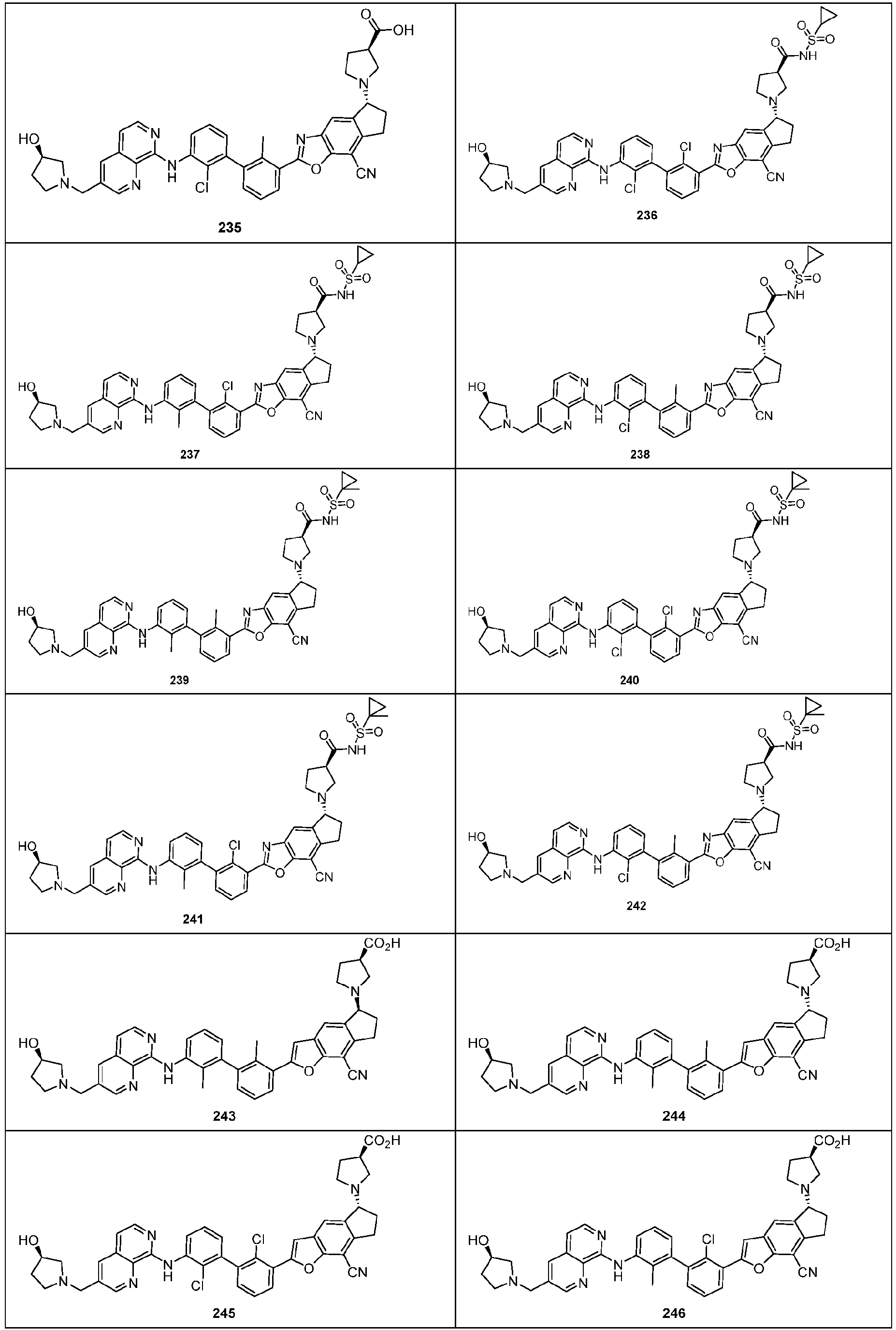

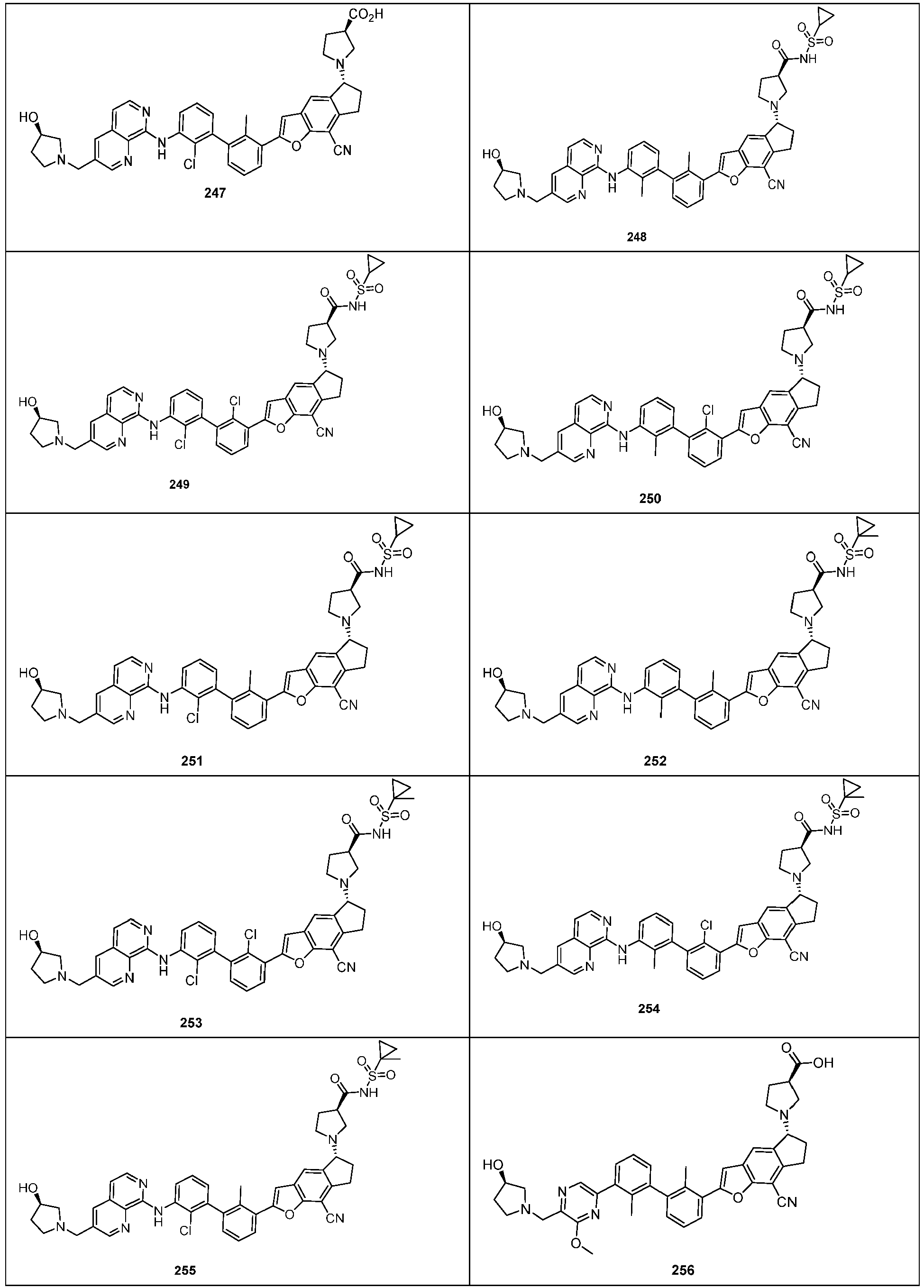

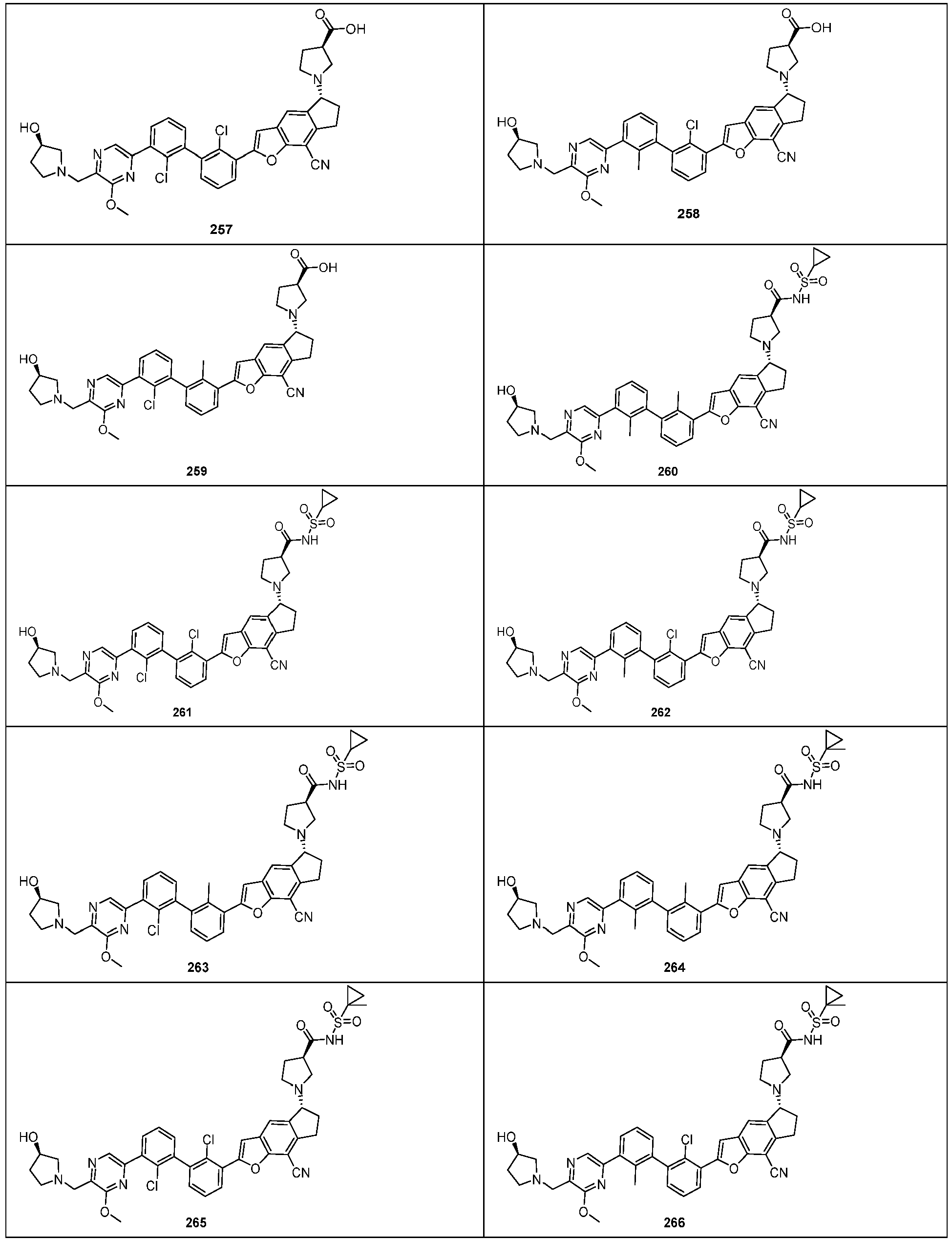

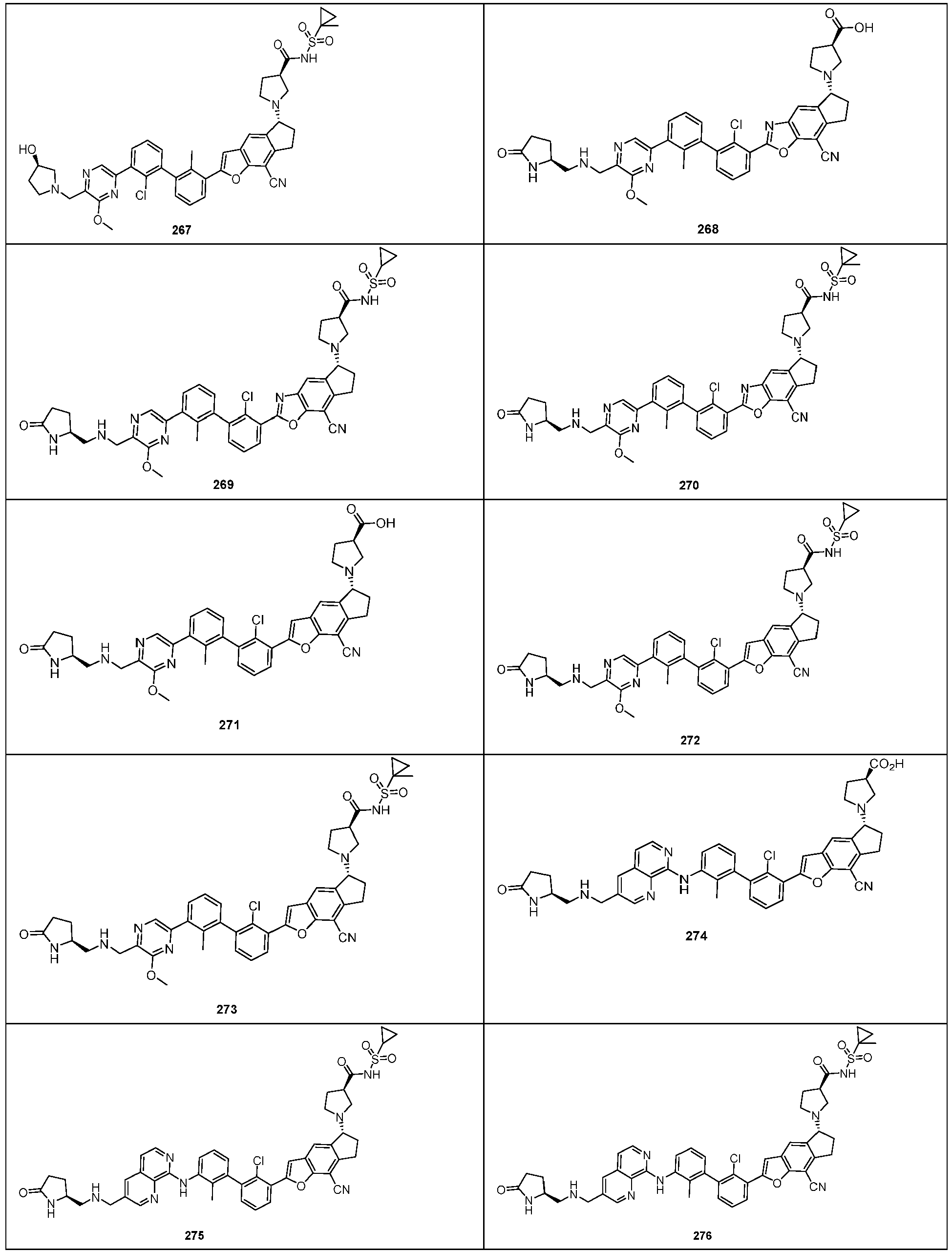

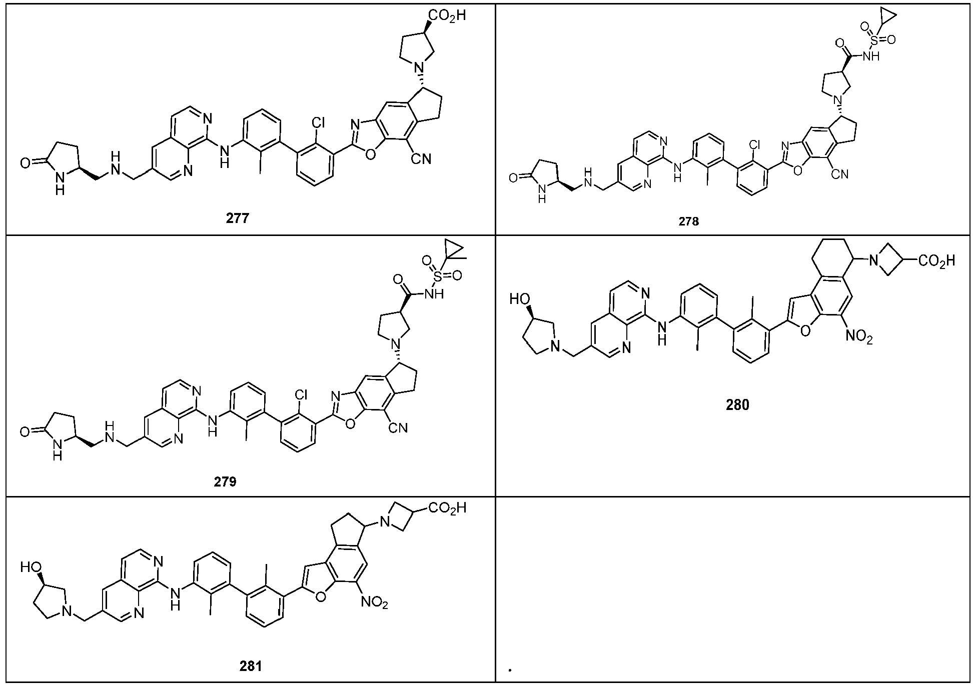

8. A preparation method of the compound of formula I according to claim 1, wherein the method comprises steps selected from the steps shown in Synthesis Scheme 1, 2 or 3:
Synthesis Scheme 1: 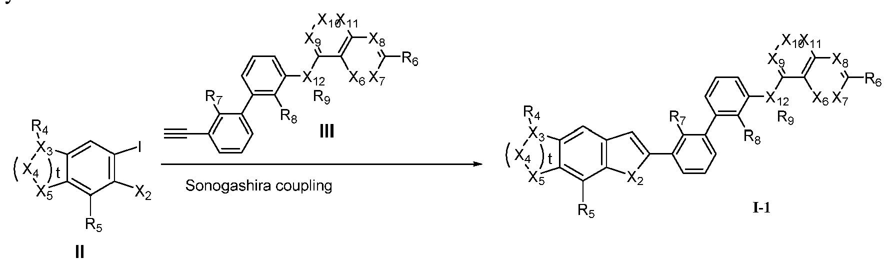
(a) subjecting intermediates **II** and **III** as raw materials to Sonogashira coupling reaction catalyzed by palladium catalyst to obtain target product **I-1;**
preferably, the preparation method of intermediate **II-2** is as follows: 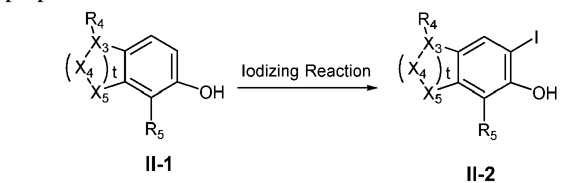
(a) subjecting II-1 as a raw material to a halogenation reaction under the catalysis of Lewis acid to obtain intermediate **II-2**;
preferably, the preparation method of intermediate **III** is as follows: 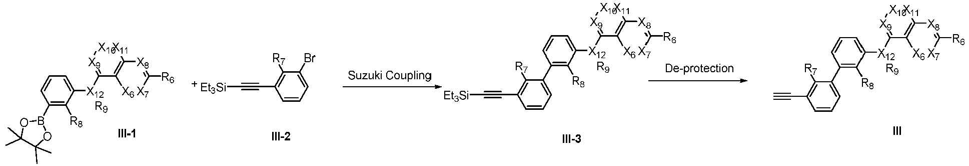
(a) subjecting compound **III-1** and **III-2** as raw materials to a coupling reaction (such as Suzuki, Buchwald, etc.) under the condition of palladium catalyst and ligand to obtain intermediate **III-3**;
(b) removing the silicon-based protecting group from **III-3** as a raw material by using a suitable reagent to obtain intermediate **III**;
Synthesis Scheme 2: 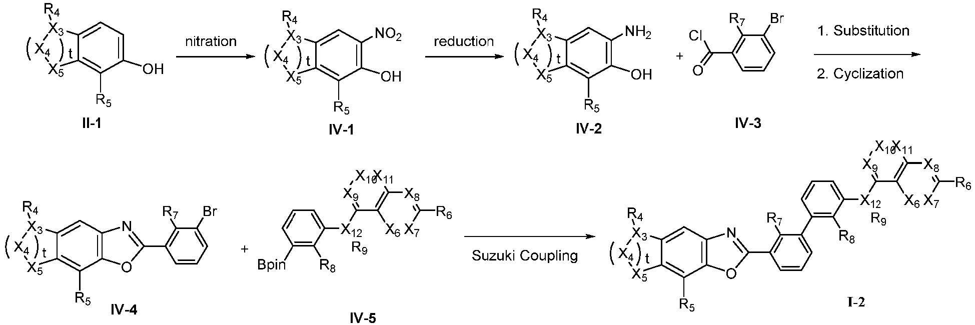
(a) reacting compound **II-1** as a raw material with nitrifying agent (such as concentrated sulfuric acid/NaNO₃, concentrated sulfuric acid/fuming nitric acid, etc.) to obtain intermediate **IV-1;**
(b) subjecting **IV-1** as a raw material to a reduction reaction under reducing condition (Pd-C/H₂; zinc powder/ammonium chloride; iron powder/acetic acid etc.) to form intermediate **IV-2;**
(c) subjecting **IV-2** and **IV-3** as raw materials to an affinity substitution reaction under alkaline condition to obtain an amide intermediate; then to a cyclization reaction in the presence of a suitable dehydration reagent (such as PPh₃/DDQ) to obtain intermediate **IV-4;**
(d) subjecting **IV-4** and **IV-5** as raw materials to a coupling reaction under the conditions of catalyst and ligand to form target intermediate **I-2;**
Synthesis Scheme 3: 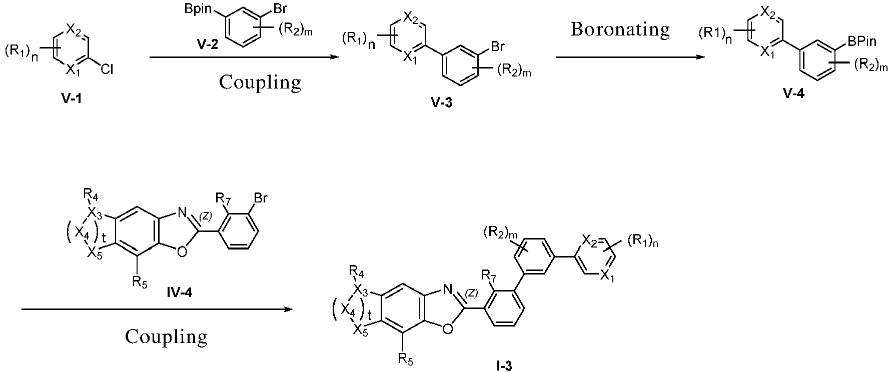
(a) subjecting **V-1** and **V-2** as raw materials to a coupling reaction under the conditions of catalyst and ligand to form intermediate **V-3;**
(d) subjecting **IV-4** and a suitable boron source (such as B₂Pin₂) as raw materials to a coupling reaction under the conditions of catalyst and ligand to form intermediate **V-4;**
(d) subjecting **V-4** and **IV-4** as raw materials to a coupling reaction under the conditions of catalyst and ligand to form target product **I-3;**
X₁-X₁₂, R₁-R₉, t, m, and n in the above formula are defiend as above.

9. A pharmaceutical composition comprising (1) the compound according to claim 1 or the stereoisomer or tautomer thereof, or the pharmaceutically acceptable salt, hydrate or solvate thereof; and (2) a pharmaceutically acceptable carrier.

10. Use of the compound according to claim 1 or the stereoisomer or tautomer thereof or the pharmaceutically acceptable salt, hydrate or solvate thereof, or the pharmaceutical composition according to claim 7, for the preparation of a pharmaceutical composition for preventing and/or treating diseases related to the activity or expression of PD-1/PD-L1.

11. The use according to claim 10, wherein the pharmaceutical composition is used to treat a disease selected from the group consisting of cancer, infectious disease, and autoimmune disease.

12. The use according to claim 10, wherein the cancer is selected from the group consisting of pancreatic cancer, bladder cancer, colorectal cancer, breast cancer, prostate cancer, kidney cancer, hepatocellular carcinoma, lung cancer, ovary cancer, cervical cancer, stomach cancer, esophageal cancer, melanoma, neuroendocrine cancer, central nervous system cancer, brain cancer, bone cancer, soft tissue sarcoma, non-small cell lung cancer, small cell lung cancer or colon cancer, skin cancer, lung cancer, urinary system tumor, blood tumor, glioma, digestive system tumor, reproductive system tumor, lymphoma, nervous system tumor, brain tumor, head and neck cancer.

13. The use according to claim 10, wherein the infectious disease is selected from bacterial infection and viral infection.

14. The use according to claim 10, wherein the autoimmune disease is selected from the group consisting of organ-specific autoimmune disease and systemic autoimmune disease.

15. The use according to claim 10, wherein the pharmaceutical composition further comprises at least one therapeutic agent selected from the group consisting of nivolumab, pembrolizumab, atezolizumab, and ipilimumab.
